(19) Europäisches Patentamt
European Patent Office
Office européen des brevets

(11) EP 1 872 942 B1

(12) **EUROPEAN PATENT SPECIFICATION**

(45) Date of publication and mention of the grant of the patent:
**18.06.2014 Bulletin 2014/25**

(21) Application number: **07012845.9**

(22) Date of filing: **29.06.2007**

(51) Int Cl.:
*B41C 1/10* (2006.01)
*C07D 213/89* (2006.01)
*C08F 2/46* (2006.01)
*G03G 13/26* (2006.01)
*G03F 7/027* (2006.01)
*G03F 7/031* (2006.01)
*G03F 7/033* (2006.01)

(54) **Lithographic printing plate precursor and lithographic printing method**

Lithographiedruckplattenvorläufer und Lithographiedruckverfahren

Précurseur de plaque d'impression lithographique et procédé d'impression lithographique

(84) Designated Contracting States:
**AT BE BG CH CY CZ DE DK EE ES FI FR GB GR HU IE IS IT LI LT LU LV MC MT NL PL PT RO SE SI SK TR**

(30) Priority:
**30.06.2006 JP 2006182543**
**28.09.2006 JP 2006264820**
**26.03.2007 JP 2007080183**

(43) Date of publication of application:
**02.01.2008 Bulletin 2008/01**

(73) Proprietor: **FUJIFILM Corporation**
**Minato-ku**
**Tokyo (JP)**

(72) Inventors:
- **Iwai, Yu**
  **Yoshida-cho,**
  **Haibara-gun,**
  **Shizuoka (JP)**
- **Taguchi, Yoshinori**
  **Yoshida-cho,**
  **Haibara-gun,**
  **Shizuoka (JP)**
- **Suzuki, Shota**
  **Yoshida-cho,**
  **Haibara-gun,**
  **Shizuoka (JP)**

(74) Representative: **HOFFMANN EITLE**
**Patent- und Rechtsanwälte**
**Arabellastrasse 4**
**81925 München (DE)**

(56) References cited:
**EP-A- 0 373 862**
**EP-A- 1 621 340**
**WO-A-03/087939**
**JP-A- 2006 241 301**
**US-A- 6 090 865**
**US-A1- 2003 082 475**
**US-A1- 2004 197 701**
**US-A1- 2006 057 492**

- **IVO REETZ ET AL.: POLYMER INTERNATIONAL, vol. 43, 1997, pages 27-32, XP002469491**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 872 942 B1

## Description

FIELD OF THE INVENTION

The present invention relates to a lithographic printing plate precursor and a lithographic printing method using the same. More specifically, it relates to a lithographic printing plate precursor capable of undergoing a so-called direct plate-making, which can be directly plate-made by scanning of laser having, for example, a wavelength of 300 to 1,200 nm, based on digital signals, for example, from a computer and a lithographic printing method wherein the above-described lithographic printing plate precursor is directly developed on a printing machine to conduct printing without undergoing a development processing step.

BACKGROUND OF THE INVENTION

In general, a lithographic printing plate is composed of an oleophilic image area accepting ink and a hydrophilic non-image area accepting dampening water (fountain solution) in the process of printing. Lithographic printing is a printing method which comprises rendering the oleophilic image area of the lithographic printing plate to an ink-receptive area and the hydrophilic non-image area thereof to a dampening water-receptive area (ink unreceptive area), thereby making a difference in adherence of ink on the surface of the lithographic printing plate, and depositing the ink only to the image area by utilizing the nature of the dampening water and oily ink to repel with each other, and then transferring the ink to a printing material, for example, paper.

In order to produce the lithographic printing plate, a lithographic printing plate precursor (PS plate) comprising a hydrophilic support having provided thereon an oleophilic photosensitive resin layer (image-recording layer) has heretofore been broadly used. Ordinarily, the lithographic printing plate is obtained by conducting plate making according to a method of exposing the lithographic printing plate precursor through an original, for example, a lith film, and then removing the unnecessary portion of the image-recording layer by dissolving with an alkaline developer or an organic solvent thereby revealing the hydrophilic surface of support while leaving the image-recording layer for forming the image area.

In the hitherto known plate-making process of lithographic printing plate precursor, after exposure, the step of removing the unnecessary portion of the image-recording layer by dissolving, for example, with a developer corresponding to the image-recording layer is required. However, it is one of the subjects to save or simplify such an additional wet treatment described above. Particularly, since disposal of liquid wastes discharged accompanying the wet treatment has become a great concern throughout the field of industry in view of the consideration for global environment in recent years, the demand for the solution of the above-described subject has been increased more and more.

As one of simple plate-making methods in response to the above-described requirement, it has been proposed a method referred to as on-press (on-machine) development wherein a lithographic printing plate precursor having an image-recording layer in which the unnecessary portion is capable of being removed in a conventional printing process is used and after exposure, the unnecessary portion of the image-recording layer is removed on a printing machine to prepare a lithographic printing plate.

Specific methods of the on-press development include, for example, a method of using a lithographic printing plate precursor having an image-recording layer that can be dissolved or dispersed in dampening water, an ink solvent or an emulsion of dampening water and ink, a method of mechanically removing an image-recording layer by contact with rollers or a blanket cylinder of a printing machine, and a method of lowering cohesion of an image-recording layer or adhesion between an image-recording layer and a support upon penetration of dampening water, ink solvent or the like and then mechanically removing the image-recording layer by contact with rollers or a blanket cylinder of a printing machine.

In the invention, unless otherwise indicated particularly, the term "development processing step" means a step of using an apparatus (ordinarily, an automatic developing machine) other than a printing machine and removing a laser-unexposed area in the lithographic printing plate precursor upon contact with liquid (ordinarily, an alkaline developer) thereby revealing a hydrophilic surface of support. The term "on-press development" means a method and a step of removing a laser-unexposed area in the lithographic printing plate precursor upon contact with liquid (ordinarily, printing ink and/or dampening water) by using a printing machine thereby revealing a hydrophilic surface of support.

On the other hand, digitalized technique of electronically processing, accumulating and outputting image information using a computer has been popularized in recent years, and various new image outputting systems responding to the digitalized technique have been put into practical use. Correspondingly, attention has been drawn to a computer-to-plate (CTP) technique of carrying digitalized image information on highly converging radiation, for example, laser light and conducting scanning exposure of a lithographic printing plate precursor with the light thereby directly preparing a lithographic printing plate without using a lith film. Thus, it is one of important technical subjects to obtain a lithographic printing plate precursor adaptable to the technique described above.

As described above, in recent years, the simplification of plate-making operation and the realization of dry system and non-processing system have been further strongly required from both aspects of the consideration for global environment and the adaptation for digitization.

As a lithographic printing plate precursor capable of undergoing scanning exposure among the lithographic printing plate precursors, a lithographic printing plate precursor comprising a hydrophilic support having provided thereon an oleophilic photosensitive resin layer containing a photosensitive compound capable of generating an active species, for example, a radical or a Bronsted acid by laser exposure has been proposed and such a printing plate precursor has been put on the market A negative lithographic printing plate can be obtained by subjecting the lithographic printing plate precursor to laser scanning on the basis of digital information to generate an active species, causing a physical or chemical change in the photosensitive layer (image-recording layer) by the action of the active species to make the image-recording layer insoluble, and then development processing the lithographic printing plate precursor. Particularly, a lithographic printing plate precursor comprising a hydrophilic support having provided thereon a photopolymerization type image-recording layer containing a photopolymerization initiator excellent in photo-speed, an addition polymerizable ethylenically unsaturated compound and a binder polymer soluble in an alkali developer and, if desired, an oxygen-blocking protective layer can provide a lithographic printing plate having desired printing properties, for example, excellent productivity, ease of undergoing development processing, and good resolving power and ink receptivity.

As a lithographic printing plate precursor capable of undergoing on-press development, for example, a lithographic printing plate precursor having provided on a hydrophilic support, an image-forming layer in which hydrophobic thermoplastic polymer particles are dispersed in a hydrophilic binder is described in Japanese Patent 2,938,397. It is described in Japanese Patent 2,938,397 that the lithographic printing plate precursor can be exposed to an infrared laser to agglomerate the hydrophobic thermoplastic polymer particles by heat thereby forming an image, and mounted on a cylinder of a printing machine to carry out on-press development by supplying dampening water and/or ink.

Although the method of forming image by the agglomeration of fine particles only upon thermal fusion shows good on-press development property, it has a problem in that the image strength (adhesion to the support) is extremely weak and printing durability is insufficient.

Further, a lithographic printing plate precursor having provided on a hydrophilic support, an image-recording layer (heat-sensitive layer) including microcapsules containing a polymerizable compound encapsulated therein is described in JP-A-2001-277740 (the term "JP-A" as used herein means an "unexamined published Japanese patent application") or JP-A-2001-277742.

Moreover, a lithographic printing plate precursor having provided on a support, an image-recording layer containing an infrared absorbing agent, a radical polymerization initiator and a polymerizable compound is described in JP-A-2002-287334.

The method using the polymerization reaction has a feature that since chemical bond density in the image area is high, the image strength is relatively good in comparison with the image area formed by the thermal fusion of fine polymer particles. However, from a practical standpoint, any of the on-press development property, reproducibility of fine lines and printing durability is still insufficient.

Furthermore, a lithographic printing plate precursor capable of undergoing on-press development having provided on a support, an image-recording layer containing a polymerizable compound and a graft polymer having a polyethylene oxide chain in its side chain or a block polymer having a polyethylene oxide block is described in U. S. Patent Publication No. 2003/0064318.

However, this technique is still insufficient in view of the reproducibility of fine lines and printing durability, although the on-press development property is good.

In general, an operation for inspection and discrimination of image formed on a printing plate is carried out in order to examine whether the image is recorded on the printing plate as intended, in advance of mounting the printing plate on a printing machine. In a conventional lithographic printing plate precursor subjected to the development processing step, it is ordinarily easily performed to confirm the image formed after the plate-making (after the development processing) and before the printing (before the mounting the printing plate on a printing machine) by means of coloration of the image-recording layer.

However, with respect to the lithographic printing plate precursor of the on-press development type or non-processing (non-development) type without accompanying the development processing prior to printing, the image is not recognized on the printing plate in the step of mounting it on a printing machine, and thus the discrimination of the printing plate can not be performed. In particular, it is important in the printing operation to determine whether a registry guide (register mark) which acts as a landmark for the registering in multicolor printing is recorded. Therefore, in the lithographic printing plate precursor of the on-press development type or non-processing (non-development) type, a means for confirming the image, that is, color formation or decoloration in the exposed or heated area, is required.

A printing plate has been proposed wherein a compound capable of generating an acid, base or radical by means of light or heat and a compound capable of undergoing color change upon interaction with the acid, base or radical generated are used as a print-out agent (for example, see JP-A-11-277927). Also, it has been proposed to utilize

color change of thermally decomposable compound as the print-out agent of a direct-drawing type lithographic printing plate precursor having a heat-sensitive layer (for example, see JP-A-2000-335129). Further, it has been proposed to use a thermally decomposable dye having a thermally decomposable temperature of 250°C or below as the print-out agent (for example, see JP-A-2003-191657).

According to these proposals, the color formation or decoloration occurs in the exposed area and the image-confirmation property increases to some extent. However, there are various problems, for example, in that color formation sufficient for performing the operation of plate inspection can not yet be obtained, in that the dye formed or the dye which has not been decomposed or decolored by the exposure stains dampening water to adversely affect finish of printed materials, in that insoluble dyes remain in ink and dampening water, and in that the dyes reacts with components of ink and dampening water to form a precipitate, that is, a scum.

EP-A-1 621 340 discloses a polymerizable composition including

(A) a compound of formula (I)

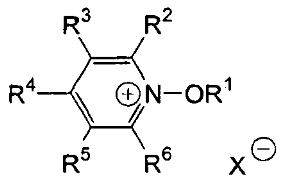

(I),

(B) an infrared absorbent, and (C) a compound having at least one addition-polymerizable ethylenically unsaturated bond, and a negative planographic printing plate precursor having a recording layer containing the polymerizable composition.

US 2003/0082475 A1 relates to an on-press developable thermosensitive lithographic plate having on a substrate a thermo-sensitive layer comprising an ethylenically unsaturated monomer, an infrared absorbing dye, and an onium or borate salt free radical initiator. The plate can be image-wise exposed with an infrared laser on a plate exposure device, and then mounted on a lithographic press for on-press development with ink and/or fountain solution and lithographic printing without needing a regular development process with a liquid developer. Alternatively, the plate can be image-wise exposed with an infrared laser while mounted on a plate cylinder of a lithographic press, on-press developed with ink and/or fountain solution, and then print images to the receiving sheets.

US 2006/0057492 A1 describes a polymer having a polymerizable group and an alkyleneoxy groups on side chains thereof, and a polymerizable composition containing the polymer. The polymerizable composition preferably contains a polymerizable compound and a polymerization initiator. Also disclosed is a planographic printing plate precursor, which can form an image without being subjected to an alkali development, and has, on a hydrophilic support, a polymerizable layer containing a polymer having a polymerizable on a side chain thereof.

JP-A-2006-241301 discloses an ink composition comprising a compound represented by formula (I) or (II) and a polymerizable compound, as well as an inkjet recording method comprises using the ink composition, and a printed article and a planographic printing plate obtained by using the ink composition.

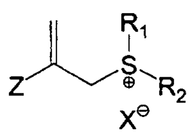 (I) 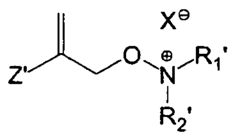 (II)

US 6,090,865 relates to compounds of formula (I) which are useful as photoinitiators, especially in combination with borate anions.

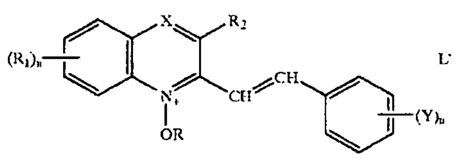

EP-A-0 373 862 describes photopolymerizable polyvinyl alcohols including a group of the formula shown below, and to photo-curable compositions for producing screen printing stencils including such polyvinyl alcohol derivatives.

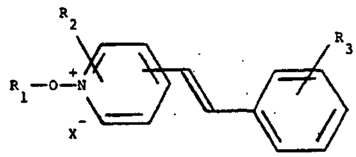

Finally, I. Reetz et al., Polymer International, 43, 27-32 (1997) is a scientific article relating to addition-fragmentation reactions for cationic polymerization using an allyloxy-piclinium salt of the following formula as a cationic polymerization initiator.

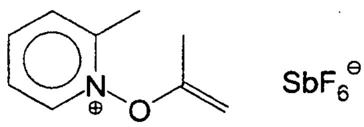

## SUMMARY OF THE INVENTION

An object of the present invention is to provide a lithographic printing plate precursor of the on-press development type which is excellent in the printing durability and on-press development property and a lithographic printing method using the same. Another object of the invention is to provide a lithographic printing plate precursor of the on-press development type which is capable of forming a print-out image having a good plate inspection property by laser exposure and a lithogaphic printing method using the same.

Thus, the present invention provides a lithographic printing plate precursor comprising an image-recording layer which is capable of being removed with at least one of printing ink and dampening water and contains a compound of formula (Ia), a sensitizing dye and a compound having at least one addition-polymerizable ethylenically unsaturated bond:

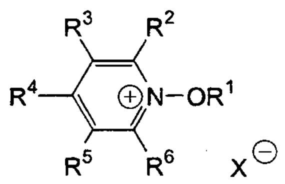

(Ia)

wherein

$R^1$ is a monovalent substituent,

$R^2$-$R^6$ each independently are H, halogen or a monovalent substituent, and

$X^e$ is an inorganic anion or a counter anion of a strong acid;

and at least one of $R^1$-$R^6$ is a polymerizable group selected from the formulae (2) - (4) : ,

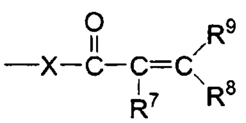

(2)

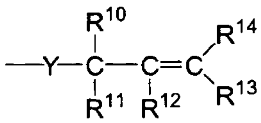

(3)

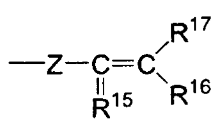

(4)

wherein X, Y, and Z each are a single bond or an organic connecting group, and $R^7$-$R^{17}$ each independently are H or a monovalent substituent.

Also, the present invention provides a lithographic printing method comprising the steps of:

- imagewise exposing the lithographic printing plate precursor of the present invention with an IR-laser; and
- supplying oily ink and an aqueous component to the exposed precursor to perform printing without carrying out a

development processing, wherein the area of the precursor unexposed to the IR-laser is removed during the printing step.

Preferred embodiments of the present invention are as defined in the following description and/or in the appended dependent claims.
According to the lithographic printing plate precursor of the invention, an image having a good plate inspection property (visibility) and a high strength by laser exposure can be formed.
The compound represented by formula (Ia) for use in the invention has an azinium salt structure and generates a radical by laser exposure or heating to initiate or proceed a polymerization reaction of a compound having at least one addition-polymerizable ethylenically unsaturated bond, thereby forming an image having high strength.
On the other hand, the color formation mechanism by laser exposure is not quite clear, but it is believed to be as follows:

In the case of using an infrared absorbing agent as a sensitizing dye, an electron transfer occurs from the infrared absorbing agent excited by infrared laser exposure to the compound represented by formula (Ia) to generate the polymerization initiation ability and based on the electron transfer, the infrared absorbing agent simultaneously undergoes change of the structure to cause change in color. By using the compound represented by formula (Ia), the electron transfer from the excited infrared absorbing agent efficiently initiates or proceeds so that large change in color can be obtained to achieve the excellent plate inspection property.

According to the present invention, a lithographic printing plate precursor of the on-press development type which is excellent in the printing durability and on-press development property and a lithogaphic printing method can be provided. Also, according to the invention, a lithographic printing plate precursor of the on-press development type which is capable of forming a print-out image having a good plate inspection property by laser exposure and a lithographic printing method can be provided.

## DETAILED DESCRIPTION OF THE INVENTION

The lithographic printing plate precursor according to the invention will be described in more detail below.

[Image-recording layer]

The lithographic printing plate precursor according to the invention is characterized by containing (A) a compound represented by formula (Ia) shown below, (B) a sensitizing dye and (C) a compound having at least one addition-polymerizable ethylenically unsaturated bond (hereinafter, also referred to as a polymerizable compound) in the image-recording layer.
The image-recording layer is a so-called image-recording layer capable of undergoing on-press development, which can be recorded by laser beam irradiation and is subjected to a printing procedure after the recording of image without undergoing any wet development processing step to conduct printing, whereby the unexposed area thereof is removed with an oily component and/or hydrophilic component, for example, ink and dampening water, in the course of printing.
In the image-recording layer, (B) the sensitizing dye is preferably an infrared absorbing agent. Such an image-recording layer is capable of being recorded by infrared ray irradiation and is an image-recording layer capable of undergoing on-press development without undergoing any wet development processing step after the recording of image by the infrared ray exposure. More preferably, the image-recording layer according to the invention contains (E) a binder polymer.
Each of the constituting components of the image-recording layer according to the invention will be described in greater detail below.

<(A) Compound represented by formula (Ia)>

First, the compound represented by formula (Ia) is described below.

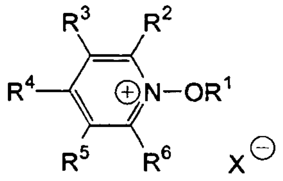

(Ia)

In formula (Ia) $R^1$ represents a monovalent substituent, $R^2$-$R^6$ each independently represent H, a halogen atom or a monovalent substituent. As the halogen atom fluorine, chlorine, bromine an iodine are exemplified, and among them chlorine and bromine are preferable. $X^{\ominus}$ is an inorganic anion or a counter anion of a strong acid.

Also, at least one of $R^1$-$R^6$ is a polymerizable group selected from the formulae (2)-(4):

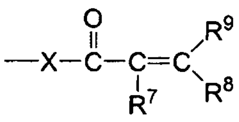

(2)

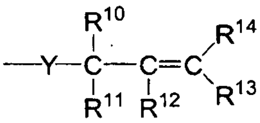

(3)

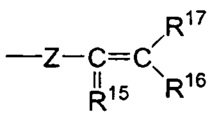

(4)

wherein X, Y, and Z each are a single bond or an organic connecting group, and $R^7$-$R^{17}$ each independently are H or a monovalent substituent.

The monovalent substituent represented by any one of $R^1$-$R^6$ is described below.

In the case where $R^1$ represents a monovalent substituent, the monovalent substituent includes, for example, a substituted carbonyl group, a silyl group, an alkyl group, an alkenyl group, an alkynyl group, an aryl group, a heterocyclic group, a sulfo group, a substituted sulfonyl group, a sulfonato group, a substituted sulfinyl group, a phosphono group, a substituted phosphono group, a phosphonato group and a substituted phosphonato group, and when it is possible to introduce a substituent, the substituent may further be introduced.

In the case where any one of $R^2$ to $R^6$ represents a monovalent substituent, the monovalent substituent includes, for example, an amino group, a substituted amino group, a substituted carbonyl group, a hydroxy group, a substituted oxy group, a mercapto group, a thioether group, a silyl group, a nitro group, a cyano group, an alkyl group, an alkenyl group, an alkynyl group, an aryl group, a heterocyclic group, a sulfo group, a substituted sulfonyl group, a sulfonato group, a substituted sulfinyl group, a phosphono group, a substituted phosphono group, a phosphonato group and a substituted phosphonato group, and when it is possible to introduce a substituent, the substituent may further be introduced.

The alkyl group represented by any one of $R^1$ to $R^6$ includes a straight-chain, branched or cyclic alkyl group having from 1 to 20 carbon atoms. Of the alkyl groups, a straight-chain alkyl group having from 1 to 12 carbon atoms, a branched alkyl group having from 3 to 12 carbon atoms and a cyclic alkyl group having from 5 to 12 carbon atoms are preferred. Specific examples thereof include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a hexadecyl group, an octadecyl group, an eicosyl group, an isopropyl group, an isobutyl group, a sec-butyl group, a tert-butyl group, an isopentyl group, a neopentyl group, a 1-methylbutyl group, an isohexyl group, a 2-ethylhexyl group, a 2-methylhexyl group, a cyclohexyl group, a cyclopentyl group and a 2-norbomyl group.

When the alkyl group represented by any one of $R^1$ to $R^6$ has a substituent (that is, in case of a substituted alkyl group), an alkyl moiety in the substituted alkyl group includes a divalent organic residue obtained by eliminating any one of hydrogen atoms on the alkyl group having from 1 to 20 carbon atoms described above. Also, the range of preferable number of carbon atoms is same as that of the alkyl group described above.

Specific preferable examples of the substituted alkyl group include a chloromethyl group, a bromomethyl group, a 2-chloroethyl group, a trifluoromethyl group, a methoxymethyl group, a methoxycarbonylmethyl group, an isopropoxymethyl group, a butoxymethyl group, a sec-butoxybutyl group, a methoxyethoxyethyl group, an allyloxymethyl group, a phenoxymethyl group, an acetyloxymethyl group, a methylthiomethyl group, a tolylthiomethyl group, a pyridylmethyl group, a tetramethylpyridinylmethyl group, an N-acetyltetramethylpyridinylmethyl group, a trimethylsilylmethyl group, a methoxyethyl group, an ethylaminoethyl group, a diethylaminopropyl group, a morpholinopropyl group, an acetyloxymethyl group, a benzoyloxymethyl group, an N-cyclohexylcarbamoyloxyethyl group, an N-phenylcarbamoyloxyethyl group, an acetylaminoethyl group, an N-methylbenzoylaminopropyl group, a 2-oxoethyl group, a 2-oxopropyl group, a carboxypropyl group, a methoxycarbonylethyl group, an allyloxycarbonylbutyl group, a chlorophenoxycarbonylmethyl group, a carbamoylmethyl group, an N-methylcarbamoylethyl group, an N,N-dipropylcarbamoylmethyl group, an N-(methoxyphenyl)carbamoylethyl group, an N-methyl-N-(sulfophenyl)carbamoylmethyl, a sulfobutyl group, a sulfonatobutyl group, a sulfamoylbutyl group, an N-ethylsulfamoylmethyl group, an N,N-dipropylsulfamoylpropyl group, an N-tolylsulfamoylpropyl group, an N-methyl-N-(phosphonophenyl)sulfamoyloctyl group, a phosphonobutyl group, a phosphonatohexyl goup, a diethylphosphonobutyl group, a diphenylphosphonopropyl group, a methylphosphonobutyl group, a methylphosphonatobutyl group, a tolylphosphonohexyl group, a tolylphosphonatohexyl, a phosphonoxypropyl group, a pbosphonatoxybutyl group, a benzyl group, a phenethyl group, an $\alpha$-methylbenzyl group, a 1-methyl-1-phenylethyl group and a p-methylbenzyl group.

Examples of the substituent capable of being introduced into the alkyl group represented by any one of $R^1$ to $R^6$ include a monovalent substituent constituting from a non-metallic atom illustrated below in addition to the substituents described in the substituted alkyl group. Preferable examples of the substituent for the alkyl group including the substituents described above include a halogen atom (e.g., -F, -Br, -Cl or -I), a hydroxy group, an alkoxy group, an aryloxy group, a mercapto group, an alkylthio group, an arylthio group, an alkyldithio group, an aryldithio group, an amino group, an N-alkylamino group, an N,N-dialkylamino group, an N-arylamino group, an N,N-diarylamino group, an N-alkyl-N-arylamino group, an acyloxy group, a carbamoyloxy group, an N-alkylcarbamoyloxy group, an N-arylcarbamoyloxy group, an N,N-dialkylcarbarmoyloxy group, an N,N-diarylcarbamoyloxy group, an N-alkyl-N-arylcarbamoyloxy group, an alkylsulfoxy group, an arylsulfoxy group, an acylthio group, an acylamino group, an N-alkylacylamino group, an N-arylacylamino group, a ureido group, an N'-alkylureido group, an N',N'-dialkylureido group, N'-arylureido group, an N',N'-diarylureido group, an N'-alkyl-N'-arylureido group, an N-alkylureido group, N-arylureido group, an N'-alkyl-N-alkylureido group, an N'-alkyl-N-arylureido group, an N',N'-dialkyl-N-alkylureido group, an N',N'-dialkyl-N-arylureido group, an N'-aryl-N-alkylureido group, an N'-aryl-N-arylureido group, an N',N'-diaryl-N-alkylureido group, an N',N'-diaryl-N-arylureido group, an N'-alkyl-N'-aryl-N-alkylureido group, an N'-alkyl-N'-aryl-N-arylureido group,

an alkoxycarbonylamino group, an aryloxycarbonylamino group, an N-alkyl-N-alkoxycarbonylamino group, an N-alkyl-N-aryloxycarbonylamino group, an N-aryl-N-alkoxycarbonylamino group, an N-aryl-N-aryloxycarbonylamino group, a formyl group, an acyl group, a carboxyl group, an alkoxycarbonyl group, an aryloxycarbonyl group, a carbamoyl group, an N-alkylcarbamoyl group, an N,N-dialkylcarbamoyl group, an N-arylcarbamoyl group, an N,N-diarylcarbamoyl group, an N-alkyl-N-arylcarbamoyl group, an alkylsulfinyl group, an arylsulfinyl group, an alkylsulfonyl group, an arylsulfonyl group, a sulfo group ($-SO_3H$) and a conjugate base group thereof (hereinafter, referred to as a sulfonato group), an alkoxysulfonyl group, an aryloxysulfonyl group, a sulfinamoyl group, an N-alkylsulfinamoyl group, an N,N-dialkylsulfinamoyl group, an N-arylsulfinamoyl group, an N,N-diarylsulfinamoyl group, an N-alkyl-N-arylsulfinamoyl group, a sulfamoyl group, an N-alkylsulfamoyl group, an N,N-dialkylsulfamoyl group, an N-arylsulfamoyl group, an N,N-diarylsulfamoyl group, an N-alkyl-N-arylsulfamoyl group,

a phosphono group ($-PO_3H_2$) and a conjugate base group thereof (hereinafter, referred to as a phosphonato group), a dialkylphosphono group ($-PO_3(alkyl)_2$) wherein "alkyl" means an alkyl group, hereinafter the same, a diarylphosphono group ($-PO_3(aryl)_2$) wherein "aryl" means an aryl group, hereinafter the same, an alkylarylphosphono group ($-PO_3(alkyl)(aryl)$), a monoalkylphosphono group ($-PO_3H(alkyl)$) and a conjugate base group thereof (hereinafter, referred to as an alkylphosphonato group), a monoarylphosphono group ($-PO_3H(aryl)$) and a conjugate base group thereof (hereinafter, referred to as an arylphosphonato group), a phosphonoxy group ($-OPO_3H_2$) and a conjugate base group thereof (hereinafter, referred to as a phosphonatoxy group), a dialkylphosphonoxy group ($-OPO_3(alkyl)_2$), a diarylphosphonoxy group ($-OPO_3(aryl)_2$), an alkylarylphosphonoxy group ($-OPO_3(alkyl)(aryl)$), a monoalkylphosphonoxy group ($-OPO_3H(alkyl)$) and a conjugate base group thereof (hereinafter, referred to as an alkylphosphonatoxy group), a monoarylphosphonoxy group ($-OPO_3H(aryl)$) and a conjugate base group thereof (hereinafter, referred to as an arylphosphonatoxy group), a cyano group, a nitro group, an aryl group, an alkenyl group, an alkynyl group, a heterocyclic group and a silyl group.

Specific examples of the alkyl moiety in the substituent capable of being introduced into the alkyl group represented by any one of $R^1$ to $R^6$ are same as those described in the case where the monovalent substituent represented by any one of $R^1$ to $R^6$ is the substituted alkyl group. Also, the range of preferable number of carbon atoms is same as that of the alkyl group described above.

Also, specific examples of the aryl moiety in the substituent capable of being introduced into the alkyl group represented by any one of $R^1$ to $R^6$ include a phenyl group, a biphenyl group, a naphthyl group, a tolyl group, a xylyl group, a mesityl group, a cumenyl group, a chlorophenyl group, a bromophenyl group, a chloromethylphenyl group, a hydroxyphenyl group, a methoxyphenyl group, an ethoxyphenyl group, a phenoxypenyl group, an acetoxyphenyl group, a benzoyloxyphenyl group, a methylthiophenyl group, a phenylthiophenyl group, a methylaminophenyl group, a dimethylaminophenyl group, an acetylaminophenyl group, a carboxyphenyl group, a methoxycarbonylphenyl group, an ethoxycarbonylphenyl group, a phenoxycarbonylphenyl group, an N-phenylcarbamoylphenyl group, a cyanophenyl group, a sulfophenyl group, a sufonatophenyl group, a phosphonophenyl group and a phosphonatophenyl group.

The alkenyl group represented by any one of $R^1$ to $R^6$ includes an alkenyl group having from 2 to 20 carbon atoms. Of the alkenyl groups, an alkenyl group having from 2 to 10 carbon atoms is preferable, and alkenyl group having from 2 to 8 carbon atoms is more referable. The alkenyl group may have a substituent. Examples of the substituent capable of being introduced include a halogen atom, an alkyl group, a substituted alkyl group, an aryl group and a substituted aryl group, and preferably a halogen atom and a straight-chain, branched or cyclic alkyl group having from

1 to 10 carbon atoms. Specific examples of the alkenyl group include a vinyl group, a 1-propenyl group, a 1-butenyl group, a cinnamyl group, a 1-pentenyl group, a 1-hexenyl group, a 1-octenyl group, a 1-methyl-1-propenyl group, a 2-methyl-1-propenyl group, 2-methyl-1-butenyl group, a 2-phenyl-1-ehtenyl group, a 2-chloro-1-ethenyl group, an allyl group, a 2-butenyl group, a 2-methylallyl group, a 2-methyl-3-butenyl group and a 3-methyl-2-butenyl group.

The alkynyl group represented by any one of $R^1$ to $R^6$ includes an alkynyl group having from 2 to 20 carbon atoms. Of the alkynyl groups, an alkynyl group having from 2 to 10 carbon atoms is preferable, and alkynyl group having from 2 to 8 carbon atoms is more referable. Specific examples of the alkynyl group include an ethynyl group, a 1-propynyl group, a 1-butynyl group, a phenylethynyl group and a trimethylsilylethynyl group, 2- propynyl group, a 2-butynyl group or a 3-butynyl group.

The aryl group represented by any one of $R^1$ to $R^6$ includes a benzene ring group, a condensed ring group of two to three benzene rings and a condensed ring group of a benzene ring and a 5-membered unsaturated ring. Specific examples of the aryl group include a phenyl group, a naphthyl group, an anthryl group, a phenanthryl group, an indenyl group, an acenaphthenyl group and a fluorenyl group. Among them, a phenyl group and a naphthyl group are preferable.

Also, the aryl group represented by any one of $R^1$ to $R^6$ may have a substituent on the carbon atom forming the ring. Such a substituent includes a monovalent substituent constituting from a non-metallic atom. Preferable examples of the substituent capable of being introduced include the above-described alkyl group and substituted alkyl group and those described for the substituents of the substituted alkyl group.

The heterocyclic group represented by any one of $R^1$ to $R^6$ is preferably a 3-membered to 8-membered heterocyclic group, more preferably a 3-membered to 6-membered heterocyclic group containing a nitrogen atom, an oxygen atom or a sulfur atom, and still more preferably a 5-membered to 6-membered heterocyclic group containing a nitrogen atom, an oxygen atom or a sulfur atom. Specific examples of the heterocyclic group include a pyrrole ring group, a furan ring group, a thiophene ring group, a benzopyrrole ring group, a benzofuran ring group, a benzothiophene ring group, a pyrazole ring group, an isoxazole ring group, an isothiazole ring group, an indazole ring, a benzisoxazole ring group, a benzisothiazole ring group, an imidazole group, an oxazole ring group, a thiazole ring group, a benzimidazole group, a benzoxazole ring group, a benzothiazole ring group, a pyridine ring group, a quinoline ring group, an isoquinoline ring group, a pyridazine ring group, a pyrimidine ring group, a pyrazine ring group, a phthalazine ring group, a quinazoline ring group, a quinoxaline ring group, an aciridine ring group, a phenanthrydine ring group, a carbazole ring group, a purine ring group, a pyrane ring group, a piperidine ring group, a piperazine ring group, a morpholine ring group, an indole ring group, an indolizine ring group, a chromene ring group, a cinnnoline ring group, an acridine ring group, a phenothiazine ring group, a tetrazole ring group and a triazine ring group.

Also, the heterocyclic group represented by any one of $R^1$ to $R^6$ may have a substituent on the carbon atom forming the ring. Such a substituent includes a monovalent substituent constituting from a non-metallic atom. Preferable examples of the substituent capable of being introduced include the above-described alkyl group and substituted alkyl group and those described for the substituents of the substituted alkyl group.

The silyl group represented by any one of $R^1$ to $R^6$ may have a substituent, and a silyl group having from 0 to 30 carbon atoms is preferable, a silyl group having from 3 to 20 carbon atoms is more preferable, and a silyl group having from 3 to 10 carbon atoms is still more preferable. Specific examples of the silyl group include a trimethylsilyl group, a triethylsilyl group, a tripropylsilyl group, a triisopropylsilyl group, a cyclohexyldimethylsilyl group and a dimethylvinylsilyl group.

The thioether group represented by any one of $R^2$ to $R^6$ may have a substituent, and a thioether group having from 0 to 30 carbon atoms is preferable, a thioether group having from 3 to 20 carbon atoms is more preferable, and a thioether group having from 3 to 10 carbon atoms is still more preferable.

Specific examples of the thioether group include an alkyl thio goup, for example, a methylthio group, an ethylthio group or a cylohexylthio group and an aryl thio group, for example, a phenylthio group.

As the substituted oxy group ($R^{06}O$-) represented by any one of $R^2$ to $R^6$, a substituted oxy group wherein $R^{06}$ represents a monovalent non-metallic atomic group exclusive of a hydrogen atom can be used. Preferable examples of the substituted oxy group include an alkoxy group, an aryloxy group, an acyloxy group, a carbamoyloxy group, an N-alkylcarbamoyloxy group, an N-arylcarbamoyloxy group, an N,N-dialkylcarbamoyloxy group, an N,N-diarylcarbamoyloxy group, an N-alkyl-N-arylcarbamoyloxy group, an alkylsulfoxy group, an arylsulfoxy group, a phosphonoxy group and a phosphonatoxy group. The alkyl group and aryl group in the above-described substituted oxy group include those described for the alkyl group, substituted alkyl group, aryl group and substituted aryl group above. As an acyl group ($R^{07}CO$-) in the acyloxy group, an acyl group wherein $R^{07}$ represents the alkyl group, substituted alkyl group, aryl group or substituted aryl group described above is exemplified. Of the substituted oxy groups, an alkoxy group, an aryloxy group, an acyloxy group and an arylsulfoxy group are more preferable. Specific preferable examples of the substituted oxy group include a methoxy group, an ethoxy group, a propyloxy group, an isopropyloxy group, a butyloxy group, a pentyloxy group, a hexyloxy group, a dodecyloxy group, a benzyloxy group, an allyloxy group, a phenethyloxy group, a carboxyethyloxy group, a methoxycarbonylethyloxy goup, an ethoxycarbonylethyloxy group, a methoxyethoxy group, a phenoxyethoxy group, a methoxyethoxyethoxy group, an ethoxyethoxyethoxy group, a morpholinoethoxy group, a morpholinopropyloxy group, an allyloxyethoxyethoxy group, a phenoxy group, a tolyloxy group, a xylyloxy group, a mesityloxy group, a mesityloxy group, a cumenyloxy group, a methoxyphenyloxy group, an ethoxyphenyloxy group, a chlorophenyloxy group, a bromophenyloxy group, an acetyloxy goup, a benzoyloxy group, a naphthyloxy group, a phenylsulfonyloxy group, a phosphonoxy group and a phosphonatoxy group.

The amino group represented by any one of $R^2$ to $R^6$ may be a substituted amino group including an amido group. As the substituted amino group including an amido group ($R^{08}NH$- or $(R^{09})(R^{010})N$-), a substituted amino group wherein $R^{08}$, $R^{09}$ and $R^{010}$ each represents a monovalent non-metallic atomic group exclusive of a hydrogen atom can be used. $R^{09}$ and $R^{010}$ may be connected with each other to form a ring. Preferable examples of the substituted amino group include an N-alkylamino group, an N,N-dialkylamino group, an N-arylamino group, an N,N-diarylamino group, an N-alkyl-N-arylamino goup, an acylamino group, an N-alkylacylamino group, an N-arylacylamino group, a ureido group, an N'-alkylureido group, an N',N'-dialkylureido group, an N'-arylureido group, an N',N'-diarylureido group, an N'-alkyl-N'-arylureido group, an N-alkylureido group, an N-arylureido group, an N'-alkyl-N-alkylureido group, an N'-alkyl-N-arylureido group, an N',N'-dialkyl-N-alkylureido group, an N'-alkyl-N'-alylureido group, an N',N'-dialkyl-N-alkylureido group, an N',N'-dialkyl-N'-arylureido group, an N'-aryl-N-alkylureido group, an N'-aryl-N-arylureido group, an N',N'-diaryl-N-alkylureido goup, an N',N'-diaryl-N-arylureido group, an N'-alkyl-N'-aryl-N-alkylureido group, an N'-alkyl-N'-aryl-N-arylureido group, an alkoxycarbonylamino group, an aryloxycarbonylamino group, an N-alkyl-N-alkoxycarbonylamino group, an N-alkyl-N-aryloxycarbonylamino group, an N-aryl-N- alkoxycarbonylamino group and an N-aryl-N-aryloxycarbonylamino group. The alkyl group and aryl group in the above-described substituted amino group include those described for the alkyl group, substituted alkyl group, aryl group and substituted aryl group above. In the acyl group ($R^{07}CO$-) of the acylamino group, N-alkylacylamino group or N-arylacylamino group described above, $R^{07}$ has the same meaning as described above. Of the substituted amino groups, an N-alkylamino group, an N,N-dialkylamino group, an N-arylamino group and an acylamino group are more preferable. Specific preferable examples of the substituted amino group include a methylamino group, an ethylamino group, a diethylamino group, a morpholino group, a piperidino group, a pyrrolidino group, a phenylamino group, a benzoylamino group and an acetylamino group.

As the substituted sulfonyl group ($R^{011}$-$SO_2$-) represented by any one of $R^1$ to $R^6$, a substituted sulfonyl group wherein $R^{011}$ represents a monovalent non-metallic atomic group can be used. Preferable examples of the substituted sulfonyl group include an alkylsulfonyl group, an arylsulfonyl group and a substituted or unsubstituted sulfamoyl group. The alkyl group and aryl group in the above-described substituted sulfonyl group include those described for the alkyl group, substituted alkyl group, aryl group and substituted aryl group above. Specific examples of the substituted sulfonyl group include a butylsulfonyl group, a phenylsulfonyl group, a chlorophenylsulfonyl group, a sulfamoyl group, an N-alkylsulfamoyl group, an N,N-dialkylsulfamoyl group, an N-arylsulfamoyl group and an N-alk-yl-N-arylsulfamoyl group.

The sulfonato group (-$SO_3$-) represented by any one of $R^1$ to $R^6$ means a conjugate base anionic group of a sulfo group (-$SO_3H$) as described above. Ordinarily, it is preferred to use together with a counter cation. Examples of the counter cation include those conventionally known, for example, various oniums (e.g., an ammonium, a sufonium, a phosphonium, an iodonium or an azinium) and metal ions (e.g., $Na^+$, $K^+$, $Ca^{2+}$ or $Zn^{2+}$).

As the substituted carbonyl group ($R^{013}$-CO-) represented by any one of $R^1$ to $R^6$, a substituted carbonyl group wherein $R^{013}$ represents a monovalent non-metallic atomic group can be used. Preferable examples of the substituted carbonyl group include a formyl group, an acyl group, a carboxyl group, an alkoxycarbonyl group, an aryloxycarbonyl group, a carbamoyl group, an N-alkylcarbamoyl group, an N,N-dialkylcarbamoyl group, an N-arylcarbamoyl group, an N,N-diarylcarbamoyl group and an N-alkyl-N-arylcarbamoyl group. The alkyl group and aryl group in the above-described substituted carbonyl group include those described for the alkyl group, substituted alkyl group, aryl group and substituted aryl group above. Of the substituted carbonyl groups, a formyl group, an acyl group, a carboxyl group, an alkoxycarbonyl group, an aryloxycarbonyl group, a carbamoyl group, an N-alkylcarbamoyl group, an N,N-dialkylcarbamoyl group and an N-arylcarbamoyl group are more preferable, and a formyl group, an acyl group, an alkoxycarbonyl group and an aryloxycarbonyl group are still more preferable. Specific preferable examples of the substituted carbonyl group include a formyl group, an acetyl group, a benzoyl group, a carboxyl group, a methoxycarbonyl group, an ethoxycarbonyl group, an allyloxycarbonyl group, a dimethylaminophenylethenylcarbonyl group, a methoxycarbonylmethoxycarbonyl group, an N-methylcarbamoyl group, an N-phenylcarbamoyl group, an N,N-diethylcarbamoyl group and a morpholinocarbonyl group.

As the substituted sulfinyl croup ($R^{014}$-SO-) represented by any one of $R^1$ to $R^6$, a substituted sulfinyl group wherein $R^{014}$ represents a monovalent non-metallic atomic group. Preferable examples of the substituted sulfinyl group include an alkylsulfinyl group, an arylsulfinyl group, a sulfinamoyl group, an N-alkylsulfinamoyl group, an N,N-dialkylsulfinamoyl group, an N-arylsulfinamoyl group, an N,N-diaxylsulfinamoyl group and an N-alkyl-N-arylsulfinamoyl group. The alkyl group and aryl group in the above-described substituted sulfinyl group include those described for the alkyl group, substituted alkyl group, aryl group and substituted aryl group above. Of the substituted sulfinyl groups, an alkylsulfinyl group and an arylsulfinyl group are more preferred. Specific examples of the substituted sulfinyl group include a hexylsulfinyl group, a benzylsulfinyl group and a tolylsulfinyl group.

The substituted phosphono group represented by any one of $R^1$ to $R^6$ means a group formed by substituting

one or two hydroxy groups of a phosphono group with one or two other organic oxy groups. Preferable examples of the substituted phosphono group include a dialkylphosphono group, a diarylphosphono group, an alkylarylphosphono group, a monoalkylphosphono group and a monoarylphosphono group as described above. Of the substituted phosphono groups, a dialkylphosphono group and a diarylphosphono group arc more preferred. Specific examples of the substituted phosphono group include a diethylphosphono group, a dibutylphosphono group and a diphenylphosphono group.

The phosphonato group ($-PO_3H^-$ or $-PO_3^{2-}$) represented by any one of $R^1$ to $R^6$ means a conjugate base anionic group of a phosphono group ($-PO_3H_2$) resulting from primary acid dissociation or secondary acid dissociation. Ordinarily, it is preferred to use together with a counter cation. Examples of the counter cation include those conventionally know for example, various oniums (e.g., an ammonium, a sulfonium, a phosphonium, an iodonium or an azinium) and metal ions (e.g., $Na^+$, $K^+$, $Ca^{2+}$ or $Zn^{2+}$).

The substituted phosphonato group represented by any one of $R^1$ to $R^6$ means a conjugate base anionic group of a group formed by substituting one hydroxy group with another organic oxy group among the substituted phosphono groups described above. Specific examples of the substituted phosphonato group include a conjugate base group of a monoalkylphosphono group ($-PO_3H$(alkyl)) and a conjugate base group of a monoarylphosphono group ($-PO_3H$(aryl)).

Further, $R^1$ to $R^6$ may be combined with each other to form a fused benzene ring or heterocyclic ring together with the carbon atoms to which $R^1$ to $R^6$ are connected.

In formula (Ia), at least one of $R^1$ to $R^6$ is a polymerizable substituent (polymerizable group), and the polymerizable group contributes to increase in the crosslinkage density of the image-recording layer after imagewise exposure.

The polymerizable group is an addition-polymerizable group selected from groups represented by the following formulae (2), (3) and (4):

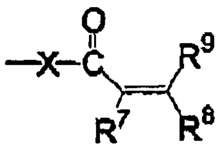

(2)

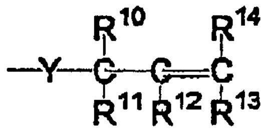

(3)

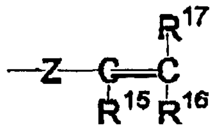

(4)

In the formulae, X, Y and Z each represents a single bond or an organic connecting group, and $R^7$ to $R^{17}$ each independently represents a hydrogen atom or a monovalent substituent.

In formula (2), $R^7$ to $R^9$ each independently represents a hydrogen atom or a monovalent substituent. The monovalent substituent represented by $R^7$ includes, for example, an alkyl group which may have a substituent. Among them, $R^7$ is preferably a hydrogen atom, a methyl group, a methoxy group or a methoxycarbonyl group. $R^8$ and $R^9$ each independently includes, for example, a hydrogen atom, a halogen atom, an amino group, a dialkylamino group, a carboxyl group, an alkoxycarbonyl group, a sulfo group, a nitro group, a cyano group, an alkyl group which may have a substituent, an aryl group which may have a substituent, an alkoxy group which may have a substituent, an aryloxy group which may have a substituent, an alkylamino group which may have a substituent, an arylamino group which may have a substituent, an alkylsulfonyl group which may have a substituent and an arylsulfonyl group which may have a substituent. Among them, a hydrogen atom, a carboxyl group, an alkoxycarbonyl group, an alkyl group which may have a substituent or an aryl group which may have a substituent is preferable. The substituent capable of being introduced includes, for example, a methoxycarbonyl group, an ethoxycarbonyl group, an isopropoxycarbonyl group, a methyl group, an ethyl group and a phenyl group.

The organic connecting group represented by X is preferably an organic group constituting from 1 to 30 carbon atoms, preferably from 1 to 10 carbon atoms, from 0 to 10 nitrogen atoms, preferably from 0 to 5 nitrogen atoms, from 0 to 20 oxygen atoms, preferably from 0 to 10 oxygen atoms, from 1 to 50 hydrogen atoms, preferably from 1 to 20 hydrogen atoms, and from 0 to 10 sulfur atoms, preferably from 0 to 5 sulfur atoms. The organic connecting group may be divalent or more. Specific examples thereof include organic connecting groups constituting individually or in combination from structures shown below.

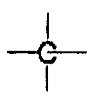 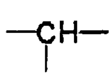 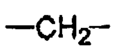 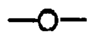 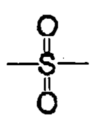

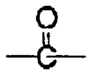 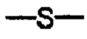 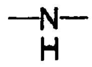 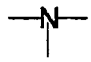 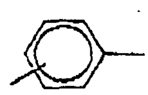

In formula (3), $R^{10}$ to $R^{14}$ each independently represents a hydrogen atom or a monovalent substituent. The monovalent substituent includes, for example, a halogen atom, an amino group, a dialkylamino group, a carboxyl group, an alkoxycarbonyl group, a sulfo group, a nitro group, a cyano group, an alkyl group which may have a substituent, an aryl group which may have a subsbtuent, an alkoxy group which may have a substituent an aryloxy group which may have a substituent, an alkylamino group which may have a substituent, an arylamino group which may have a substituent, an alkylsulfonyl group which may have a substituent and an arylsulfonyl group which may have a substituent Among them, a hydrogen atom, a carboxyl group, an alkoxycarbonyl group, an alkyl group which may have a substituent or an aryl group which may have a substituent is preferable for each of $R^{10}$ to $R^{14}$. Examples of the substituent capable of being introduced include those described in Formula (2). Y has the same meaning as Xdefined in Formula (2).

In formula (4), $R^{15}$ to $R^{17}$ each independently represents a hydrogen atom or a monovalent substituent. The monovalent substituent includes, for example, a halogen atom, an amino group, a dialkylamino group, a carboxyl group, an alkoxycarbonyl group, a sulfo goup, a nitro group, a cyano group, an alkyl group which may have a substituent, an aryl group which may have a substituent, an alkoxy group which may have a substituent, an aryloxy group which may have a substituent, an alkylamino group which may have a substituent, an arylamino group which may have a substituent, an alkylsulfonyl group which may have a substituent and an arylsulfonyl group which may have a substituent. Among them, a hydrogen atom, a carboxyl group, an alkoxycarbonyl group, an alkyl group which may have a substituent or an aryl group is preferable for each of $R^{15}$ to $R^{17}$. Examples of the substituent capable of being introduced include those described in Formula (2). Z has the same meaning as X defined in Formula (2).

In formula (Ia), when each of $R^1$ and $R^2$ to $R^6$ does not represent the polymerizable group, each of $R^1$ and $R^2$ to $R^6$ has the same meaning as each of $R^1$ and $R^2$ to $R^6$ defined in formula (I).

Preferable embodiments of $R^1$ to $R^6$ in formula (Ia) are illustrated below.

$R^1$ is preferably a polymerizable group. When $R^1$ is not a polymerizable group, $R^1$ is preferably an alkyl group having from 1 to 20 carbon atoms which may have a substituent, more preferably an alkyl group having from 1 to 10 carbon atoms which may have a substituent, and still more preferably an alkyl group having from 1 to 5 carbon atoms.

When each of $R^2$ to $R^6$ is not a polymerizable group, each of $R^2$ to $R^6$ is preferably a hydrogen atom, a halogen atom, an alkyl group having from 1 to 20 carbon atoms which may have a substituent, an alkoxy group having from 1 to 20 carbon atoms which may have a substituent, an aromatic group having from 1 to 20 carbon atoms which may have a substituent or a heterocyclic group having from 1 to 20 carbon atoms which may have a substituent. Further, an embodiment wherein $R^2$ and $R^3$, $R^3$ and $R^4$, $R^4$ and $R^5$ or $R^5$ and $R^6$ are combined with each other to form a benzene ring or hetero ring is preferable.

Moreover, in view of the plate inspection property, it is preferable for a skeleton (cation portion) of the specific structure in the compound represented by formula (I) to have a log P value of 2,00 or more, more preferably 3.00 or more, and still more preferably 4.00 or more. The term, "log P value" as used herein means a common logarithm of a partition coefficient P. It is a physical value which indicates how a certain organic compound is partitioned in an equilibrium of two-phase system of oil (ordinarily, 1-octanol) and water as a quantitative numeral.

$$\log P = \log (C_{oil}/C_{water})$$

$C_{oil}$ = molar concentration in oil phase
$C_{water}$ = molar concentration in water phase

The log P value used herein is a value of the skeleton (cation portion) of the specific structure calculated using a log P value estimation program: CLOGP (CLOGP program installed in a system: PC Models (ver 1.02) of Daylight Chemical Information Systems, Inc.: algorism = 4.01, fragment data base = 17) developed by C. Hansch, A. Leo, et al., Medchem project of Pomona Collage and Biobyte Inc. in USA.

The program is based on the Hansch-Leo fragment method and a log P is estimated by dividing a chemical structure into a partial structure (fragment) and summing up a contribution extent of log P assigned to the fragment. The method is described in greater detail in the literature shown below and the description can be referred to.

C. Hansch and A. Leo, Substituent Constants for Correlation Analysis in Chemistry and Biology, John Wiley & Sons, and A. J. Leo, Calculating log Poct from structure, Chem. Rev., Vol. 93, pages 1281 to 1306 (1993).

$X^-$ represents an inorganic anion. Specific examples of which include a halogen atom anion, $BF_4^-$, $BCl_4^-$, $ZnCl_4^-$,

$SbCl_6^-$, $FeCl_4^-$, $GaCl_4^-$, $GaBr_4^-$, Af4", $AlC_4^-$, $SbF_6^-$, $CF_3SO_3^-$, $PF_6^-$, $BPh_4^-$, or a counter anion of a strong acid. From this point of view, $PF_6^-$, $BF_4^-$, $CF_3SO_3^-$ and $C_4F_9SO_3^-$ are preferable, and $PF_6^-$ is most preferable.

The compound represented by formula (Ia) also includes a compound which contains in its molecule two or more of the skeletons (cation portions) of the specific structure in the compound represented by formula (Ia) connected through $R^1$, and such a compound is also preferably used.

As the compound represented by formula (Ia) according to the embodiment in which two or more of the skeletons of the specific structure are included in the molecule, a compound represented by formula (IIa) shown below is particularly preferable. It is believed that since such a compound can generate an alkoxy radical at multiple points of the molecule and form a three-dimensional crosslinkage with (C) a compound having at least one addition-polymerizable ethylenically unsaturated bond described hereinafter, the compound is useful for the formation of the image area excellent in printing durability.

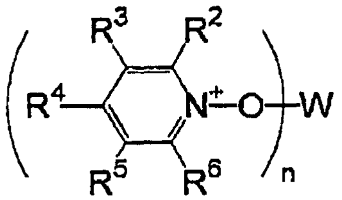

(IIa)

In formula (II), $R^2$, $R^3$, $R^4$, $R^5$ and $R^6$ each independently represents a hydrogen atom, a halogen atom or a monovalent substituent, W represents an n-valent organic connecting group, n represents an integer of 2 or more, and $X^-$ represents an anion.

The monovalent substituent represented by any one of $R^2$ to $R^6$ in formula (IIa) has the same meaning as the monovalent substituent represented by any one of $R^2$ to $R^6$ in formula (Ia) and preferable examples thereof are also same as those described above.

Plural $R^2$s to $R^6$s present in the compound may be the same or different from each other, but it is preferable to be the same in view of synthesis aptitude.

The n-valent organic connecting group represented by W is a multi-valent organic group and preferably a multi-valent organic group constituting from 1 to 60 carbon atoms, from 0 to 10 nitrogen atoms, from 0 to 50 oxygen atoms, from 1 to 100 hydrogen atoms and from 0 to 20 sulfur atoms. Specific examples thereof include organic connecting groups constituting individually or in combination from structures shown below.

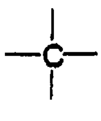 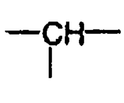 $-CH_2-$ $-O-$ 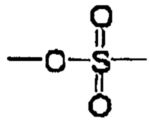

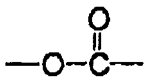 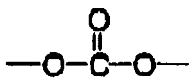 $-S-$ 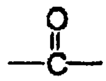 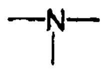

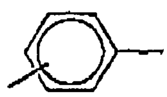 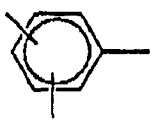 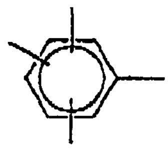 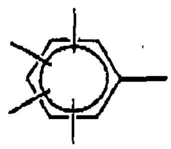

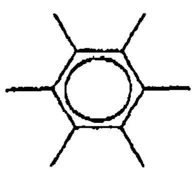 ,

multi-valent naphthalene, multi-valent anthracene

The organic connecting group represented by W may have a substituent. The substituent capable of being

introduced includes, for example, a halogen atom, a hydroxy group, a carboxyl group, a sulfonato group, a nitro group, a cyano group, an amido group, an amino group, an alkyl group, an alkenyl group, an alkynyl group, an aryl group, a substituted oxy group, a substituted sulfonyl group, a substituted carbonyl group, a substituted sulfinyl group, a sulfo group, a phosphono group, a phosphonato group, a silyl group and a heterocyclic group.

The organic connecting group represented by W is more preferably an organic connecting group containing from 1 to 5 oxygen atoms, still more preferably an organic connecting group containing 1 oxygen atom. Most preferable example of the organic connecting group is an organic connecting group having 1 oxygen atom and from 4 to 6 carbon atoms.

n represents an integer of 2 or more. n is preferably from 2 to 6, and more preferably 2 or 3.

$X^-$ in formula (IIa) has the same meaning as $X^-$ in formula (Ia) and preferable examples thereof are also same as those described above.

Moreover, an embodiment in which the compound represented by formula (Ia) is introduced into a polymer side chain through any one of $R^1$ to $R^6$ is also a preferable embodiment included in formula (Ia).

As the embodiment in which the compound represented by formula (Ia) is introduced into a polymer side chain, a compound represented by formula (IVa) shown below is particularly preferable.

It is believed that the compound can form a three-dimensional crosslinkage with the polymerizable compound similar to the compound including two or more of the skeletons of the specific structure through $R^1$ in its molecule described above. It is also believed that since the three-dimensional crosslinkage structure can be formed in higher density by using the polymer having two or more specific structures introduced into its side chains, the polymer compound is preferable from the standpoint of improvement in the sensitivity and printing durability.

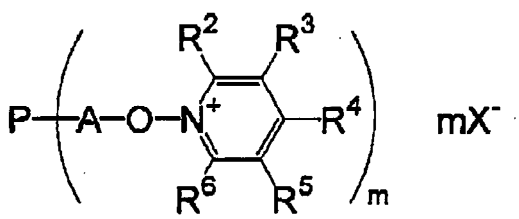

(IVa)

In formula (IVa), $R^2$, $R^3$, $R^4$, $R^5$ and $R^6$ each independently represents a hydrogen atom or a monovalent substituent, provided that at least one of $R^2$ to $R^6$ is a polymerizable group, P represents a polymer main chain, A represents a single bond or a divalent organic connecting group, m represents an integer of 1 or more, and $X^-$ represents an anion.

The monovalent substituent and polymerizable group represented by any one of $R^2$ to $R^6$ in formula (IVa) has the same meaning as those described in formula (Ia) and preferable examples thereof are also same as those described above.

The polymer main chain represented by P is not particularly restricted and preferably includes a polyvinyl chin, a polyurethane chain and a polyacetal chain. Among them, from the standpoint of the printing durability and the like when the polymer compound is applied to an image-recording layer of lithographic printing plate precursor, a poly(methacrylate) chain and a polystyrene chain and the like are particularly preferable.

The divalent organic connecting group represented by A has the same meaning as that defined in formula (IV). The divalent organic connecting group represented by A may have a substituent same as in formula (IV). m represents an integer of 1 or more. m is preferably from 1 to 500, more preferably from 1 to 200, and still more preferably from 1 to 100.

$X^-$ in formula (IVa) has the same meaning as $X^-$ in formula (Ia) and preferable examples thereof are also same as those described above.

Specific examples [Compounds (Aa-1) to (Aa-36)] of the compound represented by formula (Ia), (IIa), (III) or (IVa) are set forth below.

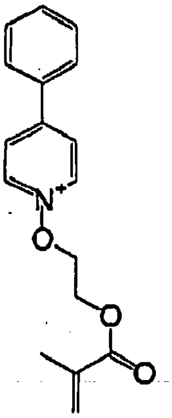

X⁻

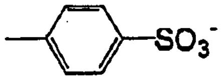 (Aa-1)

$PF_6^-$ (Aa-2)

$ClO_4^-$ (Aa-3)

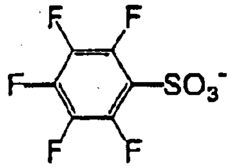 (Aa-4)

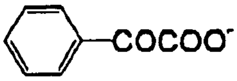 (Aa-5)

$CF_3SO_3^-$ (Aa-6)

BF4⁻ (Aa-7)

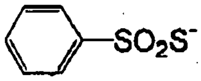 (Aa-8)

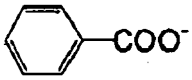 (Aa-9)

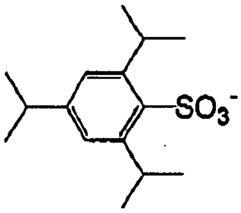 (Aa-10)

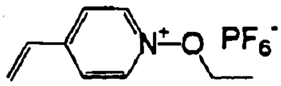 (Aa-11)

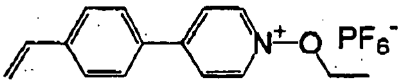 (Aa-12)

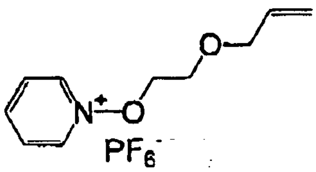 (Aa-13)

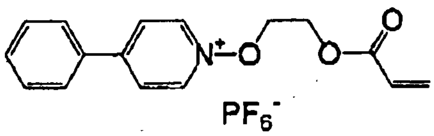 (Aa-14)

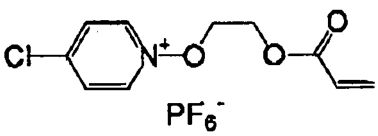 (Aa-15)

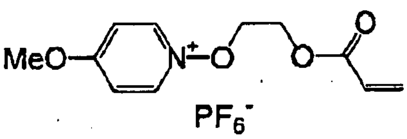 (Aa-16)

(continued)

X-

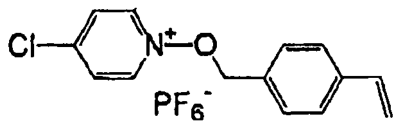

(Aa-17)

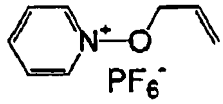

(Aa-18)

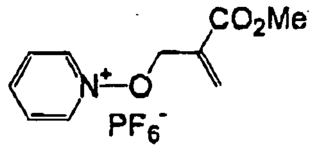

(Aa-19)

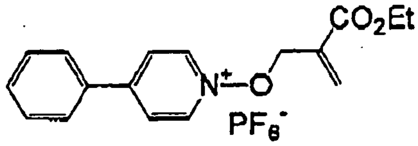

(Aa-20)

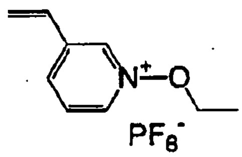

(A-21)

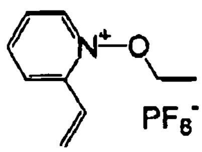

(Aa-22)

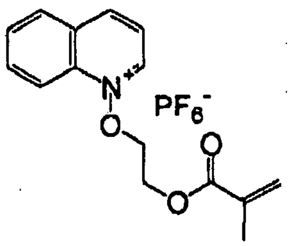

(Aa-23)

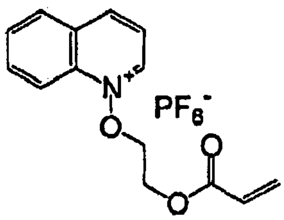

(Aa-24)

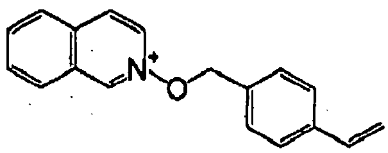

(Aa-25)

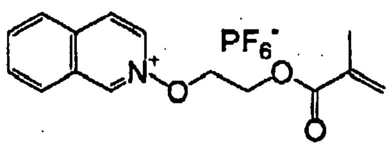

(Aa-26)

(continued)

X-

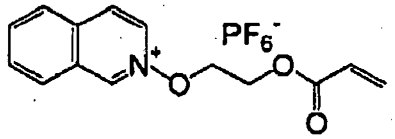

(Aa-27)

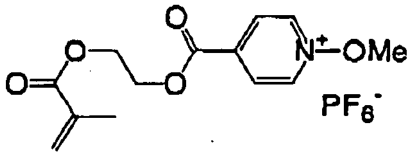

(Aa-28)

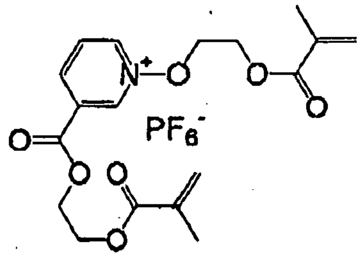

(Aa-29)

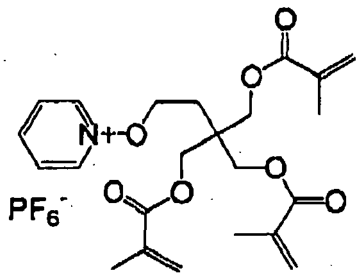

(Aa-30)

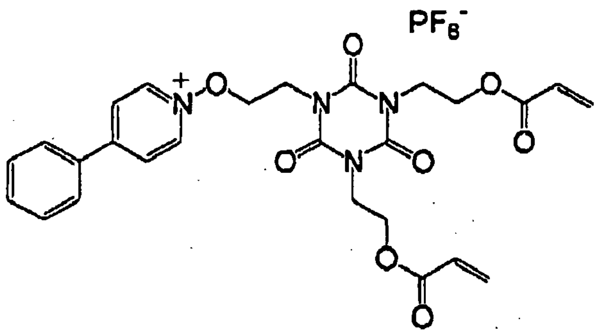

(Aa-31)

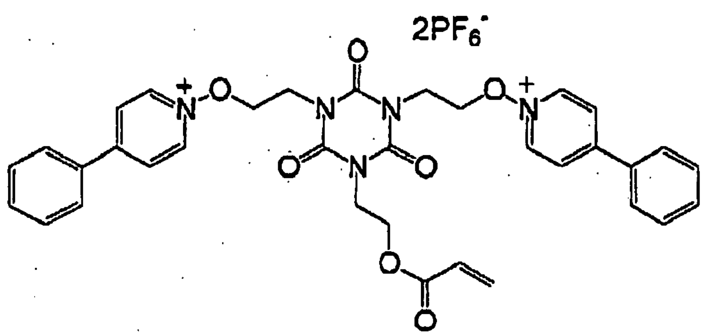

(Aa-32)

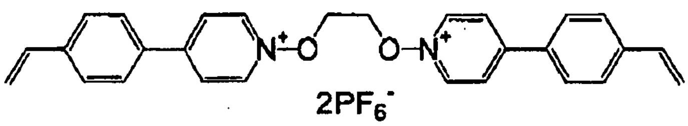

(Aa-33)

(continued)

X-

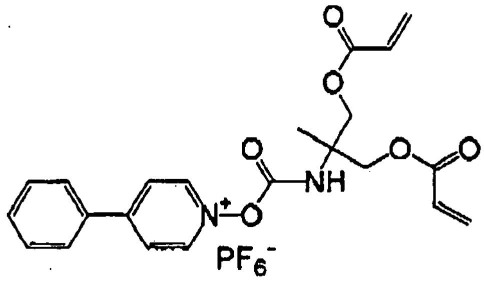

(Aa-34)

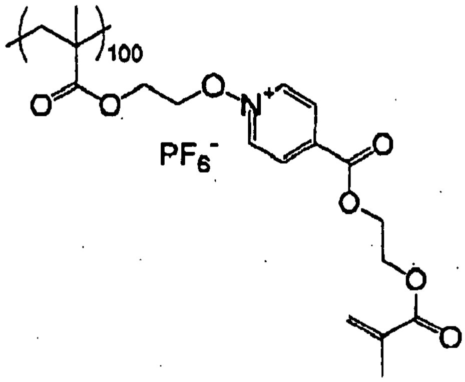

(Aa-35)

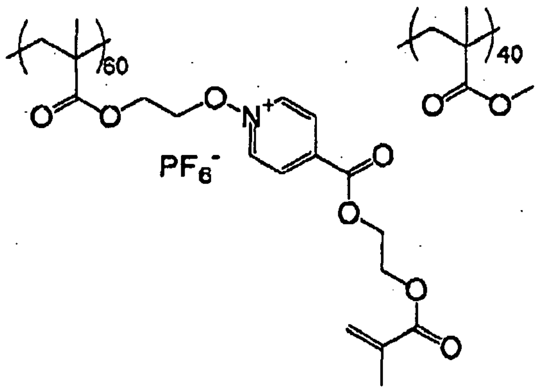

(Aa-36)

The compounds represented by formula (I) or (Ia) may be used individually or in combination of two or more thereof. When two or more thereof are used, a different kind of compounds, for example, a single compound, a dimer or a polymer may be used in combination.

The compound represented by any one of formulae (I), (Ia), (II), (IIa), (III), (IV) and (IVa) is referred to as a specific compound, hereinafter.

The content of the specific compound in the image-recording layer of the lithographic printing plate precursor according to the invention is preferably from 0.1 to 50% by weight, more preferably from 0.5 to 30% by weight, still more preferably from 1 to 20% by weight, based on the total solid content of the image-recording layer.

In the above-described range of the content, good sensitivity and good stain resistance in the non-image area at printing are achieved. The specific compound may be added together with other components to one layer or may be added to a different layer separately provided.

<Other polymerization initiator>

Since the specific compound has the polymerization initiation ability, other polymerization initiator is not essential for the invention. However, a polymerization initiator other than the specific compound may be used together as long as the effect of the invention is not impaired.

The polymerization initiator capable of being used together with the specific compound in the invention is a compound that generates a radical with light energy, heat energy or both energies to initiate or accelerate polymerization of a compound having a polymerizable unsaturated group and includes, for example, known thermal polymerization initiators, compounds containing a bond having small bond dissociation energy and photopolymerization initiators. The polymerization initiator capable of being used together with the specific compound in the invention can be appropriately selected from various kinds of known photopolymerization initiators or combination systems of two or more photopolymerization initiators (photopolymerization initiation systems) described in patents and literature depending on a wavelength of a light source to be used.

The polymerization initiators include, for example, (a) organic halogen compounds, (b) carbonyl compounds, (c) organic peroxides, (d) azo compounds, (e) azide compounds, (f) metallocene compounds, (g) hexaarylbiimidazole compounds, (h) organic borate compounds, (i) disulfone compounds, (j) oxime ester compounds and (k) onium salt compounds.

The respective compounds are described below.

The organic halogen compounds (a) specifically include, for example, compounds described in Wakabayashi et al., Bull. Chem. Soc. Japan, 42, 2924 (1969), U.S. Patent 3,905,815, JP-B-46-4605 (the term "JP-B" as used herein means an "examined Japanese patent publication, JP-A-48-35281, JP-A-55-32070, JP-A-60-239736, JP-A-61-169835, JP-A-61-169837, JP-A-62-58241, JP-A-62-212401, JP-A-63-70243, JP-A-63-298339 and M. P. Hutt, Journal of Heterocyclic Chemistry, 1, No. 3 (1970). Particularly, oxazole compounds and s-triazine compounds each substituted with a trihalomethyl group are exemplified.

More preferably, s-triazine derivatives in which at least one of mono-, di- or tri-halogen substituted methyl group is connected to the s-triazine ring are exemplified. Specific examples thereof include 2,4,6-tris(monochloromethyl)-s-triazine, 2,4,6-tris(dichloromethyl)-s-triazine, 2,4,6-tris(trichloromethyl)-s-triazine, 2-methyl-4,6-bis(trichloromethyl)-s-triazine, 2-n-propyl-4,6-bis(trichloromethyl)-s-triazine, 2-($\alpha,\alpha,\beta$-trichloroethyl)-4,6-bis(trichloromethyl)-s-triazine, 2-phenyl-4,6-bis(trichloromethyl)-s-triazine, 2-(p-methoxyphenyl)-4,6-bis(trichloromethyl)-s-triazine, 2-(3,4-epoxyphenyl)-4,6-bis(trichloromethyl)-s-triazine, 2-(p-chlorophenyl)-4,6-bis(trichloromethyl)-s-triazine, 2-[1-(p-methoxyphenyl)-2,4-butadienyl]-4,6-bis(trichloromethyl)-s-triazine, 2-styryl-4,6-bis(trichloromethyl)-s-triazine, 2-(p-methoxystyryl-4,6-bis(trichloromethyl)-s-tiazine, 2-(p-isopropyloxystyryl-4,6-bis(trichloromethyl)-s-triazine, 2-(p-tolyl)-4,6-bis(trichloromethyl)-s-triazine, 2-(4-methoxynaphthyl)-4,6-bis(trichloromethyl)-s-triazine, 2-phenylthio-4,6-bis(trichloromethyl)-s-triazine, 2-benzylthio-4,6-bis(trichloromethyl)-s-triazine, 2,4,6-tris(dibromomethyl)-s-triazine, 2,4,b-tris(tribromomethyl)-s-triazine, 2-methyl-4,6-bis(tribromomethyl)-s-triazine and 2-methoxy-4,6-bis(tribromomethyl)-s-triazine.

The carbonyl compounds (b) include, for example, benzophenone derivatives, e.g., benzophenone, Michler's ketone, 2-methylbenzophenone, 3-methylbenzophenone, 4-methylbenzophenone, 2-chlorobenzophenone, 4-bromobenzophenone or 2-carboxybenzophenone, acetophenone derivatives, e.g., 2,2-dimethoxy-2-phenylacetophenone, 2,2-diethoxyacetophenone, 1-hydroxycyclohexylphenylketone, $\alpha$-hydroxy-2-methylphenylpropane, 1-hydroxy-1-methylethyl-(p-isopropylphenyl)ketone, 1-hydroxy-1-(p-dodecylphenyl)ketone, 2-methyl-(4'-(methylthio)phenyl)-2-morpholino-1-propane or 1,1,1,-trichloromethyl-(p-butylphenyl)ketone, thioxantone derivatives, e.g., thioxantone, 2-ethylthioxantone, 2-isopropylthioxantone, 2-chlorothioxantone, 2,4-dimetylthioxantone, 2,4-dietylthioxantone or 2,4-diisopropylthioxantone, and benzoic acid ester derivatives, e.g., ethyl p-dimethylaminobenzoate or ethyl p-diethylaminobenzoate.

The organic peroxides (c) include, for example, trimethyleyelohexanone peroxide, acetylacetone peroxide, 1,1-bis(tert-butylperoxy)-3,3,5-trimethylcyclohexane, 1,1-bis(tert-butylperoxy)cyclohexane, 2,2-bis(tert-butylperoxy)butane, tert-butylhydroperoxide, cumene hydroperoxide, diisopropylbenzene hydroperoxide, 2,5-dimethylhexane-2,5-dihydroperoxide, 1,1,3,3-tetramethylbutyl hydroperoxide, tert-butylcumyl peroxide, dicumyl peroxide, 2,5-dimethyl-2,5-di(tert-butylperoxy)hexane, 2,5-oxanoyl peroxide, peroxy succinic acid, benzoyl peroxide, 2,4-dichlorobenzoyl peroxide, diisopropylperoxy dicarbonate, di-2-ethylhexylperoxy dicarbonate, di-2-ethoxyethylperoxy dicarbonate, dimethoxyisopropylperoxy dicarbonate, di(3-methyl-3-methoxybutyl)peroxy dicarbonate, tert-butylperoxy acetate, tert-butylperoxy pivalate, tert-butylperoxy neodecanoate, tert-butylperoxy octanoate, tert-buxylperoxy laurate, tersyl carbonate, 3,3',4,4'-tetra(tert-butylperoxycarbonyl)benzophenone, 3,3',4,4'-tetra(tert-hexylperoxycarbonyl)benzophenone, 3,3',4,4'-tetra(p-isopropylcumylperoxycarbonyl)benzophenone, carbonyl di(tert-butylperoxydihydrogen diphihalate) and carbonyl di(tert-hexylperoxydihydrogen diphthalate).

As the azo compounds (d), for example, azo compounds described in JP-A-8-108621 can be used.

The azide compounds (c) include, for example, azide polymers described in Nihon Shashin Gakkai 1968-nen Syunki Kenkyu Happyokai Koenyoshisyu (Summery of Research Presentation Meeting of The Society of Photographic Science and Technology of Japan in 1986 Spring), page 55 (1968), and 2-azidobenzoxazole, benzoylazide and 2-azidobenzimidazole described in U.S. Patent 3,282,693.

The metallocene compounds (f) are appropriately selected from various titanocene compounds described, for example, in JP-A-59-152396, JP-A-61-151197, JP-A-63-41483, JP-A-63-41484, JP-A-2-249, JP-A-2-291, JP-A-2-4705, JP-A-3-27393, JP-A-3-12403, JP-A-5-83588 and JP-A-6-41170. specific examples thereof include dicyclopentadienyl-Ti-dichloride, dicyclopentadienyl-Ti-bisphenyl, dicyclopentadienyl-Ti-bis-2,6-difluorophen-1-yl, dicyclopentadienyl-Ti-bis-2,4-difluorophen-1-yl, dicyclopentadienyl-Ti-bis-2,4,6-triafluorophen-1-yl, dicyclopentadienyl-Ti-bis-2,3,5,6-tetrafluorophen-1-yl, dicyclopentadienyl-Ti-bis-2,3,4,5,6-pentafluorophen-1-yl, dimethylcyclopentadienyl-Ti-bis-2,4-difluorophen-1-yl, dimethylcyclopentadienyl-Ti-bis-2,6-difluorophen-1-yl, dimethylcyclopentadienyl-Ti-bis-2,4,6-triafluorophen-1-yl, dimethylcyclopentadienyl-Ti-bis-2,3,5,6-tetrafluorophen-1-yl, dimethylcyclopentadienyl-Ti-bis-2,3,4,5,6-pentafluorophen-1-yl, dimethylcyclopentadienyl-Ti-bis-2,3,4,5,6-pentafluorophen-1-yl and bis(cyclopentadienyl)-bis[2,6-difluoro-3-(pyr-1-yl)phenyl]titanium.

As other metallocene compounds, iron-arene complexes described in JP-A-1-304453 and JP-A-1-152109 are illustrated.

The hexaarylbiimidazole compounds (g) include, for example, various compounds described in JP-B-6-29285 and U.S. Patents 3,479,185, 4,311,783 and 4,622,286, specifically, for example, 2,2'-bis(o-chlorophenyl)-4,4',5,5'-tetraphenylbiimidaxole, 2,2'-bis(o-bromophenyl)-4,4',5,5'-tetraphenylbiimidazole, 2,2'-bis(o,p-dichlorophenyl)-4,4',5,5'-tetraphenylbiimidazole, 2,2'-bis(o-chlorophenyl)-4,4',5,5'-tetra(m-methoxyphenyl)biimidazole, 2,2'-bis(o,o'-dichlorophenyl)-4,4',5,5'-tetrapbenylbiimidazole, 2,2'-bis(o-nitrophenyl)-4,4',5,5'-tetraphenylbiimidazole, 2,2'-bis(o-methylphenyl)-4,4',5,5'-tetraphenylbiimidazole and 2,2'-bis(o-trifluoromethylphenyl)-4,4',5,5'-tetraphenylbiimidazole.

The organic borate compounds (h) include, for example, organic boric acid salts described in JP-A-62-143044, JP-A-62-150242, JP-A-9-188685, JP-A-9-188686, JP-A-9-188710, JP-A-2000-131837, JP-A-2002-107916, Japanese Patent No. 2764769, JP-A-2002-116539 and Martin Kunz, Rad Tech '98, Proceeding, April 19-22, 1998, Chicago, organic boron sulfonium complexes or organic boron oxosulfonium complexes described in JP-A-6-157623, JP-A-6-175564 and JP-A-6-175561, organic boron iodonium complexes described in JP-A-6-175554 and JP-A-6-175553, organic boron phosphonium complexes described in JP-A-9-188710, and organic boron transition metal coordination complexes described in JP-A-6-348011, JP-A-7-12878S, JP-A-7-140589, JP-A-7-306527 and JP-A-7-292014.

The disulfone compounds (i) include, for example, compounds described in JP-A-61-166544 and JP-A-2002-328465,

The oxime ester compounds (j) include compounds represented by the following formula (i).

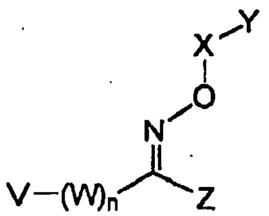

(i)

In formula (i), X represents a carbonyl group, a sulfone group or a sulfoxide group. Y represents a cyclic or chain alkyl group having from 1 to 12 carbon atoms, an alkenyl group, an alkynyl group, an aryl group having from 6 to 18 carbon atoms or a heterocyclic group. The aryl group includes an aromatic hydrocarbon group, for example, a benzene ring, a naphthalene ring, an anthracene ring, a phenanthrene group, a pyrene group or a triphenylene group, and the heterocyclic group includes an aromatic compound having at least one of a nitrogen atom, a sulfur atom and an oxygen atom in the cyclic structure thereof, for example, a pyrrole group, a furan group, a thiophene group, a selenophene group, a pyrazole group, an imidazole group, a triazole group, a tetrazole group, an oxazole group, a thiazole group, an indole group, a benzofuran group, a benzimidazole group, a benzoxazole group, a benzothiazole group, a pyridine group, a pyrimidine group, a pyrazine group, a triazine group, a quinoline group, a carbazole group, an acrydine group, a phenoxazine group and a phenothiazine group. The group represented by Y may be substituted with a halogen atom, a hydroxy group, a nitrile group, a nitro group, a carboxyl group, an aldehyde group, an alkyl group, a mercapto group, an aryl group, an alkenyl group, an alkynyl group, an ether group, an ester group, a urea group, an amino group, an amido group, a sulfido group, a disulfido group, a sulfoxido group, a sulfo group, a sulfone group, a hydrazine group, a carbonyl group, an imino group, a halogen atom, a hydroxy group, a nitrile group, a nitro group, a carboxyl group, a carbonyl group, a urethane group, an alkyl group, a mercapto group, an aryl group, a phophoroso group, a phospho group or a carbonyl ether group.

In formula (i), Z has the same meaning as Y or represents a nirtile group, a halogen atom, a hydrogen atom or an amino group. The group represented by Z may be substituted with a halogen atom, a hydroxy group, a nitrile group, a nitro group, a carboxyl group, an aldehyde group, an alkyl group, a mercapto group, an aryl group, an alkenyl group, an alkynyl group, an ether group, an ester group, a urea group, an amino group, an amido group, a sulfido group, a disulfido group, a sulfoxido group, a sulfo group, a sulfone group, a hydrazine group, a carbonyl group, an imino group, a halogen atom, a hydroxy group, a nitrile group, a nitro group, a carboxyl group, a carbonyl group, a urethane group, an alkyl group, a mercapto group, an aryl group, a phophoroso group, a phospho group or a carbonyl ether group.

In formula (i), W represents a divalent organic group, for example, a methylene group, a carbonyl group, a sulfoxido group, a sulfone group or an imino group. The methylene group and imino group may be each substituted with

an alkyl group, an aryl group, an ester group, a nitrile group, a carbonyl ether group, a sulfo group, a sulfo ether group or an ether goup, n represents an integer of 0 or 1.

In formula (i), V represents a cyclic or chain alkyl group having from 1 to 12 carbon atoms, an alkenyl group, an alkynyl group, an aryl group having from 6 to 18 carbon atoms, an alkoxy group or an aryloxy goup. The aryl group includes an aromatic hydrocarbon group, for example, a benzene ring, a naphthalene ring, an anthracene ring, a phenanthrene group, a pyrene group or a triphenylene group, and a hetero atom-containing aromatic compound, for example, a pyrrole group, a furan group, a thiophene group, a selenophene group, a pyrazole group, an imidazole group, a triazole group, a tetrazole group, an oxazole group, a thiazole group, an indole group, a benzofuran group, a benzimidazole group, a benzoxazole group, a benzothiazole group, a pyridine group, a pyrimidine group, a pyrazine group, a triazine group, a quinoline group, a carbazole group, an acrydine group, a phenoxazine group and a phenothiazine group. The group represented by V may be substituted with a halogen atom, a hydroxy group, a nitrile group, a nitro group, a carboxyl group, an aldehyde group, an alkyl group, a mercapto group, an aryl group, an alkenyl group, an alkynyl group, an ether group, an ester group, a urea group, an amino group, an amido group, a sulfido groups, a disulfido group, a sulfoxido group, a sulfo group, a sulfone group, a hydrazine group, a carbonyl group, an imino group, a halogen atom a hydroxy group, a nitrile group, a nitro group, a carboxyl group, a carbonyl group, a urethane group, an alkyl group, a mercapto group, an aryl group, a phophoroso group, a phospho group or a carbonyl ether group.

Alternatively, V and Z may be combined with each other to from a ring.

In the oxime ester compounds represented by formula (i), it is preferred that X represents a carbonyl group, Y represents an aryl group or a benzoyl group, Z represents an alkyl group or an aryl group, W represents a carbonyl group and V represents an aryl group in view of sensitivity. It is more preferred that the aryl group represented by V has a thioether substituent The structure of N-O bond in formula (i) may be any one of E isomer and Z isomer.

Examples of the oxime ester compound preferably used in the invention include compounds described in Progress in Organic Coatings, 13, 123-150 (1985), J. C. S. Perkin II, 1653-1660 (1979), Journal of Photopolymer Science and Technology, 205-232 (1995), J. C. S Perkin II, 156-162 (1979), JP-A-2000-66385 and JP-A-2000-80068.

Specific examples of the oxime ester compound preferably used in the invention are set forth below, but the invention should not be construed as being limited thereto.

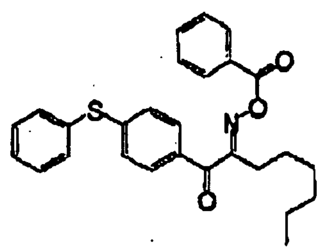
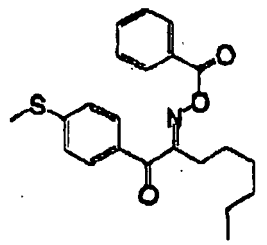

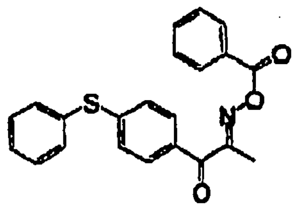
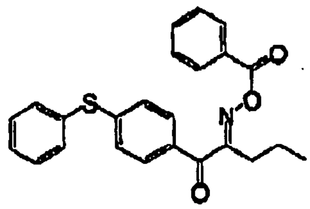

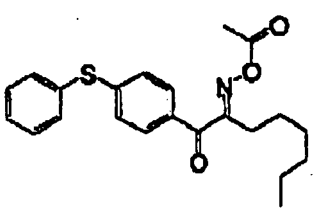
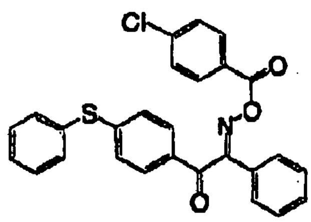

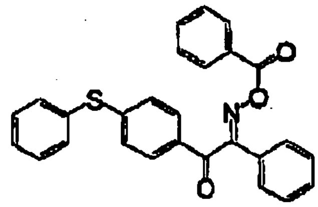
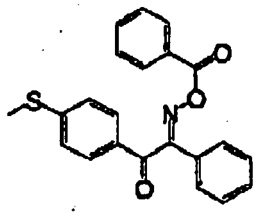
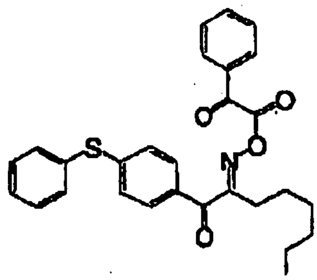
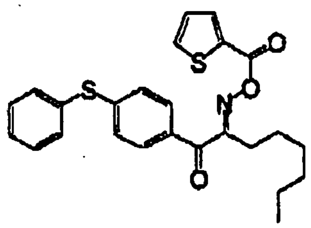
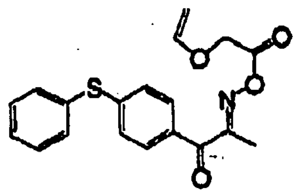
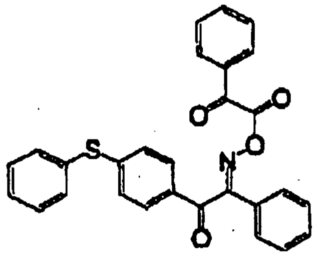
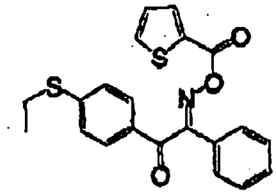
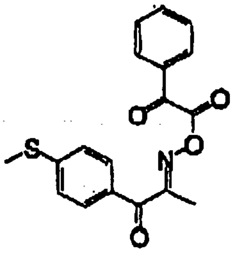
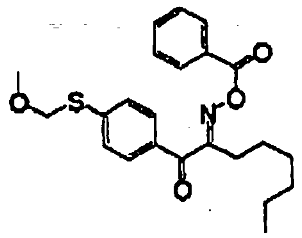
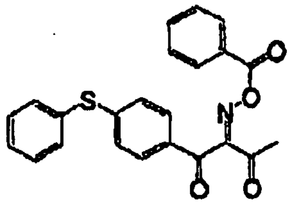
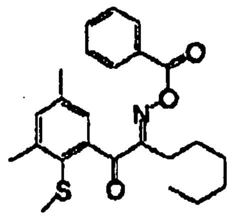
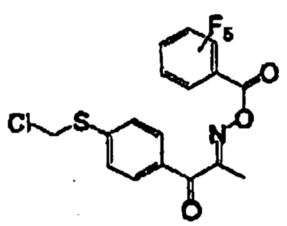
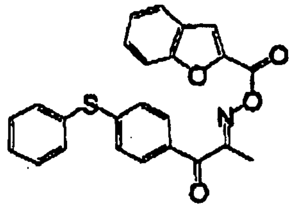

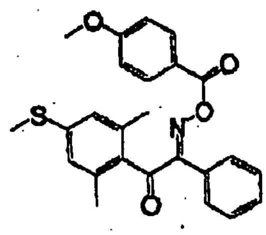

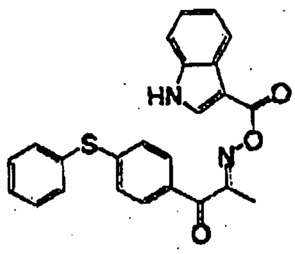

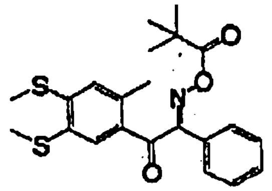

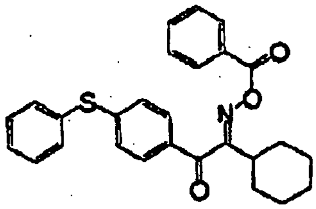

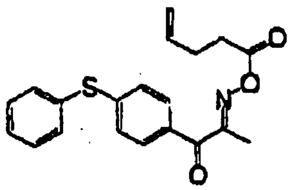

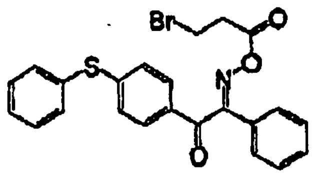

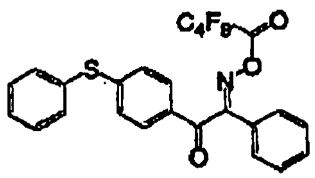

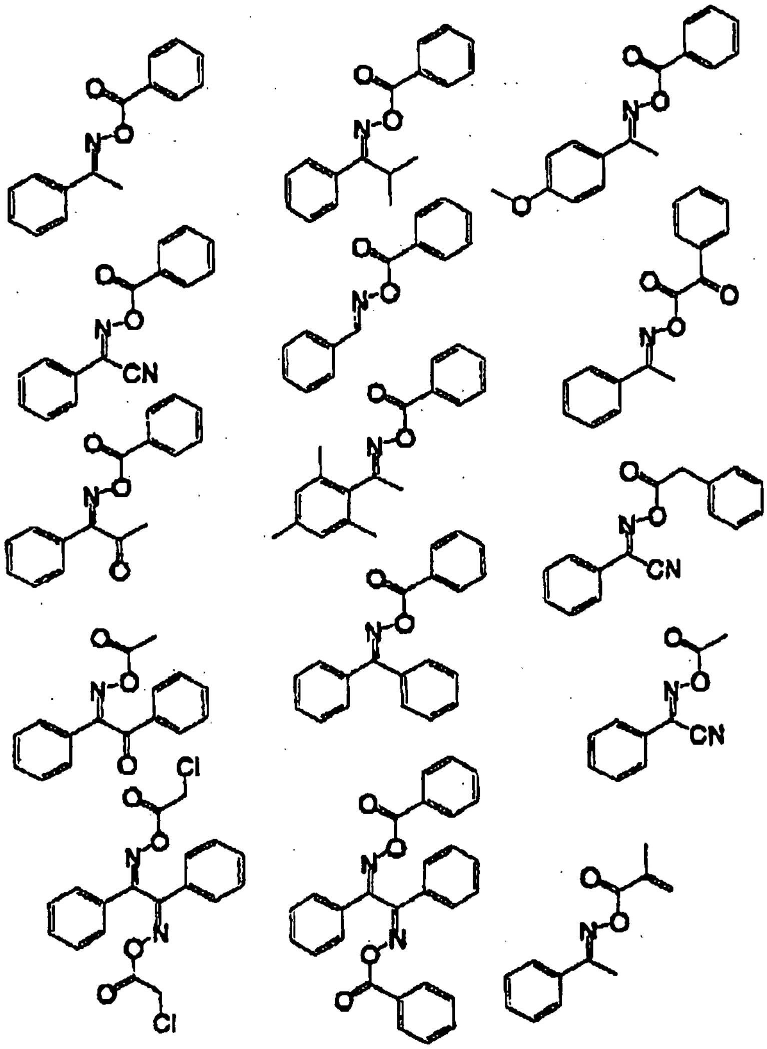

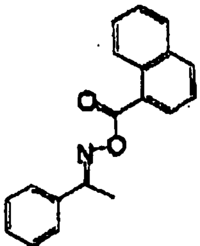
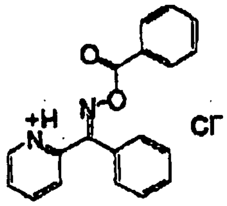
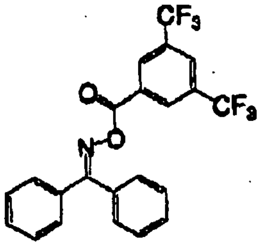
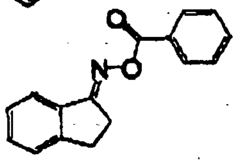
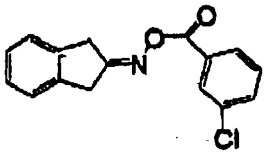
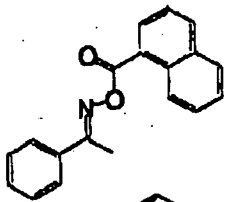
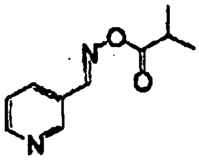
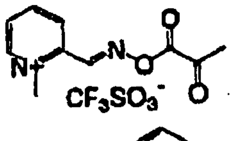
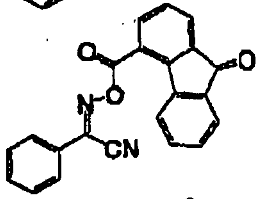
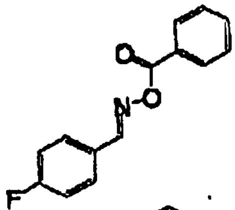
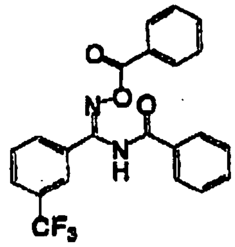
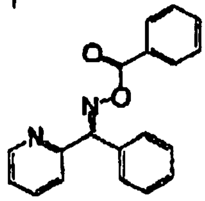
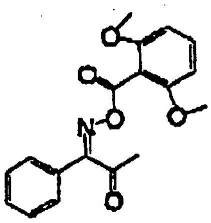
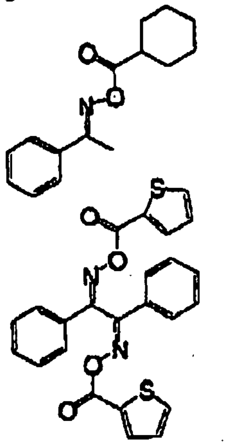

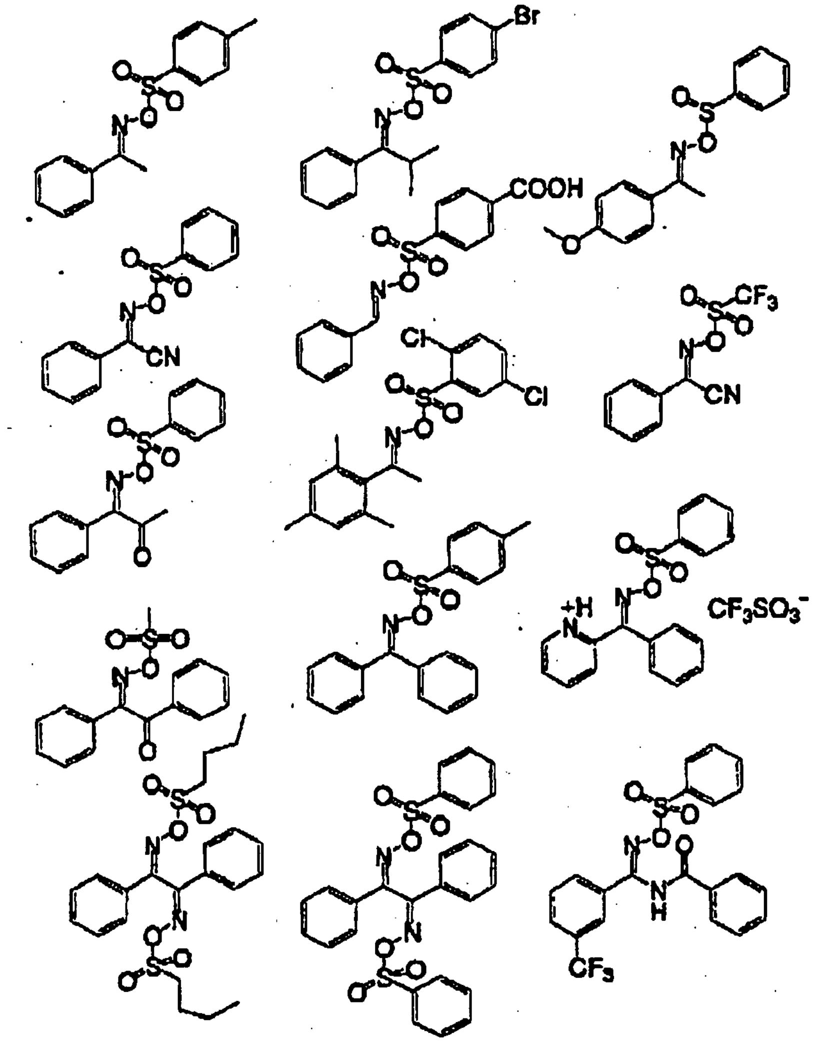

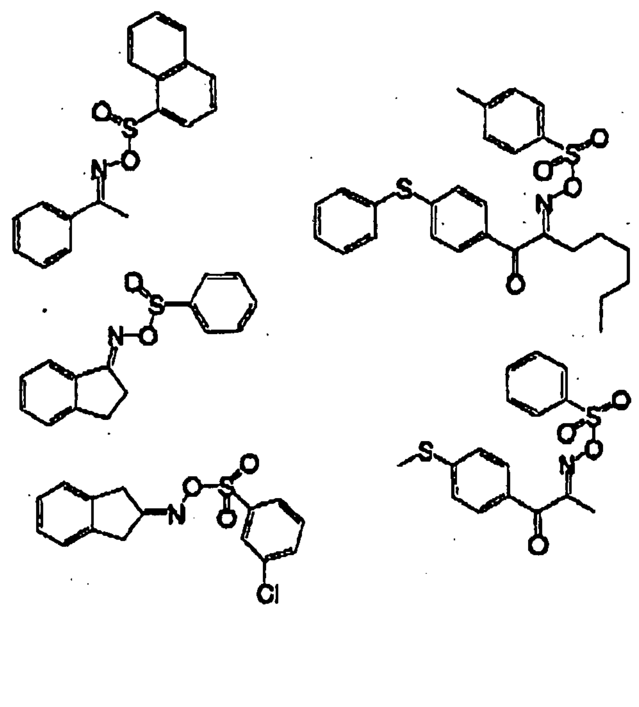

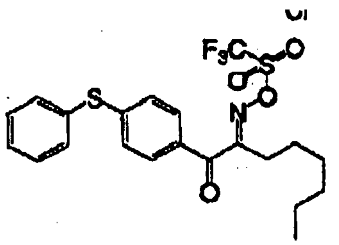

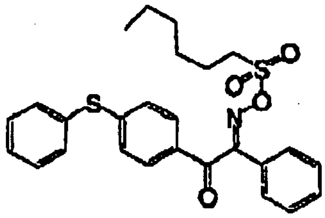

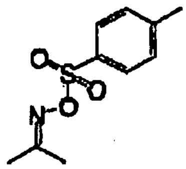

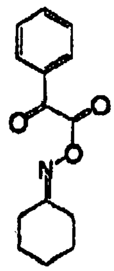

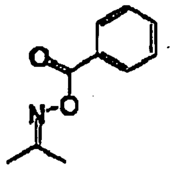

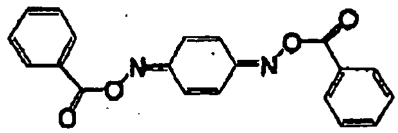

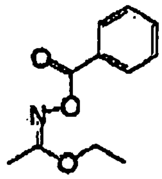

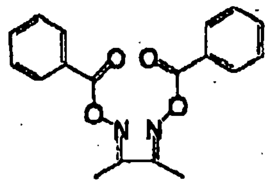

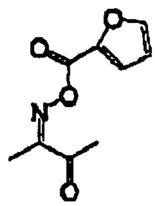

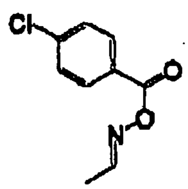

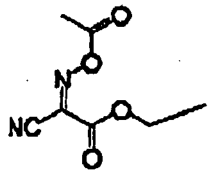

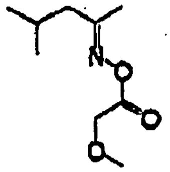

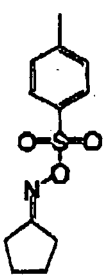

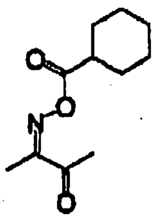 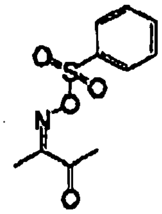

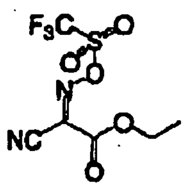 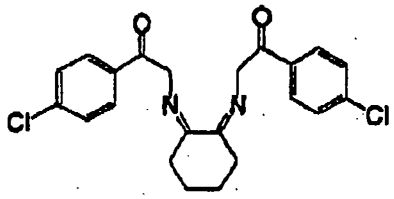

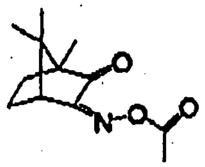 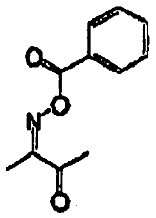

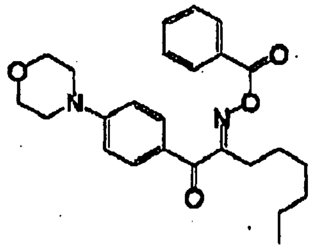 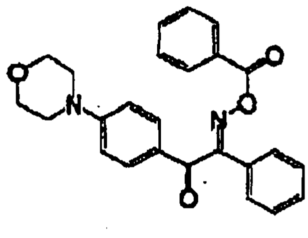

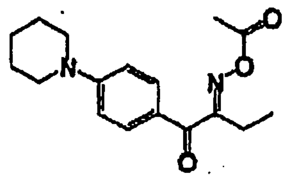 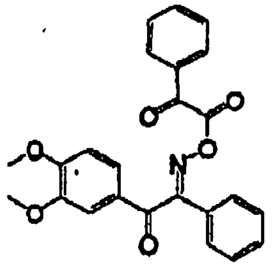

The onium salt compounds (k) include, for example, diazonium salts described in S. L Schlesinger, Photogr. Sci. Eng., 18, 387 (1974) and T. S. Bal et al., Polymer, 21, 423 (1980), ammonium salts described in U.S. Patent 4,069,055 and JP-A-4-365049, phosphonium salts, described in U.S. Patents 4,069,055 and 4,069,056, iodonium salts described in European Patent 104,143, U.S. Patents 339,049 and 410,201, JP-A-2-150848 and JP-A-2-296514, sulfonium salts described in European Patents 370,693, 390,214, 233,567, 297,443 and 297,442, U.S. Patents 4,933,377, 161,811, 410,201, 339,049, 4,760,013, 4,734,444 and 2,833,827 and German Patents 2,904,626, 3,604,580 and 3,604,581, selenonium salts described in J.V. Crivello et al., Macromolecules, 10 (6), 1307 (1977) and J.V. Crivello et al., J. Polymer Sci., Polymer Chem. Ed., 17, 1047 (1979), and arsonium salts described in C.S. Wen et al., Teh, Proc. Conf. Rad. Curing ASIA, p. 478, Tokyo, Oct. (1988).

Particularly, in view of reactivity and stability, the oxime ester compounds described above and diazonium compounds, iodonium compounds and sulfonium compounds described in detail below are illustrated.

In the invention, the onium salt functions not as an acid generator, but as an ionic radical polymerization initiator.

The onium salts preferably used in the invention include onium salts represented by the following formulae (RI-I) to (RI-III):

$Ar^{11}-N^{+}\equiv N\ Z^{11-}$ (RI-I)

$Ar^{21}-I^{+}-Ar^{22}\ Z^{21-}$ (RI-II)

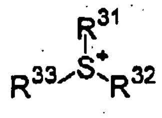 $Z^{31-}$ (RI-III)

In formula (RI-I), $Ar^{11}$ represents an aryl group having 20 or less carbon atoms, which may have I to 6 substituents. Preferable example of the substituent includes an alkyl group having from 1 to 12 carbon atoms, an alkenyl group having from 1 to 12 carbon atoms, an alkynyl group having from 1 to 12 carbon atoms, an aryl group having from 1 to 12 carbon atoms, an alkoxy group having from 1 to 12 carbon atoms, an aryloxy group having from 1 to 12 carbon atoms, a halogen atom, an alkylamino group having from 1 to 12 carbon atoms, a dialkylimino group having from 1 to 12 carbon atoms, an alkylamido group or arylamido group having from 1 to 12 carbon atoms, a carbonyl group, a carboxyl group, a cyano group, a sulfonyl group, an thioalkyl group having from 1 to 12 carbon atoms and an thioaryl group having from 1 to 12 carbon atoms.

$Z^{11-}$ represents a monovalent anion. Specific examples of the monovalent anion include a halogen ion, a perchlorate ion, a hexafluorophosphate ion, a tetrafluoroborate ion, a sulfonate ion, a sulfinate ion, a thosulfonate ion and a sulfate ion. Among them, a perchlorate ion, a hexafluorophosphate ion, a tetrafluoroborate ion, a sulfonate ion and a sulfinate ion are preferred in view of stability.

In formula (RI-II), $Ar^{21}$ and $Ar^{22}$ each independently represents an aryl group having 20 or less carbon atoms, which may have 1 to 6 substituents. Preferable example of the substituent includes an alkyl group having from 1 to 12 carbon atoms, an alkenyl group having from 1 to 12 carbon atoms, an alkynyl group having from 1 to 12 carbon atoms, an aryl group having from 1 to 12 carbon atoms, an alkoxy group having from 1 to 12 carbon atoms, an aryloxy group having from 1 to 12 carbon atoms, a halogen atom, an alkylamino group having from 1 to 12 carbon atoms, a dialkylimino group having from 1 to 12 carbon atoms, an alkylamido group or arylamido group having from 1 to 12 carbon atoms, a carbonyl group, a carboxyl group, a cyano group, a sulfonyl group, an thioalkyl group having from 1 to 12 carbon atoms and an thioaryl group having from 1 to 12 carbon atoms.

$Z^{21-}$ represents a monovalent anion. Specific examples of the monovalent anion include a halogen ion, a perchlorate ion, a hexafluorophosphate ion, a tetrafluoroborate ion, a sulfonate ion, a sulfinate ion, a thosulfonate ion, a sulfate ion and a carboxylate ion. Among them, a perchlorate ion, a hexafluorophosphate ion, a tetrafluoroborate ion, a sulfonate ion, a sulfinate ion and a carboxylate ion are preferred in view of stability and reactivity.

In formula (RI-III), $R^{31}$, $R^{32}$ and $R^{33}$ each independently represents an aryl group having 20 or less carbon atoms, which may have 1 to 6 substituents, an alkyl group, an alkenyl group or an alkynyl group. Among them, the aryl group is preferred in view of reactivity and stability. Preferable example of the substituent includes an alkyl group having from 1 to 12 carbon atoms, an alkenyl group having from 1 to 12 carbon atoms, an alkynyl group having from 1 to 12 carbon atoms, an aryl group having from 1 to 12 carbon atoms, an alkoxy group having from 1 to 12 carbon atoms, an aryloxy group having from 1 to 12 carbon atoms, a halogen atom, an alkylamino group having from 1 to 12 carbon atoms, a dialkylimino group having from 1 to 12 carbon atoms, an alkylamido group or arylamido group having from 1 to 12 carbon atoms, a carbonyl group, a carboxyl group, a cyano group, a sulfonyl group, an thioalkyl group having from 1 to 12 carbon atoms and an thioaryl group having from 1 to 12 carbon atoms.

$Z^{31-}$ represents a monovalent anion. Specific examples of the monovalent anion include a halogen ion, a perchlorate ion, a hexafluorophosphate ion, a tetrafluoroborate ion, a sulfonate ion, a sulfinate ion, a thosulfonate ion, a sulfate ion and a carboxylate ion. Among them, a perchlorate ion, a hexafluorophosphate ion, a tetrafluoroborate ion, a sulfonate ion, a sulfinate ion and a carboxylate ion are preferred in view of stability and reactivity. Especially, carboxylate ions described in JP-A-2001-343742 are preferable, and carboxylate ions described in JP-A-2002-148790 are more preferable.

Specific examples of the onium salt compound which is preferably used as the polymerization initiator in the invention are set forth below, but the invention should not be construed as being limited thereto.

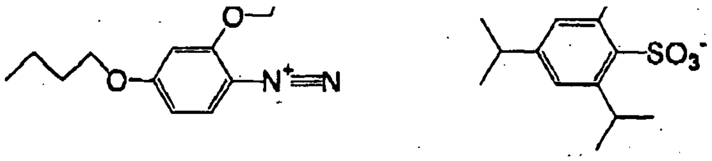

(N-1)

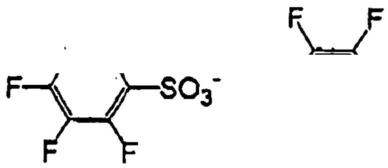

(N-2)

$PF_6^-$

$PF_6^-$ (N-3)

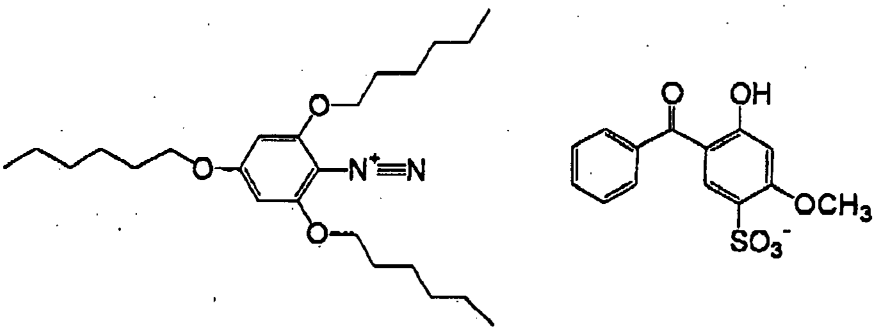

(N-4)

$ClO_4^-$ (N-5)

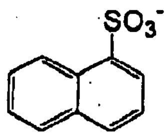

(N-6)

$PF_6^-$ (N-7)

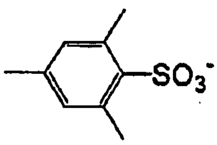

(N-8)

(N-9)

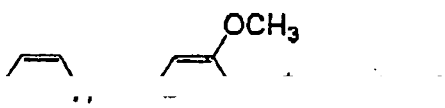

(N-10)

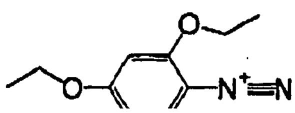

$PF_6^-$

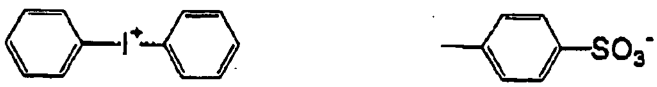

(I-1)

(I-1)

$PF_6^-$ (I-2)

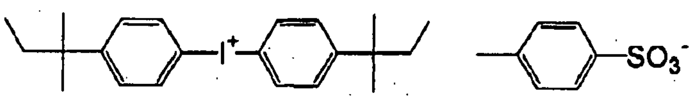

(I-3)

(I-3)

(continued)

$ClO_4^-$ (I-4)

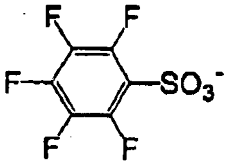

(I-5) (I-5)

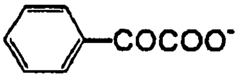

(I-6) (I-6)

$CF_3SO_3^-$ (I-7)

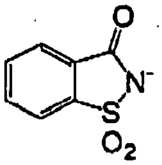

(I-8) (I-8)

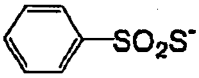

(I-9) (I-9)

$PF_6^-$ (I-10)

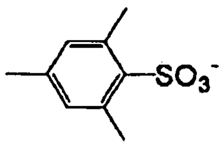

(I-11) (I-11)

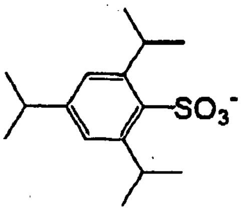

(I-12) (I-12)

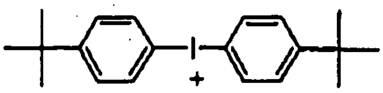

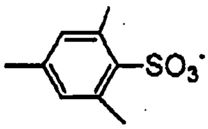

(I-13) (I-13)

$ClO_4^-$ (I-14)

$PF_6^-$ (I-15)

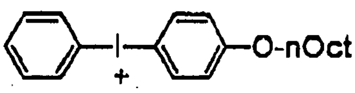

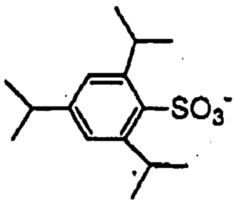

(I-16) (I-16)

$CF_3COO^-$ (I-17)

$CF_3SO_3^-$ (I-18)

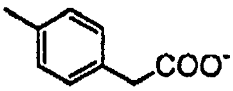

(I-19) (I-19)

(continued)

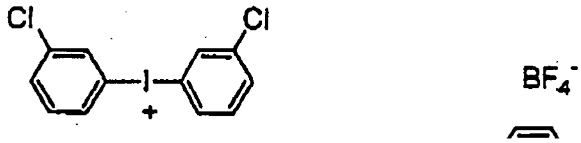

(I-20)

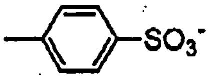

(I-21)

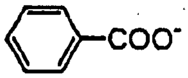

(I-22)

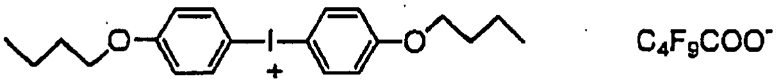

(I-23)

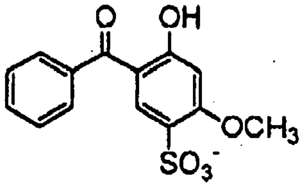

(I-24)

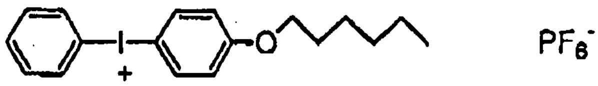

(I-25)

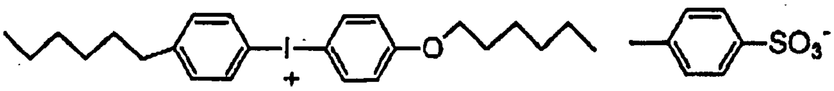

(I-26)

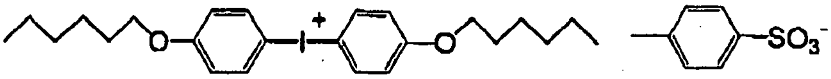

(I-27)

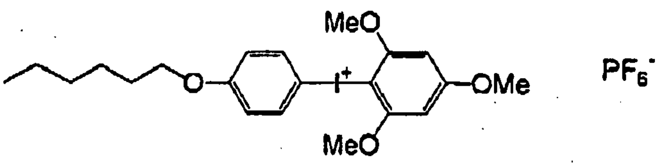

(I-28)

$ClO_4^-$ (I-29)

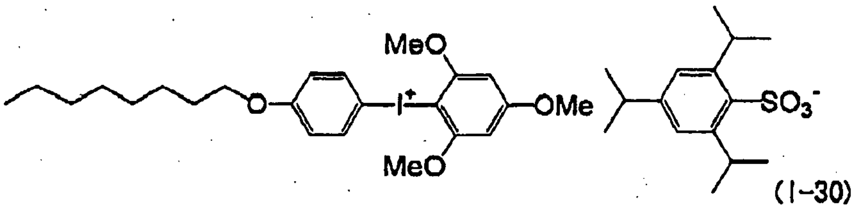

(I-30)

$PF_6^-$ (I-31)

$C_4F_9SO_3^-$ (I-32)

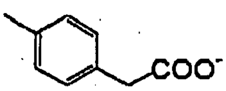

(I-33)

$BF_4^-$ (I-34)

(continued)

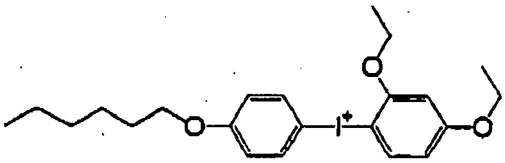 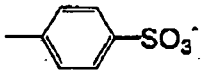 (I-35) (I-35)

$PF_6^-$ (I-36)

$PF_6^-$ (I-37)

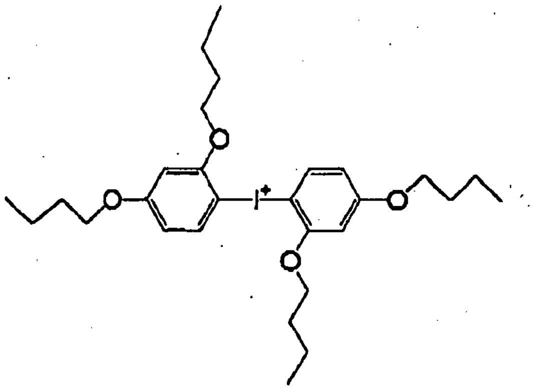 C4F9$SO_3^-$ (I-38)

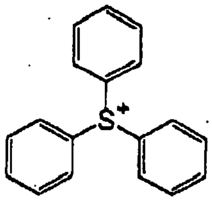 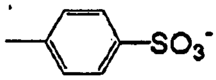 (S1)

$PF_6^-$ (S-2)

$ClO_4^-$ (S-3)

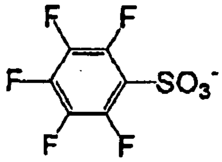 (S-4)

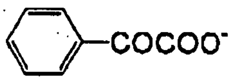 (S-5)

$CF_3SO_3^-$ (S-6)

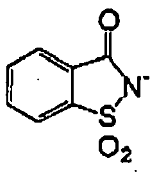 (S-7)

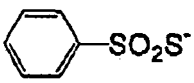 (S-8)

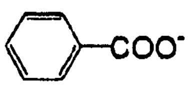 (S-9)

(continued)

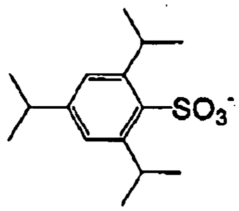

(S-10)

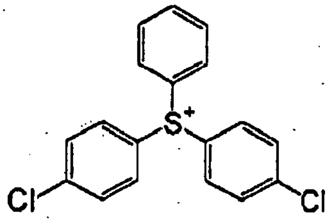

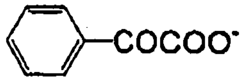

(S-11)

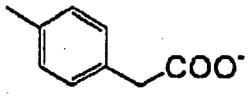

(S-12)

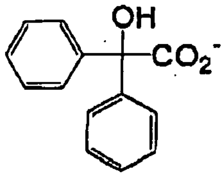

(S-13)

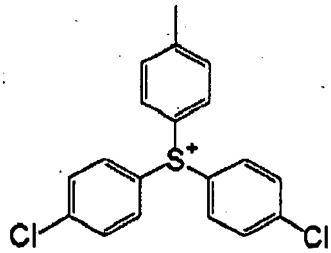

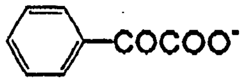

(S-14)

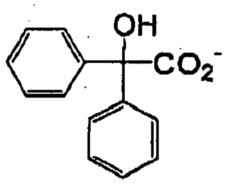

(S-15)

$BF_4^-$ (S-16)

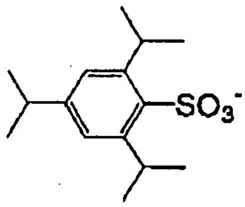

(S-17)

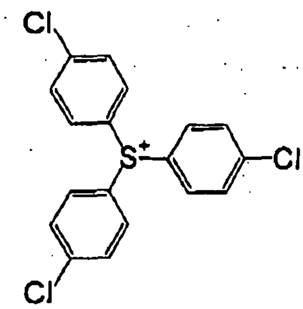

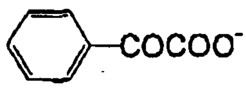

(S-18)

$CF_3SO_3^-$ (S-19)

(continued)

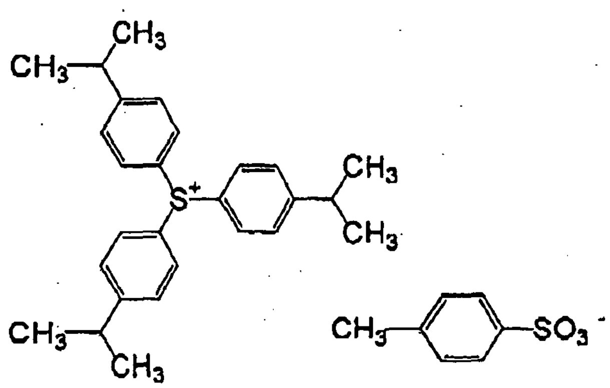

(S-20)

In the case of using as the light source, a blue semiconductor laser, an Ar laser, a second harmonic of an infrared semiconductor laser or an SHG-YAG laser, various photopolymerization initiators (systems) have been proposed. For instance, a certain kind of photo-reducing dyes, for example, Rose Bengal, Eosin or Erythrosine as described in U.S. Patent 2,850,445, and a combination system comprising a dye and an initiator, for example, a composite initiation system comprising a dye and an amine as described in JP-B-44-20189, a combination system of a hexaarylbiimidazole, a radical generator and a dye as described in JP-B-45-37377, a combination system of a hexaarylbiimidazole and a p-dialkylaminobenzylidene kotone as described in JP-B-47-2528 and JP-A-54-155292, a combination system of a cyclic cis-$\alpha$ -dicarbonyl compound and a dye as described in JP-A-48-84183, a combination system of a cyclic triazine and a merocyanine dye as described in JP-A-54-151024, a combination system of a 3-ketocoumarin and an activator as described in JP-A-52-112681 and JP-A-58-15503, a combination system of a biimidazole, a styrene derivative and a thiol as described in JP-A-59-140203, a combination system of an organic peroxide and a dye as described in JP-A-59-1504, JP-A-59-140203, JP-A-59-189340, JP-A-62-174203, JP-B-62-1641 and U.S. Patent 4,766,055, a combination system of a dye and an active halogen compound as described in JP-A-63-178105, JP-A-63-258903 and JP-A-3-264771, a combination system of a dye and a borate compound as described in JP-A-62-143044, JP-A-62-150242, JP-A-64-13140, JP-A-64-13141, JP-A-64-13142, JP-A-64-13143, JP-A-64-13144, JP-A-64-17048, JP-A-1-229003, JP-A-1-298348 and JP-A-1-138204, a combination system of a dye having a rhodanine ring and a radical generator as described in JP-A-2-179643 and JP-A-2-244050, a combination system of a titanocene and a 3-ketocoumarin dye as described in JP-A-63-221110, a combination system of a titanocene, a xanthene dye and an addition-polymerizable ethylenically unsaturated compound having an amino group or a urethane group as described in JP-A-4-221958 and JP-A-4-219756, a combination system of a titanocene and a specific merocyanine dye as described in JP-A-6-295061, and a combination system of a titanocene and a dye having a benzopyran ring as described in JP-A-8-334897 are exemplified.

Of the polymerization initiators and polymerization initiation systems capable of being used together with the specific compound, onium salt compounds including as a counter ion, an inorganic anion, for example, $PF_6^-$, $BF_4^-$ or $C_4F_9SO_3^-$ are preferable from the standpoint of improvement in the plate inspection property. Further, in view of being excellent in the color-forming property, diaryl iodonium salts including two or more electron donating groups are preferable. These polymerization initiators are compounds having a diaryl iodonium skeleton and have two or more, preferably three or more electron donating groups, for example, an alkyl group or an alkoxy group on the aryl group. It is preferred that the electron donating groups are introduced into the para positions and ortho positions of the aryl groups of the iodonium salt

The polymerization initiator for use in the invention has preferably an absorption maximum wavelength of 400 nm or shorter, more preferably 360 nm or shorter. By adjusting the absorption wavelength in an ultraviolet region as above, bandling of the lithographic printing plate precursor can be conducted under white light.

The other polymerization initiators may be used individually or in combination of two or more thereof. Further, the other polymerization initiator may be added together with other components to one layer or may be added to a different layer separately provided.

The total content of the polymerization initiator, that is, the total amount of the specific compound represented by formula (I), (Ia) or the like and the other polymerization initiator capable of being used together according to the invention is preferably from 0.1 to 50% by weight, more preferably from 0.5 to 30% by weight, most preferably from 1 to 20% by weight, based on the total solid content of the image-recording layer.

When the other polymerization initiator is used together with the specific compound, the content thereof is

preferably from 0 to 200% by weight, more preferably from 0 to 100% by weight, based on the content of the specific compound.

<(B) Sensitizing dye>

The specific compound according to the invention can be used in the image formation using an infrared laser or a visible light or ultraviolet laser as a light source. In order to improve the compatibility to such a light source, it is preferred to use the sensitizing dye (B) in the invention.

In the case of conducting the image formation using as the light source, a laser emitting an infrared ray of 760 to 1,200 nm, ordinarily, it is essential that an infrared absorbing agent (B1) is used as the sensitizing dye (B). In response to the visible light or ultraviolet laser, a sensitizing dye having an absorption peak in a wavelength range of 350 to 850 nm (B2) is preferably used.

(B1) Infrared absorbing agent

The infrared absorbing agent has a function of converting the infrared ray absorbed to heat and a function of being excited by the infrared ray to perform electron transfer/energy transfer to a polymerization initiator (radical generator) including the specific compound described above. The infrared absorbing agent for use in the invention includes a dye and pigment each having an absorption maximum in a wavelength range of 760 to 1,200 nm. The infrared absorbing dye is preferable from the standpoint of the effect.

As the dye, commercially available dyes and known dyes described in literatures, for example, Senryo Binran (Dye Handbook) compiled by The Society of Synthetic Organic Chemistry, Japan (1970) can be utilized. Specifically, the dyes includes azo dyes, metal complex azo dyes, pyrazolone azo dyes, naphthoquinone dyes, anthraquinone dyes, phthalocyanine dyes, carbonium dyes, quinoneimine dyes, methine dyes, cyanine dyes, squarylium dyes, pyrylium salts and metal thiolate complexes.

Preferable examples of the dye include cyanine dyes described, for example, in JP-A-58-125246, JP-A-59-84356 and JP-A-60-78787; methane dyes described, for example, in JP-A-58-173696, JP-A-58-181690 and JP-A-58-194595; naphthoquinone dyes described, for example, in JP-A-58-112793, JP-A-58-224793, JP-A-59-48187, JP-A-59-73996, JP-A-60-52940 and JP-A-60-63744; squarylium dyes described, for example, in JP-A-58-112792; and cyanine dyes described, for example, in British Patent 434,875.

Also, near infrared absorbing sensitizers described in U.S. Patent 5,156,938 are preferably used. Further, substituted arylbenzo(thio)pyrylium salts described in U.S. Patent 3,881,924, trimethinethiapyrylium salts described in JP-A-57-142645 (corresponding to U.S. Patent 4,327,169), pyrylium compounds described in JP-A-58-181051, JP-A-58-220143, JP-A-59-41363, JP-A-59-84248, JP-A-59.54249, JP-A-59-146063 and JP-A-59-146061, cyanine dyes described in JP-A-59-216146, pentamethinethiopyrylium salts described in U.S. Patent 4,283,475, and pyrylium compounds described in JP-B-5-13514 and JP-B-5-19702 are also preferably used Other preferred examples of the dye include near infrared absorbing dyes represented by formulae (I) and (II) described in U.S. Patent 4,756,993.

Other preferabe examples of the infrared absorbing dye according to the invention include specific indolenine cyanine dyes described in JP-A-2002-278057 as illustrated below.

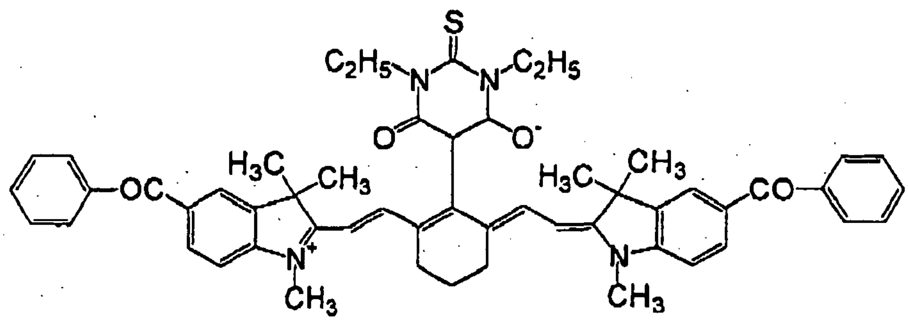

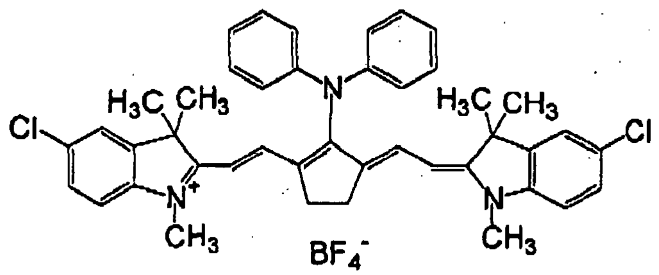

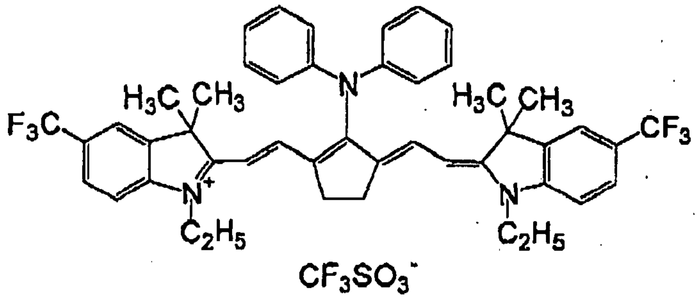

In particular, among the dyes, cyanine dyes, squarylium dyes, pyrylium dyes, nickel thiolate complexes and indolenine cyanine dyes are preferred, and from the standpoint of color change due to electron transfer, dyes containing a 5-membered ring, particularly a nitrogen-containing 5-membered hetero ring, in their molecules, are preferred. The cyanine dyes and indolenine cyanine dyes are more preferred. As a particularly preferable example of the dye, a cyanine dye represented by formula (ii) shown below is exemplified.

Formula (ii):

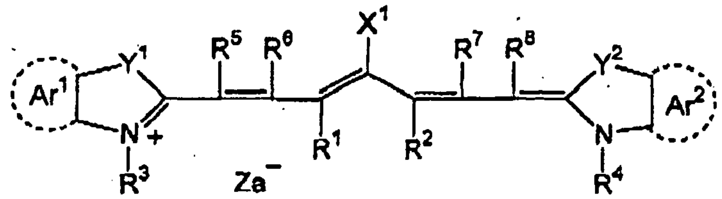

In formula (ii), $X^1$ represents a hydrogen atom, a halogen atom, -N(aryl)$_2$, $X^2$-$L^1$ or a group represented by a structural formula shown below. Aryl represents an aryl group which may have a substituent. $X^2$ represents an oxygen atom, a nitrogen atom or a sulfur atom, and $L^1$ represents a hydrocarbon group having from 1 to 12 carbon atoms, an aromatic cyclic group containing a hetero atom or a hydrocarbon group having from 1 to 12 carbon atoms and containing a hetero atom. The betero atom means a nitrogen atom, a sulfur atom, an oxygen atom, a halogen atom or a selenium atom.

In the structural formula shown below, $Xa^-$ has the same meaning as $Za^-$ defined hereinafter, and $R^n$ represents a hydrogen atom or a substituent selected from an alkyl group, an aryl group, a substituted or unsubstituted amino group and a halogen atom.

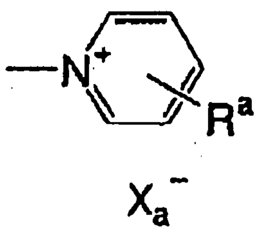

$R^1$ and $R^2$ each independently represents a hydrocarbon group having from 1 to 12 carbon atoms. In view of the preservation stability of a coating solution for image-recording layer, it is preferred that $R^1$ and $R^2$ each represents a hydrocarbon group having two or more carbon atoms, and particularly preferably, $R^1$ and $R^2$ are combined with each

other 1to form a 5-membered or 6-membered ring.

$Ar^1$ and $Ar^2$, which may be the same or different, each represents an aromatic hydrocarbon group which may have a substituent Preferable examples of the aromatic hydrocarbon group include a benzene ring and a naphthalene ring. Also, preferable examples of the substituent include a hydrocarbon group having 12 or less carbon atoms, a halogen atom and an alkoxy group having 12 or less carbon atoms, and a hydrocarbon group having 12 or less carbon atoms and an alkoxy group having 12 or less carbon atoms are most preferable. $Y^1$ and $Y^2$, which may be the same or different, each represents a sulfur atom or a dialkylmethylene group having 12 or less carbon atoms. $R^3$ and $R^4$, which may be the same or different, each represents a hydrocarbon group having 20 or less carbon atoms which may have a substituent. Preferable examples of the substituent include an alkoxy group having 12 or less carbon atoms, a carboxyl group and a sulfo group, and an alkoxy group having 12 or less carbon atoms is most preferable. $R^5$, $R^6$, $R^7$ and $R^8$, which may be the same or different, each represents a hydrogen atom or a hydrocarbon group having 12 or less carbon atoms. From the standpoint of the availability of raw materials, a hydrogen atom is preferred $Za^-$ represents a counter anion. However, $Za^-$ is not necessary when the cyanine dye represented by formula (ii) has an anionic substituent in the structure thereof so that neutralization of charge is not needed. Preferable examples of the counter anion for $Za^-$ include a halogen ion, a perchlorate ion, a tetrafluoroborate ion, a hexafluorophosphate ion and a sulfonate ion, and particularly preferable examples thereof include a perchlorate ion, a tetrafluoroborate ion, a hexafluorophosphate ion and an arylsulfonate ion in view of the preservation stability of a coating solution for image-recording layer.

From the standpoint of improvement in the plate inspection property, $Z_n^-$ is preferably an inorganic anion or a counter anion of a strong acid. From this point of view, $PF_6^-$, $BF_4^-$, $CF_3SO_3^-$ and $C_4F_9SO_3^-$ are preferable, and $PF_6^-$ is most preferable.

Specific examples of the cyanine dye represented by formula (ii) which can be preferably used in the invention include those described in paragaphs [0017] to [0019] of JP-A-2001-133969.

Further, other more preferable examples include the specific indolenine cyanine dyes described in JP-A-2002-278057 described above.

As the infrared absorbing agent particularly preferably used in the invention, a cyanine dye represented by formula (iii) shown below is exemplified.

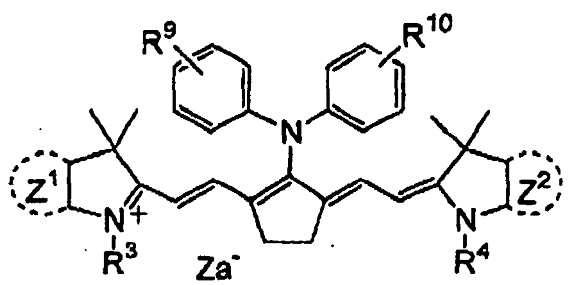

(iii)

In the formulae, $Z^1$ and $Z^2$ each independently represents an aromatic ring which may have a substituent or a hetero aromatic ring which may have a substituent and has the same meaning as defined for $Ar^1$ or $Ar^2$ in formula (ii). $R^3$ and $R^4$ each independently represents a hydrocarbon group having 20 or less carbon atoms which may have a substituent and has the same meaning as defined for $R^3$ or $R^4$ in formula (ii). $R^9$ and $R^{10}$ each independently represents a hydrogen atom or an alkoxy group which may have a substituent. $Z_a^-$ represents a counter anion which exists in case of being necessary for neutralizing a charge and has the same meaning as defined for $Z_a^-$ in formula (ii).

Above all, the infrared absorbing agent particularly preferable for the lithographic printing plate precursor of the invention is a compound including at least one solvent-soluble group in the skeleton of a cyanine dye. The solvent-soluble group for use in the invention means an organic functional group capable of increasing solvent-solubility of the infrared absorbing agent and it is not particularly restricted as long as it is a functional group having such a function. It is preferably includes, for example, an alkyloxy group, an aryloxy group, an alkylcarbonyl group, an arylcarbonyl group, an alkyloxycarbonyl group, an aryloxycarbonyl group, a sulfonylamido group, a carboxyl group, a sulfo group, a hydroxy group, an alkylcarbonyloxy group, an arylcarbonyloxy group and an amido group. Among them, an alkyloxy group, an aryloxy goup, an alkyloxycarbonyl group and an aryloxycarbonyl group are more preferable solvent-soluble groups, and an alkyloxy group and an aryloxy group are most preferable solvent-soluble groups.

As the infrared absorbing agent, more specifically, a compound having the cyanine dye structure represented by formula (iii) described above and including at least one solvent-soluble group in its molecule is preferable. The solvent-soluble group can be introduced into any position of the cyanine dye structure represented by formula (iii), and it is preferably introduced into an aromatic hydrocarbon group represented by $Z^1$ or $Z^2$, $R^3$ or $R^4$ on the nitrogen atom at the terminal, or $R^9$ or $R^{10}$ on $-NPh_2$. From the standpoint of improvement in the on-press development property, it is most preferable that the solvent-soluble groups are introduced into the nitrogen atoms at the both terminals thereof Although

a number of the solvent-soluble groups introduced is at least one, it is preferable that 2 to 6 solvent-soluble groups are introduced into one molecule of the cyanine dye from the standpoint that the image-recording layer can be coated in high concentration without unevenness, that the formation of scum resulting from the components in the image-recording layer at the on-press development is prevented, and that the on-press development property is improved.

Specific examples of the infrared absorbing agent which can be preferably used in the invention include those set forth below, but the invention should not be construed as being limited thereto.

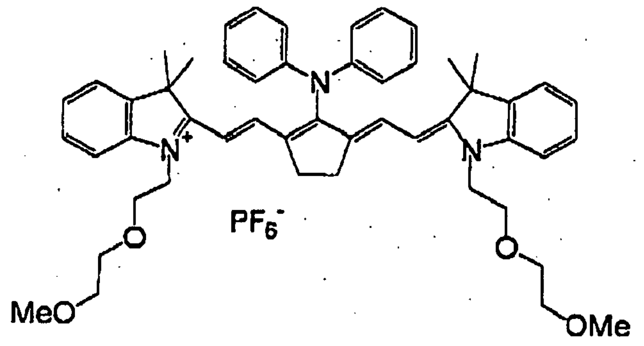

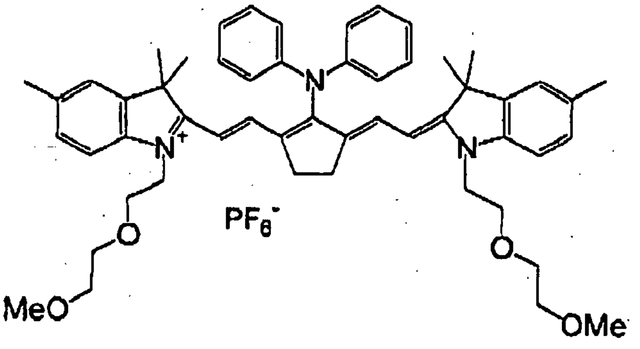

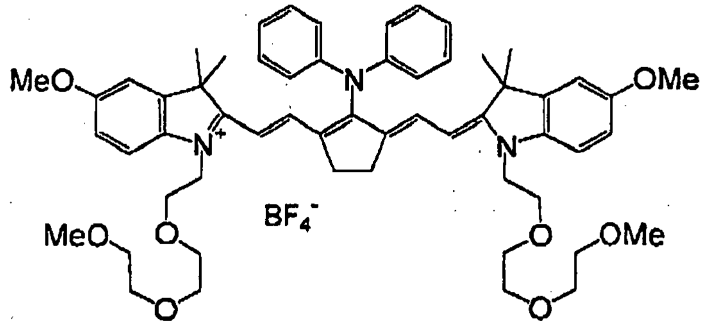

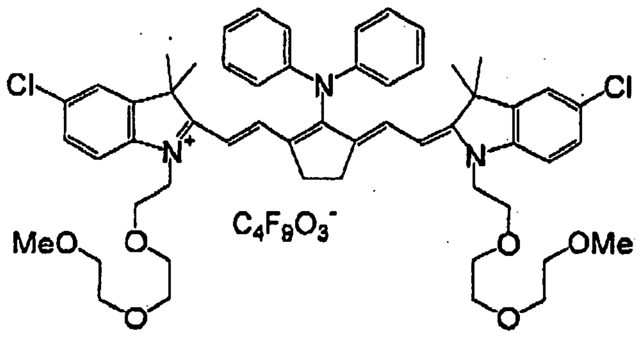

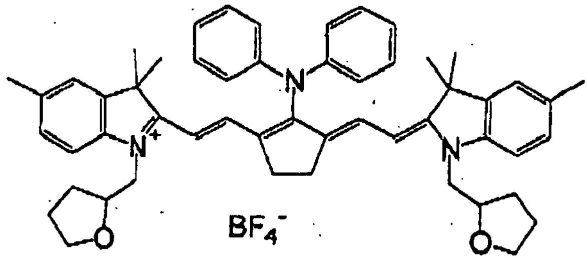

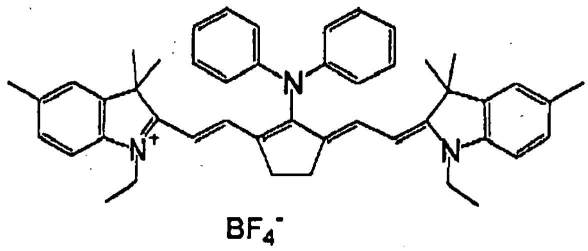

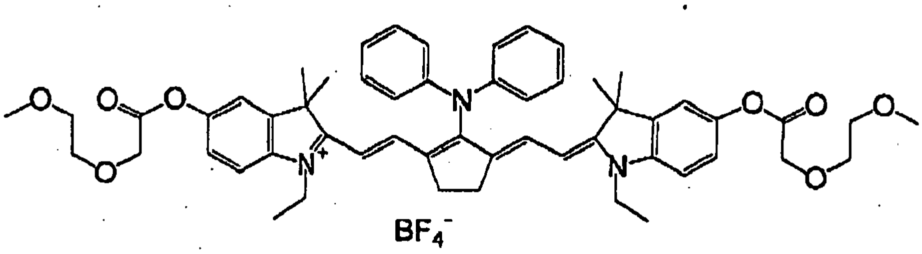

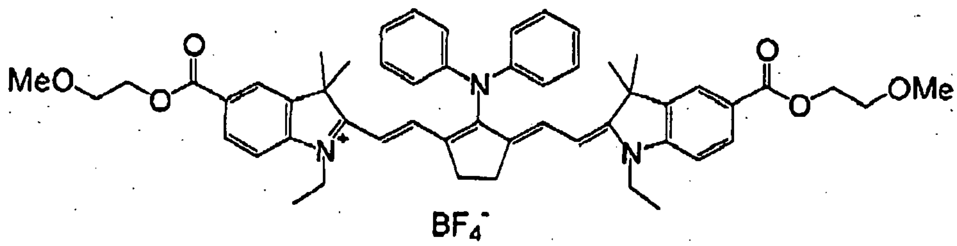

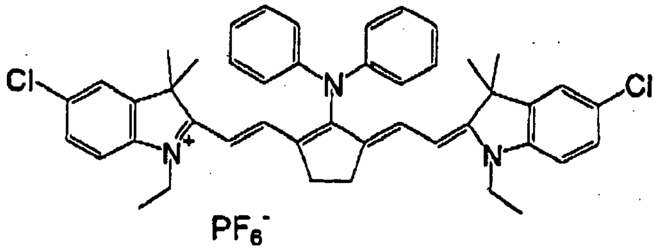

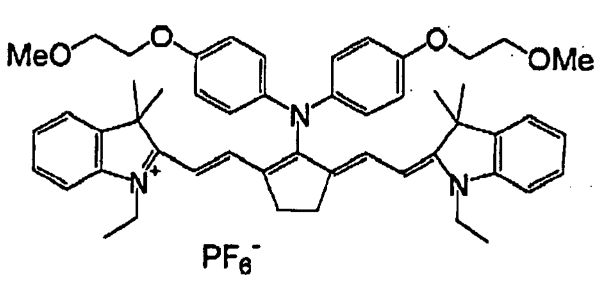

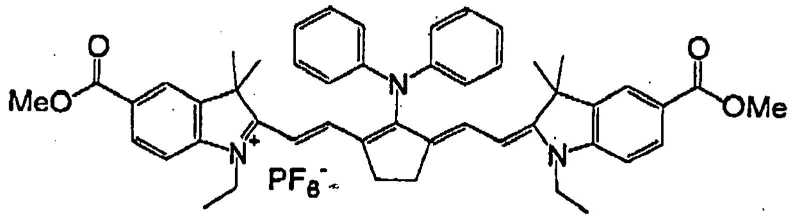

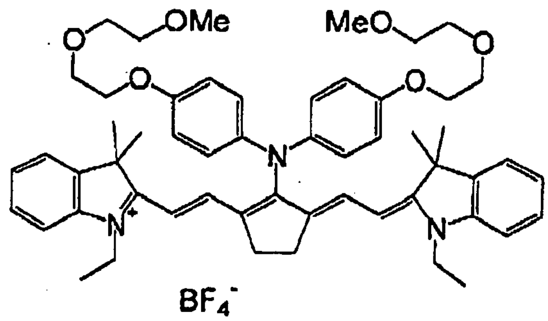

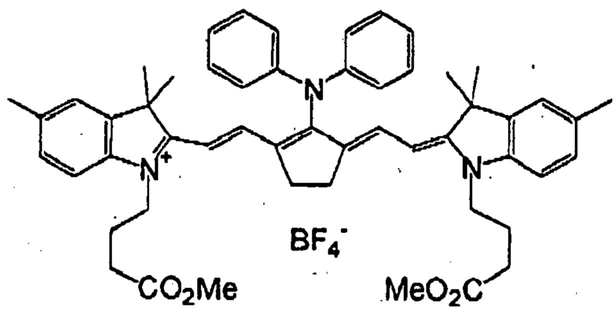

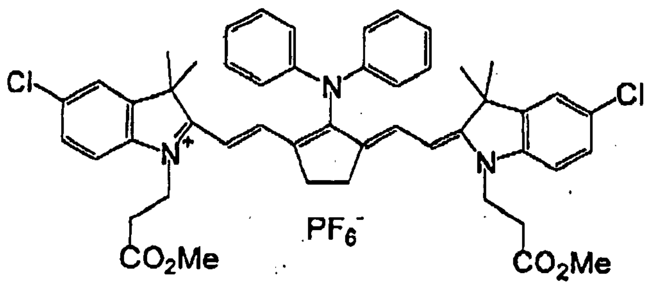

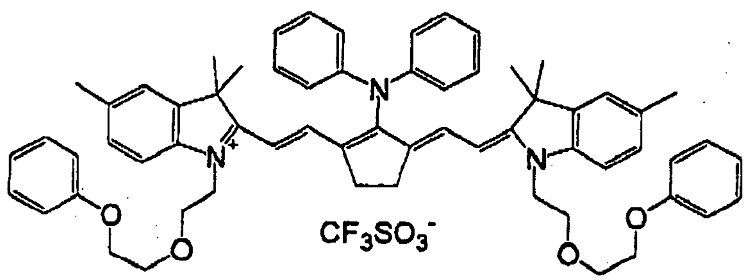

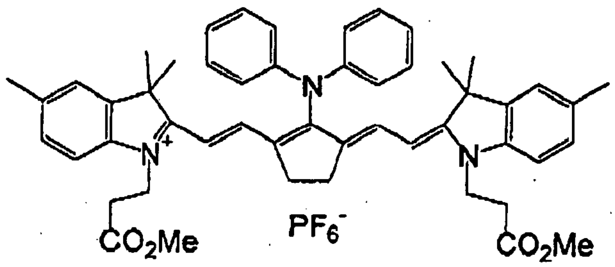

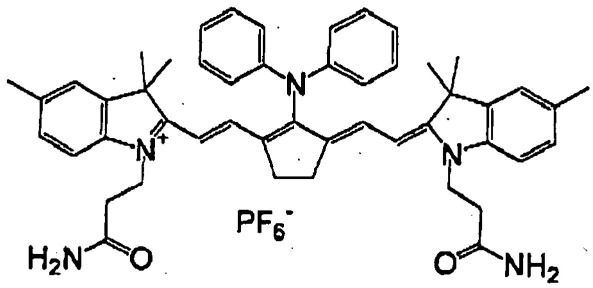

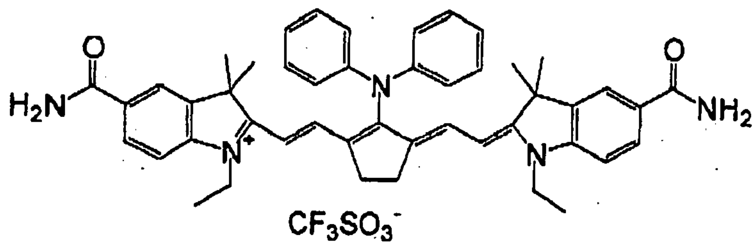

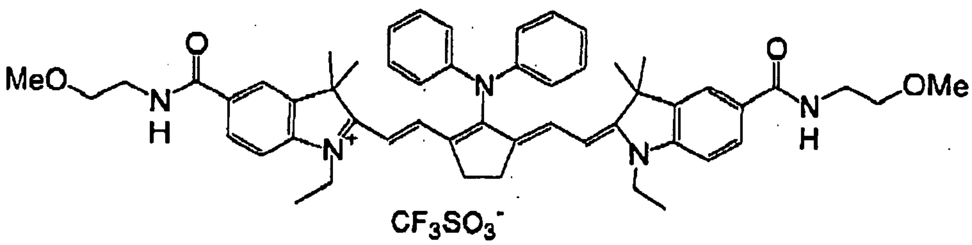

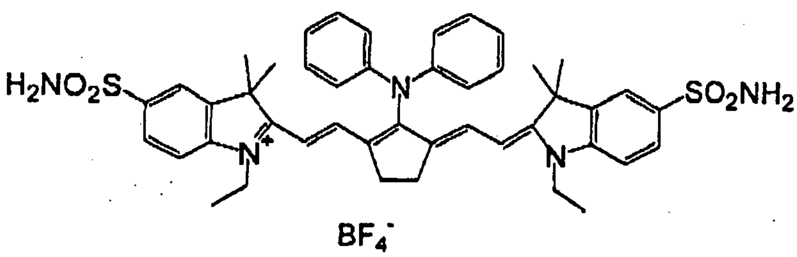

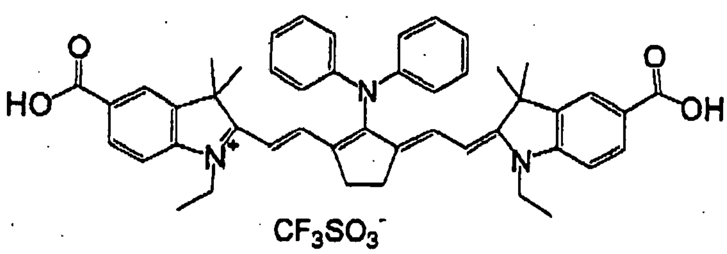

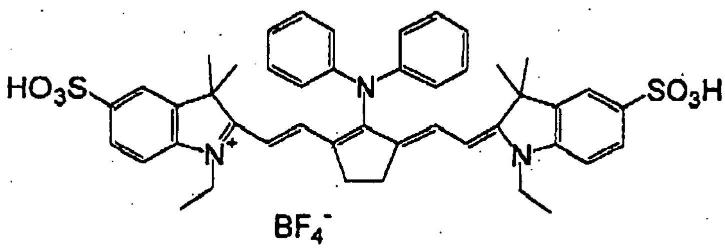

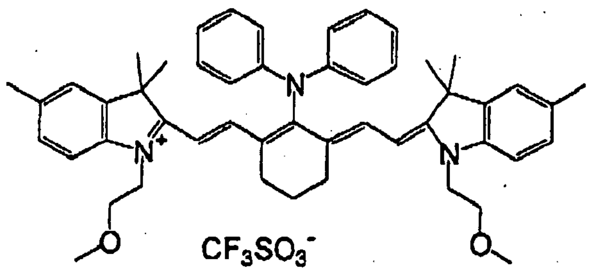

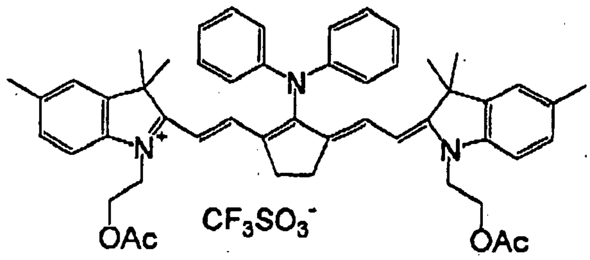

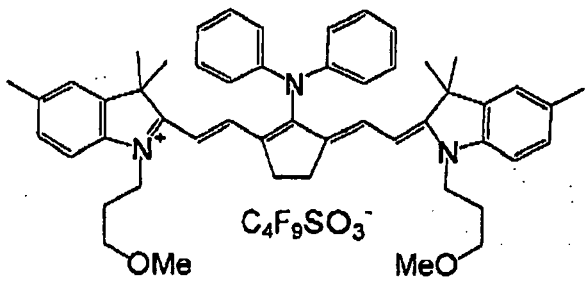

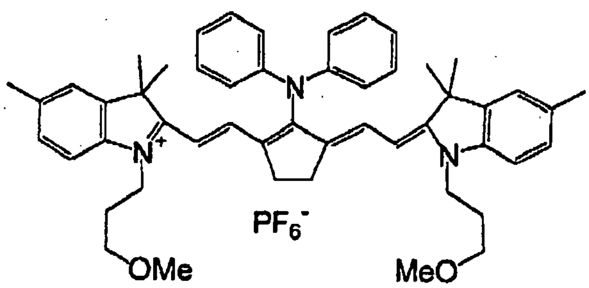

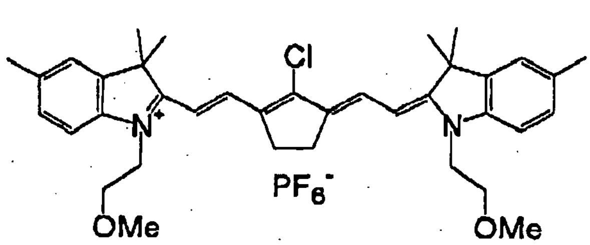

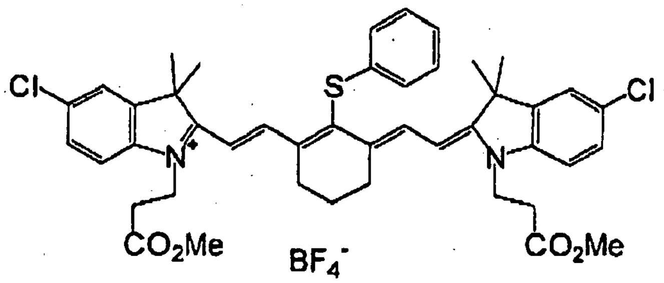

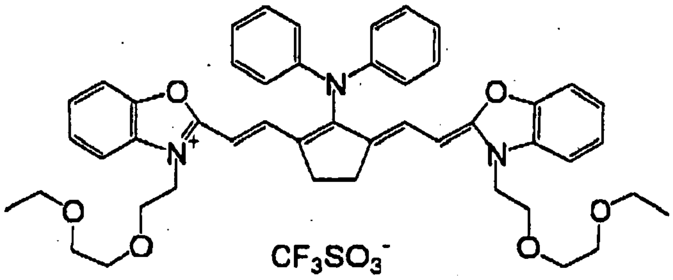

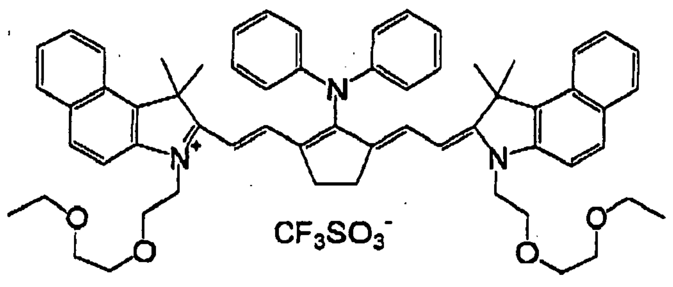

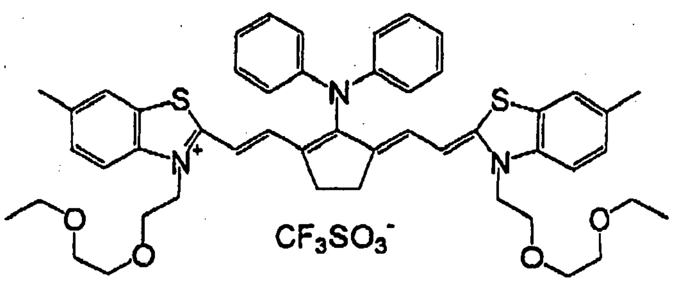

Examples of the pigment used in the invention include commercially available pigments and pigments described in Colour Index (C.I.), Saishin Ganryo Binran (Handbook of Newest Pigments) compiled by Pigment Technology Society of Japan (1977), Saishin Ganryo Oyou Gijutsu (Newest Application Technologies of Pigments), CMC Publishing Co., Ltd. (1986) and Insatsu Ink Gijutsu (Printing Ink Technology), CMC Publishing Co., Ltd (1984).

Examples of the pigment include black pigments, yellow pigments, orange pigments, brown pigments, red pigments, purple pigments, blue pigments, green pigments, fluorescent pigments, metal powder pigments and polymer-bonded dyes. Specific examples of the pigment used include insoluble azo pigments, azo lake pigments, condensed azo pigments, chelated azo pigments, phthalocyanine pigments, anthraquinone pigments, perylene and perynone pigments, thioindigo pigments, quinacridone pigments, dioxazine pigments, isoindolinone pigments, quinophthalone pigments, dying lake pigments, azine pigments, nitroso pigments, nitro pigments, natural pigments, fluorescent pigments, inorganic pigments and carbon black. Of the pigments, carbon black is preferred.

The pigment may be used without undergoing surface treatment or may be used after conducting the surface treatment. For the surface treatment, a method of coating a resin or wax on the pigment surface, a method of attaching a surfactant to the pigment surface and a method of bonding a reactive substance (for example, a silane coupling agent, an epoxy compound or a polyisocyanate) to the pigment surface. The surface treatment methods are described in Kinzoku Sekken no Seishitsu to Oyo (Properties and Applications of Metal Soap), Saiwai Shobo, Insatsu Ink Gijutsu (Printing Ink Technology), CMC Publishing Co., Ltd. (1984), and Saishin Ganryo Oyo Gijutsu (Newest Application Technologies of Pigments), CMC Publishing Co., Ltd. (1986).

A particle size of the pigment is preferably in a range from 0.01 to 10 μm, more preferably in a range from 0.05 to 1 μm, particularly preferably in a range from 0.1 to 1 μm. In the above-described range, good stability of the pigment dispersion in a coating solution for image-recording layer and good uniformity of the image-recording layer can be obtained.

As a method for dispersing the pigment, a known dispersion technique for use in the production of ink or toner can be used. Examples of the dispersing machine include an ultrasonic dispersing machine, a sand mill, an attritor, a pearl mill, a super-mill, a ball mill, an impeller, a disperser, a KD mill, a colloid mill, a dynatron, a three roll mill and a pressure kneader. The dispersing methods are described in detail in Saishin Ganryo Oyo Gijutsu (Newest Application Technologies of Pigments), CMC Publishing Co., Ltd. (1986).

The infrared absorbing agent may be added together with other components to one layer or may be added to a different layer separately provided. With respect to an amount of the infrared absorbing agent added, in the case of preparing a negative-working lithographic printing plate precursor, the amount is so controlled that absorbance of the image-recording layer at the maximum absorption wavelength in the wavelength region of 760 to 1,200 nm measured by reflection measurement is ordinarily in a range of 0.3 to 1.2, preferably in a range of 0.4 to 1.1. In the above-described range, the polymerization reaction proceeds uniformly in the thickness direction of the image-recording layer and good film strength of the image area and good adhesion of the image area to the support are achieved.

The absorbance of the image-recording layer can be controlled depending on the amount of the infrared absorbing agent added to the image-recording layer and the thickness of the image-recording layer. The measurement of the absorbance can be carried out in a conventional manner. The method for measurement includes, for example, a method of forming an image-recording layer having a thickness appropriately determined in the range of coating amount after drying required for the lithographic printing plate precursor on a reflective support, for example, an aluminum plate, and measuring reflection density of the image-recording layer by an optical densitometer or a spectrophotometer according to a reflection method using an integrating sphere.

The amount of the infrared absorbing agent added to the image-recording layer is preferably from 0.1 to 30% by weight, more preferably from 0.5 to 20% by weight, still more preferably from 1 to 10% by weight, based on the total solid content of the image-recording layer. (B2) Sensitizing dye having an absorption peak in a wavelength range of 350 to 850 nm

The sensitizing dye having an absorption peak in a wavelength range of 350 to 850 nm for use in the invention include a spectral sensitizing dye, and a dyestuff or pigment which absorbs light of a light source to cause an interaction with the photopolymerization initiator shown below.

The spectral sensitizing dye or dyestuff preferably used includes, for example, a multi-nuclear aromatic compound (for example, pyrene, peryrene or triphenylene), a xanthene (for example, Fluoresceine, Eosine, Erythrocin, Rhodamine B or Rose Bengale), a cyanine (for example, thiacarbocyanine or oxacarbocyanine), a merocyanine (for example, merocyanine or carbomerocyanine), a thiazine (for example, Thionine, Methylene Blue or Toluidine Blue), an acridine (for example, Acridine Orange, chloroflavine or acriflavine), a phthalocyanine (for example, phthalocyanine or metallo-pbthalocyanine), a porphyrin (for example, tetraphenyl porphyrin or center metal-substituted porphyrin), a chlorophyll (for example, chlorophyll, chlorophyllin or center metal-substituted chlorophyll), a metal complex, an anthraquinone (for example, anthraquinone) and a squalium (for example, squalium).

More preferable examples of the spectral sensitizing dye or dyestaff include styryl dyes as described in JP-B-37-13034, cationic dyes as described in JP-A-62-143044, quinoxalinium salts as described in JP-B-59-24147, new Methylene Blue compounds as described in JP-A-64-33104, anthraquinones as described in JP-A-64-56767, benzoxanthene dyes as described in JP-A-2-1714, acridines as described in JP-A-2-226148 and JP-A-2-226149, pyrylium salts as described in JP-B-40-28499, cyanines as described in JP-B-46-42363, benzofuran dyes as described in JP-A-2-63053, conjugated ketone dyes as described in JP-A-2-85858 and JP-A-2-216154, dyes as described in JP-A-57-10605, azocinnamylidene derivatives as described in JP-B-2-30321, cyanine dyes as described in JP-A-1-287105, xanthene dyes as described in JP-A-62-31844, JP-A-62-31848 and JP-A-62-143043, aminostyryl ketones as described in JP-B-59-28325, merocycnine dyes as described in JP-B-61-9621, dyes as described in JP-A-2-179643, merocycnine dyes as described in JP-A-2-244050, merocycnine dyes as described in JP-B-59-28326, merocycnine dyes as described in JP-A-59-89803, merocycnine dyes as described in JP-A-8-129257, and benzopyran dyes as described in JP-A-8-334897.

The content of the sensitizing dye in response to the visible light or ultraviolet laser is preferably from 0.1 to 50% by weight, more preferably from 0.5 to 30% by weight, still more preferably from 1 to 20% by weight, based on the total solid content of the image-recording layer.

<(C) Compound having at least one addition-polymenzable ethylenically unsaturated bond>

The image-recording layer according to the invention contains (C) a compound having at least one addition-polymerizable ethylenically unsaturated bond (hereinafter, also referred to as a "polymerizable compound") in order to perform an efficient curing reaction. The polymerizable compound for use in the invention is an addition-polymerizable

compound having at least one ethylenically unsaturated double bond and it is selected from compounds having at least one, preferably two or more, terminal ethylenically unsaturated double bonds. Such compounds are widely known in the field of art and they can be used in the invention without any particular limitation. The compound has a chemical form for example, a monomer, a prepolymer, specifically, a dimer, a trimer or an oligomer, or a copolymer thereof, or a mixture thereof. Examples of the monomer and copolymer thereof include unsaturated carboxylic acids (for example, acrylic acid, methacrylic acid, itaconic acid, crotonic acid, isocrotonic acid or maleic acid) and esters or amides thereof. Preferably, esters of an unsaturated carboxylic acid with an aliphatic polyhydric alcohol compound and amides of an unsaturated carboxylic acid with an aliphatic polyvalent amine compound are used. An addition reaction product of an unsaturated carboxylic acid ester or amide having a nucleophilic substituent, for example, a hydroxy group, an amino group or a mercapto group, with a monofunctional or polyfunctional isocyanate or epoxy, or a dehydration condensation reaction product of the unsaturated carboxylic acid ester or amide with a monofunctional or polyfunctional carboxylic acid is also preferably used. Furthermore, an addition reaction product of an unsaturated carboxylic acid ester or amide having an electrophilic substituent, for example, an isocyanato group or an epoxy group with a monofunctional or polyfimctional alcohol, amine or thiol, or a substitution reaction product of an unsaturated carboxylic acid ester or amide having a releasable substituent, for example, a halogen atom or a tosyloxy group with a monofunctional or polyfunctional alcohol, amine or thiol is also preferably used. In addition, compounds in which the unsaturated carboxylic acid described above is replaced by an unsaturated phosphonic acid, styrene, vinyl ether or the like can also be used.

Specific examples of the monomer, which is an ester of an aliphatic polyhydric alcohol compound with an unsaturated carboxylic acid, include acrylic acid esters, for example, ethylare glycol diacrylate, triethylene glycol diacrylate, 1,3-butanediol diacrylate, tetramethylene glycol diacrylate, propylene glycol diacrylate, neopentyl glycol diacrylate, trimethylolpropane triacaylate, trimethylolpropane tri(acryloyloxypropyl) ether, trimethylolethane triacrylate, hexanediol diacrylate, 1,4-cyclohexanediol diacrylate, tetraethylene glycol diacrylate, pentaerythritol diacrylate, pentaerythritol triacrylate, pentaerythritol tetraacrylate, dipentaerythritol diacrylate, dipentaerythritol hexaacrylate, sorbitol triacrylate, sorbitol tetraacrylate, sorbitol pentaacrylate, sorbitol hexaacrylate, tri(acryloyloxyethyl) isocyanurate, polyester acrylate oligomer or isocyanuric acid EO modified triacrylate;

methacrylic acid esters, for example, tetramethylene glycol dimethacrylate, tricthylenc glycol dimethacrylate, neopentyl glycol dimethacrylate, trimethylolpropane trimethacrylate, trimethylolethane trimethacrylate, ethylene glycol dimethacrylate, 1,3-butanediol methacrylate, hexanediol dimethacrylate, pentaerythritol dimethacrylate, pentaerythritol trimethacrylate, pentaerythritol tetramethacrylate, dipentaerythritol dimethacrylate, dipentaerythritol hexamethacrylate, sorbitol trimethacrylate, sorbitol tetramethacrylate, bis[p-(3-methayloxy-2-hydroxypropoxy)phenyl]dimethylmethane or bis[p-(methacryloxyethoxy)phenyl]dimethylmethane;

itaconic acid esters, for example, ethylene glycol diitaconate, propylene glycol diitaconate, 1,3-butanediol diitaconate, 1,4-butanediol diitaconate, tetramethylene glycol diitaconate, pentaerythritol diitaconate or sorbitol tetraitaconate; crotonic acid esters, for example, ethylene glycol dicrotonate, tetramethylene glycol dicrotonate, pentaerythritol dicrotonate or sorbitol tetradicrotonate; isocrotonic acid esters, for example, ethylene glycol diisocrotonate, pentaerythritol diisocrotonate or sorbitol tetraisocrotonate; and maleic acid esters, for example, ethylene glycol dimaleste, triethylene glycol dimaleate, pentaerythritol dimaleate and sorbitol tetramaleate.

Other examples of the ester, which can be preferably used, include aliphatic alcohol esters described in JP-B-51-47334 and JP-A-57-196231, esters having an aromatic skeleton described in JP-A-59-5240, JP-A-59-5241 and JP-A-2-226149, and esters containing an amino group described in JP-A-1-165613.

The above-described ester monomers can also be used as a mixture.

Specific examples of the monomer, which is an amide of an aliphatic polyvalent amine compound with an unsaturated carboxylic acid, include methylene bisacrylamide, methylene bismethacrylamide, 1,6-hexamethylene bisacrylamide, 1,6-hexamethylene bismethacrylamide, diethylenetriamine trisaoylamide, xylylene bisacrylamide and xylylene bismethacrylamide. Other preferred examples of the amide monomer include amides having a cyclohexylene structure described in JP-B-54-21726.

Urethane type addition polymerizable compounds produced using an addition reaction between an isocyanate and a hydroxy group are also preferably used, and specific examples thereof include vinylurethane compounds having two or more polymerizable vinyl groups per molecule obtained by adding a vinyl monomer containing a hydroxy group represented by formula (a) shown below to a polyisocyanate compound having two or more isocyanate groups per molecule, described in JP-B-48-41708.

$$CH_2=C(R^4)COOCH_2CH(R^5)OH \qquad (a)$$

wherein $R^4$ and $R^5$ each independently represents H or $CH_3$.

Also, urethane acrylates described in JP-A-51-37193, JP-B-2-32293 and JP-B-2-16765, and urethane compounds having an ethylene oxide skeleton described in JP-B-58-49860, JP-B-56-17654, JP-B-62-39417 and JP-B-62-39418 are preferably used. Furthermore, a photopolymerizable composition having remarkably excellent photosensitive speed can be obtained by using an addition polymerizable compound having an amino structure or a sulfide structure in its molecule, described in JP-A-63-277653, JP-A-63-260909 and JP-A-1-105238.

Other examples include polyfunctional acrylates and methacrylates, for example, polyester acrylates and epoxy acrylates obtained by reacting an epoxy resin with acrylic acid or methacrylic acid, described in JP-A-48-64183, JP-B-49-43191 and JP-B-52-30490. Specific unsaturated compounds described in JP-B-46-43946, JP-B-140337 and JP-B-1-40336, and vinylphosphonic acid type compounds described in JP-A-2-25493 can also be exemplified In some cases, structure containing a perfluoroalkyl group described in JP-A-61-22048 can be preferably used. Moreover, photocurable monomers or oligomers described in Nippon Secchaku Kyokaishi (Journal of Japan Adhesion Society), Vol. 20, No. 7, pages 300 to 308 (1984) can also be used.

Details of the method of using the polymerizable compound, for example, selection of the structure, individual or combination use, or an amount added, can be appropriately arranged depending on the characteristic design of the final lithographic printing plate precursor. For instance, the compound is selected from the following standpoint.

In view of the sensitivity, a structure having a large content of unsaturated groups per molecule is preferred and in many cases, a bifunctional or more functional compound is preferred. In order to increase the strength of image area, that is, hardened layer, a trifunctional or more functional compound is preferred. A combination use of compounds different in the functional number or in the kind of polymerizable unsaturated group (for example, an acrylic acid ester, a methacrylic acid ester, a styrene compound or a vinyl ether compound) is an effective method for controlling both the sensitivity and the strength.

The selection and use method of the polymerizable compound are also important factors for the compatibility and dispersibility with other components (for example, a binder polymer, a polymerization initiator or a coloring agent) in the image-recording layer. For instance, the compatibility may be improved in some cases by using the compound of low purity or using two or more kinds of the compounds in combination. A specific structure may be selected for the purpose of improving an adhesion property to a support or a protective layer described hereinafler.

The polymerizable compound (C) is preferably used in an amount from 5 to 80% by weight, more preferably from 25 to 75% by weight, based on the nonvolatile component of the image-recording layer. The polymerizable compounds may be used individually or in combination of two or more thereof. In the method of using the polymerizable compound, the structure, blend and amount added can be appropriately selected by taking account of the extent of polymerization inhibition due to oxygen, resolution, fogging property, change in refractive index, surface adhesion and the like. Further, depending on the case, a layer construction, for example, an undercoat layer or an overcoat layer, and a coating method, may also be considered.

<(E) Binder polymer>

In the image-recording layer according to the invention, a binder polymer can further be used, if desired, for the purpose, for example, of improving a film property of the image-recording layer to be formed.

A binder polymer which can be used in the invention can be selected from those heretofore known without restriction, and linear organic polymers having a film forming property are preferable. Examples of the binder polymer include acrylic resins, polyvinyl acetal resins, polyurethane resins, polyurea resins, polyimide resins, polyamide resins, epoxy resins, methacrylic resins, polystyrene resins, novolac type phenolic resins, polyester resins, synthesis rubbers and natural rubbers.

The binder polymer preferably has a crosslinkable property in order to improve the film strength of the image area. In order to impart the crosslinkable property to the binder polymer, a crosslinkable functional group, for example, an ethylenically unsaturated bond is introduced into a main chain or side chain of the polymer. The crosslinkable functional group may be introduced by copolymerixation.

Examples of the polymer having an ethylenically unsaturated bond in the main chain thereof include poly-1,4-butadiene and poly-1,4-isoprene.

Examples of the polymer having an ethylenically unsaturated bond in the side chain thereof include a polymer of an ester or amide of acrylic acid or methacrylic acid, which is a polymer wherein the ester or amide residue (R in -COOR or -CONHR) has an ethylenically unsaturated bond.

Examples of the residue (R described above) having an ethylenically unsaturated bond include $-(CH_2)_nCR^1=CR^2R^3$, $-(CH_2O)_nCH_2CR^1=CR^2R^3$, $-(CH_2CH_2O)_nCH_2CR^1=CR^2R^3$, $-(CH_2)_nNH-CO-O-CH_2CR^1-CR^2R^3$, $-(CH_2)_n-O-CO-CR^1-CR^2R^3$ and $-(CH_2CH_2O)_2-X$ (wherein $R^1$ to $R^3$ each represents a hydrogen atom, a halogen atom or an alkyl group having from 1 to 20 carbon atoms, an aryl group, alkoxy group or aryloxy group, or $R^1$ and $R^2$ or $R^1$ and $R^3$ may be combined with each other to form a ring. n represents an integer of 1 to 10. X represents a dicyclopentadienyl residue).

Specific examples of the ester residue include $-CH_2CH=H_2$ (described in JP-B-7-21633), $-CH_2CH_2O-CH_2CH=CH_2$, $-CH_2C(CH_3)=CH_2$, $-CH_2CH=CH-C_6H_5$, $-CH_2CH_2OCOCH=CH-C_6H_5$, $-CH_2CH_2-NHCOO-CH_2CH=CH_2$ and $-CH_2CH_2O-X$ (wherein X represents a dicyclopentadienyl residue).

Specific examples of the amide residue include $-CH_2CH=CH_2$, $-CH_2CH_2-Y$ (wherein Y represents a cyclohexene residue) and $-CH_2CH_2-OCO-CH=CH_2$.

The binder polymer having crosslinkable property is cured, for example, by adding a free radical (a polymerization initiating radical or a growing radical of a polymerizable compound during polymerization) to the crosslinkable functional group of the polymer and undergoing addition polymerization between the polymers directly or through a polymerization chain of the polymerizable compound to form crosslinkage between the polymer molecules. Alternately, it is cured by generation of a polymer radical upon extraction of an atom in the polymer (for example, a hydrogen atom on a carbon atom adjacent to the functional crosslinkable group) by a free radial and connecting the polymer radicals with each other to form cross-linkage between the polymer molecules.

The content of the crosslinkable group in the binder polymer (content of the radical polymerizable unsaturated double bond determined by iodine titration) is preferably from 0.1 to 10.0 mmol, more preferably from 1.0 to 7.0 mmol and most preferably from 2.0 to 5.5 mmol, based on 1 g of the binder polymer. In the above-described range, preferable sensitivity and good preservation stability can be obtained,

From the standpoint of improvement in the on-press development property, it is preferred that the binder polymer has high solubility or dispersibility in ink and/or dampening water.

In order to improve the solubility or dispersibility in the ink, the binder polymer is preferably oleophilic and in order to improve the solubility or dispersibility in the dampening water, the binder polymer is preferably hydrophilic. Therefore, it is effective in the invention that an oleophilic binder polymer and a hydrophilic binder polymer are used in combination.

The hydrophilic binder polymer preferably includes, for example, a polymer having a hydrophilic group, for example, a hydroxy group, a carboxyl group, a carboxylate group, a hydroxyethyl group, a polyoxyethyl group, a hydroxypropyl group, a polyoxypropyl group, an amino group, an aminoethyl group, an aminopropyl group, an ammonium group, an amido group, a carboxymethyl group, a sulfonic acid group or a phosphoric acid group.

Specific examples thereof include gum arabic, casein, gelatin, a starch derivative, carboxy methyl cellulose and a sodium salt thereof, cellulose acetate, sodium alginate, vinyl acetate-maleic acid copolymer, styrene-maleic acid copolymer, polyacrylic acid and a salt thereof, polymethacrylic acid and a salt thereof, a homopolymer or copolymer of hydroxyethyl methacrylate, a homopolymer or copolymer of hydroxyethyl acrylate, a homopolymer or copolymer of hydroxypropyl methacrylate, a homopolymer or copolymer of hydroxypropyl acrylate, a homopolymer or copolymer of hydroxybutyl methacrylate, a homopolymer or copolymer of hydroxybutyl acrylate, a polyethylene glycol, a hydroxypropylene polymer, a polyvinyl alcohol, a hydrolyzed polyvinyl acetate having a hydrolysis degree of 60% by mole or more, preferably 80% by mole or more, a polyvinyl formal, a polyvinyl butyral, a polyvinyl pyrrolidone, a homopolymer or copolymer of acrylamide, a homopolymer or polymer of methacrylamide, a homopolymer or copolymer of N-methylolacrylamide, a polyvinyl pyrrolidone, an alcohol-soluble nylon, a polyether of 2,2-bis-(4-hydroxyphenyl)propane and epichlorohydrin.

The weight average molecular weight of the binder polymer is preferably 5,000 or more, more preferably from 10,000 to 300,000. The number average molecular weight of the binder polymer is preferably 1,000 or more, more preferably from 2,000 to 250,000. The polydispersity (weight average molecular weight/number average molecular weight) thereof is preferably from 1.1 to 10.

The binder polymer may be any of a random polymer and a block polymer, and preferably a random polymer. The binder polymers may be used individually or as a mixture of two or more thereof.

The binder polymer is available by purchasing a commercial product or synthesizing according to a conventionally known method. A solvent used for the synthesis include, for example, tetrahydrofuran, ethylene dichloride, cyclohexanone, methyl ethyl ketone, acetone, methanol, ethanol, ethylene glycol monomethyl ether, ethylene glycol monoethyl ether, 2-methoxyethyl acetate, diethylene glycol dimethyl ether, 1-methoxy-2-propanol, 1-methoxy-2-propylacetate, N,N-dimethylformamide, N,N-dimetInylacctoar+de, toluene, ethyl acetate, methyl lactate, ethyl lactate, dimethylsulfoxide and water. The solvents may be used individually or as a mixture of two or more thereof.

As a radical polymerization initiator used for the synthesis of binder polymer, a known compound, for example, an azo type initiator or a peroxide initiator can be employed.

The content of the binder polymer (E) is from 0 to 90% by weight, preferably from 0 to 80% by weight, and more preferably from 0 to 70% by weight, based on the total solid content of the image-recording layer. In the above-described range, good strength of the image area and preferable image-forming property can be obtained.

It is preferred that the polymerizable compound (C) and the binder polymer (E) are used in a weight ratio of 0.5/1 to 4/1.

<Other components>

Into the image-recording layer of the lithographic printing plate precursor according to the invention, various compounds can further be incorporated depending on the purposes to the extent that they do not damage the effect of

the invention, in addition to the above-described components (A) to (D) and the other polymerization initiator and binder polymer (E) used, if desired.

<Surfactant>

In the invention, it is preferred to use a surfactant in the image-recording layer in order to promote the on-press development property at the start of printing and to improve the state of coated surface. The surfactant used includes, for example, a nonionic surfactant, an anionic surfactant, a cationic surfactant, an amphoteric surfactant and a fluorine-based surfactant. The surfactants may be used individually or in combination of two or more thereof.

The nonionic surfactant used in the invention is not particular restricted, and those hitherto known can be used. Examples of the nonionic surfactant include polyoxyethylene alkyl ethers, polyoxyethylene alkyl phenyl ethers, polyoxyethylene polystyryl phenyl ethers, polyoxyethylene polyoxypropylene alkyl ethers, glycerin fatty acid partial esters, sorbitan fatty acid partial esters, pentaerythritol fatty acid partial esters, propylene glycol monofatty acid esters, sucrose fatty acid partial esters, polyoxyethylene sorbitan fatty acid partial esters, polyoxyethylene sorbitol fatty acid partial esters, polyethylene glycol fatty acid esters, polyglycerol fatty acid partial esters, polyoxyethylenated castor oils, polyoxyethylene glycerol fatty acid partial esters, fatty acid diethanolamides, N,N-bis-2-hydroxyalkylamines, polyoxyethylene alkylamines, triethanolamine fatty acid esters, trialylamine oxides, polyethylene glycols, and copolymers of polyethylene glycol and polypropylene glycol.

The anionic surfactant used in the invention is not particularly restricted and those hitherto known can be used. Examples of the anionic surfactant include fatty acid salts, abietic acid salts, hydroxyalkanesulfonic acid salts, alkanesulfonic acid salts, dialkylsulfosuccinic ester salts, straight-chain alkylbenzenesulfonic acid salts, branched alkylbenzenesulfonic acid salts, alkylnaphthalenesulfonic acid salts, alkylphenoxypolyoxyethylene propylsulfonic acid salts, polyoxyethylene alkylsulfophenyl ether salts, N-methyl-N-olcyltaurine sodium salt, N-alkylsulfosuccinic monoamide disodium salts, petroleum sulfonic acid salts, sulfated beef tallow oil, sulfate ester slats of fatty acid alkyl ester, alkyl sulfate ester salts, polyoxyethylene alkyl ether sulfate ester salts, fatty acid monoglyceride sulfate ester salts, polyoxyethylene alkyl phenyl ether sulfate ester salts, polyoxyethylene styrylphenyl ether sulfate ester salts, alkyl phosphate ester salts, polyoxyethylene alkyl ether phosphate ester salts, polyoxyethylene alkyl phenyl ether phosphate ester salts, partial saponification products of styrene/maleic anhydride copolymer, partial saponification products of olefin/maleic anhydride copolymer and naphthalene sulfonate formalia condensates.

The cationic surfactant used in the invention is not particularly restricted and those hitherto known can be used. Examples of the cationic surfactant include alkylamine salts, quaternary ammonium salts, polyoxyethylene alkyl amine salts and polyethylene polyamine derivatives.

The amphoteric surfactant used in the invention is not particularly restricted and those hitherto known can be used. Examples of the amphoteric surfactant include carboxybetaines, aminocarboxylic acids, sulfobetaines, aminosulfuric esters, and imidazolines.

In the surfactants described above, the term "polyoxyethylene" can be replaced with "polyoxyalkylene", for example, polyoxymethylene, polyoxypropylene or polyoxybutylene, and such surfactants can also be used in the invention.

Further, a preferable surfactant includes a fluorine-based surfactant containing a perfluoroalkyl group in its molecule. Examples of the fluorine-based surfactant include an anionic type, for example, perfluoroalkyl carboxylates, perfluoroalkyl sulfonates or perfluoroalkylphosphates; an amphoteric type, for example, perfluoroalkyl betaines; a cationic type, for example, perfluoroalkyl trimethyl ammonium salts; and a nonionic type, for example, perfluoroalkyl amine oxides, perfluoroalkyl ethylene oxide adducts, oligomers having a perfluoroalkyl group and a hydrophilic group, oligomers having a perfluoroalkyl group and an oleophilic group, oligomers having a perfluoroalkyl group, a hydrophilic group and an oleophilic group or urethanes having a perfluoroalkyl group and an oleophilic group. Further, fluorine-based surfactant described in JP-A-62-170950, JP-A-62-226143 and JP-A-60-168144 are also preferably exemplified.

The surfactants can be used individually or in combination of two or more thereof

The content of the surfactant is preferably from 0.001 to 10% by weight, more preferably from 0.01 to 7% by weight, based on the total solid content of the image-recording layer.

<Coloring agent>

According to the invention, a dye having a large absorption in the visible region can be used as a coloring agent of the image formed. Specifically, the dye includes Oil yellow #101, Oil yellow #103, Oil pink #312, Oil green BG, Oil blue BOS, Oil blue #603, Oil black BY, Oil black BS, Oil black T-505 (produced by Orient Chemical Industries, Ltd.), Victoria pure blue, Crystal violet (CI42555), Methyl violet (CI42535), Ethyl violet, Rhodamine B (CI45170B), Malachite green (CI42000), Methylene blue (CI52015) and dyes described in JP-A-62-293247. Further, a pigment, for example, a phthalocyaninc pigment, an azo pigment, carbon black or titanium oxide can also preferably be used.

It is preferred to add the coloring agent since distinction between the image area and the non-image area is easily conducted after the formation of image. The amount of the coloring agent added is preferably from 0.01 to 10% by weight based on the total solid content of the image-recording layer.

<Print-out agent>

To the image-recording layer according to the invention, a compound causing discoloration by an acid or a radical can be added in order to form a print-out image.

According to the invention, although such a print-out agent is not always necessary because the image excellent in the plate inspection property is formed in the exposed area due to the synergetic effect of the specific compound and the infrared absorbing agent, the print-out agent can be used together for the purpose of improving the plate inspection property

As a compound used for such a purpose, for example, dyes of diphenylmethane type, triphenylmethane type, thiazine type, oxazine type, xanthene type, anthraquinone type, iminoquinone type, azo type and azomethine type are effectively used.

Specific examples thereof include dyes, for example, Brilliant green, Ethyl violet, Methyl green, Crystal violet, basic Fuchsine, Methyl violet 2B, Quinaldine red, Rose Bengal, Methanyl yellow, Thimol sulfophthalein, Xylenol blue, Methyl orange, Paramethyl red, Congo red, Benzo purpurin 4B, $\alpha$-Naphthyl red, Nile blue 2B, Nile blue A, Methyl violet, Malachite green, Parafuchsine, Victoria pure blue BOH (produced by Hodogaya Chemical Co., Ltd.), Oil blue #603 (produced by Orient Chemical Industries, Ltd.), Oil pink #312 (produced by Orient Chemical Industries, Ltd.), Oil red 5B (produced by Orient Chemical Industries, Ltd.), Oil scarlet #308 (produced by Orient Chemical Industries, Ltd.), Oil red OG (produced by Orient Chemical Industries, Ltd.), Oil red RR (produced by Orient Chemical Industries, Ltd.), Oil green #502 (produced by Orient Chemical Industries, Ltd.), Spiron Red BEH special (produced by Hodogaya Chemical Co., Ltd.), m-Cresol purple, Cresol red, Rhodamine B, Rhodamine 6G, Sulfo rhodamine B, Auramine, 4-p-diethylaminophenyliminonaphthoquione, 2-carboxyanilino-4-p-diethylaminophenyliminonaphthoquinone, 2-carboxystenylamino-4-p-N,N-bis(hydroxyethyl)aminophenyliminonaphthoquinone, 1-phenyl-3-methyl-4-p-diethylaminophenylimino-5-pyrazolon or 1-$\beta$-naphtyl-4-p-diethylaminophenylimino-5-pyrazolon, and a leuco dye, for example, p, p', p''-hexamethyltriaminotriphenylmethane (leuco crystal violet) or Pergascript Blue SRB (produced by Ciba Geigy Ltd.).

In addition to those described above, a leuco dye known as a material for heat-sensitive paper or pressure-sensitive paper is also preferably used. Specific examples thereof include crystal violet lactone, malachite green lactone, benzoyl leuco methylene blue, 2-(N-phenyl-N-methylamino)-6-(N-p-tolyl-N-ethyl)aminofluoran, 2-anilino-3-methyl-6-(n-ethyl-p-tolidino)fluoran, 3,6-dimethoxyfluoran, 3-(N,N-diethylamino)-5-methyl-7-(N,N-dibenzylamino)fluoran, 3-(N-cyclohexyl-N-methylamino)-6-methyl-7-anilinofluoran, 3-(N-N-diethylamino)-6-methyl-7-anilinofluoran, 3-(N,N-diethylamino)-6-methyl-7-xylidinofluoran, 3-(N,N-diethylamino)-6-methyl-7-chlorofluoran, 3-(N,N-diethylamino)-6-methoxy-7-aminofluoran, 3-(N,N-diethylanino)-7-(4-chloroanilino)fluoran, 3-(NN-diethylamino)-7-chlorofluoran, 3-(N,N-diethylamino)-7-benzylaminofluoran, 3-(N,N-diethylamino)-7,8-benzofluoran, 3-(N,N-dibutylamino)-6-methyl-7-anilinofluoran, 3-(N,N-dibutylamino)-6-methyl-7-xylidinofluoran, 3-pipelidino-6-methyl-7-anilinofluoran, 3-pyrolidino-6-methyl-7-anilinofluoran, 3,3-bis(1-ethyl-2-methylindol-3-yl)phthalide, 3,3-bis(1-n-butyl-2-methylindol-3-yl)phthalide, 3,3-bis(p-dimethylaminophenyl)-6-dimethylamiaophthalide, 3-(4-diethylamino-2-ethoxyphenyl)-3-(1-ethyl-2-methylindol-3-yl)-4-phthalide and 3-(4-diethylaminophenyl)-3-(1-ethyl-2-methylindol-3-yl)phthalide.

The dye discolored by an acid or radical is preferably added in an amount of 0.01 to 10% by weight based on the solid content of the image-recording layer.

<polymerization inhibitor>

It is preferred to add a small amount of a thermal polymerization inhibitor to the image-recording layer according to the invention in order to inhibit undesirable thermal polymerization of the polymerizable compound (C) during the production or preservation of the image-recording layer.

The thermal polymerization inhibitor preferably includes, for example, hydroquinone, p-methoxyphenol, di-tert-butyl-p-cresol pyrogallol, tert-butyl catechol, benzoquinone, 4,4'-thiobis(3-methyl-6-tert-butylpbenol), 2,2'-methylenebis(4-methyl-6-tert-butylphenol) and N-nitroso-N-phenylhydroxylamine aluminum salt.

The amount of the thermal polymerization inhibitor added is preferably from about 0.01 to about 5% by weight based on the total solid content of the image-recording layer.

<Higher fatty acid derivative>

To the image-recording layer according to the invention, a higher fatty acid derivative, for example, behenic acid or behenic acid amide may be added to localize on the surface of the image-recording layer during a drying step

after coating in order to avoid polymerization inhibition due to oxygen. The amount of the higher fatty acid derivative added is preferably from about 0.1 to about 10% by weight based on the total solid content of the image-recording layer.

<Plasticizer>

The image-recording layer according to the invention may also contain a plasticizer in order to improve the on-press development property. The plasticizer preferably includes, for example, a phthalic acid ester, e.g., diemthylphthalate, diethylphthalate, dibutylphthalate, diisobutylphthalate, dioctylphthalate, octylcaprylphthalate, dicyclohexylphthalate, ditridecylphthalate, butylbenzylphthalate, diisodecylphthalate or diallylphthalate; a glycol ester, e.g., dimethylglycolphthalate, ehtylphtalylethylglycolate, methylphthalylethylglycolate, butylphthalylbutylglycolate or triethylene glycol dicaprylate ester; a phosphoric acid ester, e.g., tricresylphosphate or triphenylphosphate; an aliphatic dibasic acid ester, e.g., diisobutyladipate, dioctyladipate, dimethylsebacaie, dibutylsebacate, dioctylazelate or dibutylmaleate; polyglycidylmethacrylate, triethyl citrate, glycerin triacetyl ester and butyl laurate.

The amount of the plasticizer is preferably about 30% by weight or less based on the total solid content of the image-recording layer.

<Fine inorganic particle>

The image-recording layer according to the invention may contain fine inorganic particle in order to increase the cured film strength in the image area and to improve the on-press development property in the non-imaging area.

The fine inorganic particle preferably includes, for example, silica, alumina, magnesium oxide, titanium oxide, magnesium carbonate, calcium alginate and a mixture thereof. Even if the fine inorganic particle has no light to heat converting property, it can be used, for example, for strengthening the film or enhancing interface adhesion due to surface roughening.

The fine inorganic particle preferably has an average particle size from 5 nm to 10 μm and more preferably from 0.5 to 3μm. In the above-described range, it is stably dispersed in the image-recording layer, sufficiently maintains the film strength of the image-recording layer and can fom the non-imaging area excellent in hydrophilicity and prevented from stain during printing.

The fine inorganic particle described above is easily available as a commercial product, for example, colloidal silica dispersion.

The amount of the fine inorganic particle added is preferably 20% by weight or less and more preferably 10% by weight or less based on the total solid content of the image-recording layer.

<Hydrophilic low molecular weight compound>

The image-recording layer according to the invention may contain a hydrophilic low molecular weight compound in order to improve the on-press development property. The hydrophilic low molecular weight compound includes a water soluble organic compound, for example, a glycol compound, e.g., ethylene glycol, diethylene glycol, triethylene glycol, propylene glycol, dipropylene glycol or tripropylene glycol, or an ether or ester derivative thereof, a polyhydroxy compound, e.g., glycerine or pentaerythritol, an organic amine compound, e.g., triethanol amine, diethanol amine or monoethanol amine, or a salt thereof, an organic sulfonic acid compound, e.g., toluene sulfonic acid or benzene sulfonic acid, or a salt thereof, an organic phosphonic acid compound, e.g., phenyl phosphonic acid, or a salt thereof, an organic carboxylic acid, e.g., tartaric acid, oxalic acid, citric acid, malic acid, lactic acid, gluconic acid or an amino acid, or a salt thereof.

<Phosphonium compound>

According to the lithographic printing plate precursor of the invention, a phosphonium compound may be added to the image-recording layer and/or protective layer in order to improve the ink receptivity. As the phosphonium compound, a phosphonium compound represented by formula (iv) shown below described in JP-A-2006-297907 is exemplified. In formula (iv), $R_1$ to $R_4$ each independently represents an alkyl group, an alkenyl group, an alkynyl group, a cycloalkyl group, an alkoxy group, an aryl group, an aryloxy group, an alkylthio group or a heterocyclic group each of which may have a substituent or a hydrogen atom. Alternatively, at least two of $R_1$ to $R_4$ may be combined with each other to from a ring. $X^-$ represents a counter anion. Specific examples of the phosphonium compound represented by formula (iv) include tetrabutylphosphonium chloride, tetra-tert-butylphosphonium bromide, tetraphenylphosphonium bromide, methyltriphenylphosphonium bromide, hexyltriphenylphosphonium bromide, benzyltriphenylphosphonium bromide and allyltnphenylphosphonium bromide.

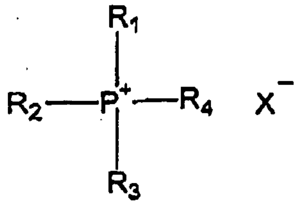 (iv)

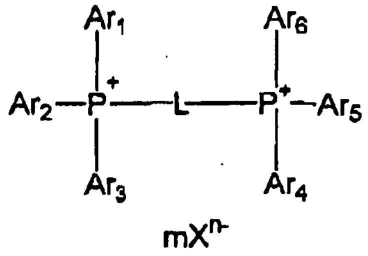 (v)

Further, as another preferable phosphonium compound, a compound represented by formula (v) shown above is exemplified. In formula (v), $Ar_1$ to $Ar_6$ each independently represents an aryl group or a heterocyclic group, L represents a divalent connecting group, $X^n$represents a n-valent counter anion, n represents an integer of 1 to 3, and m represents a number satisfying n x m = 2. The aryl group preferably includes, for example, a phenyl group, a naphthyl group, a tolyl group, a xylyl group, a fluorophenyl group, a chlorophenyl group, a bromophenyl group, a methoxyphenyl group, an ethoxyphenyl group, a dimethoxyphenyl group, a methoxycarbonylphenyl group and a dimethylaminophenyl group. The heterocyclic group preferably includes, for example, a pyridyl group, a quinolyl group, a pirimidinyl group, a thienyl group and a furyl group.

L is preferably a connecting group having from 6 to 15 carbon atoms, and more preferably a connecting group having from 6 to 12 carbon atoms.

Preferable examples of the counter anion represented by $X^{n-}$ include a halogen anion, for example, $Cl^-$, $Br^-$ or $I^-$, a sulfate anion, a carboxylate anion, a sulfate anion, $PF_6^-$, $BF_4^-$ and a perchlorate anion. Among them, a halogen anion, for example, $Cl^-$, $Br^-$ or $I^-$, a sulfate anion or a carboxylate anion is particularly preferable.

Specific examples of the phosphonium compound represented by formula (v) are set forth below.

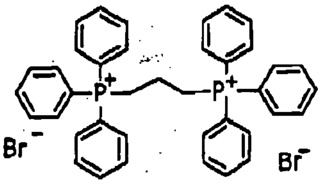

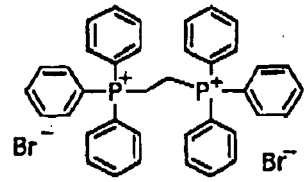

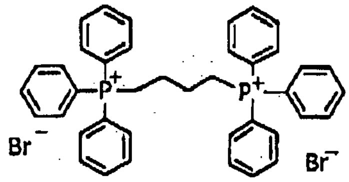

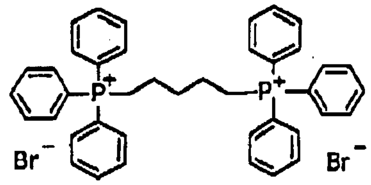

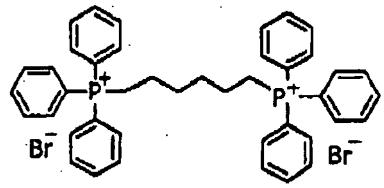

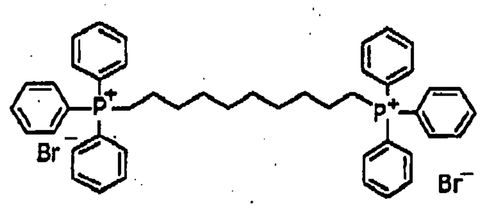

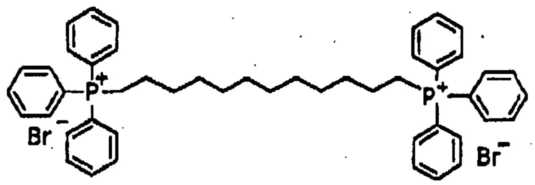

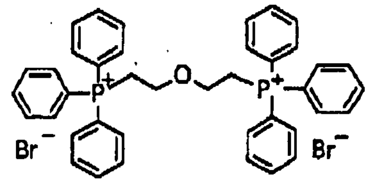

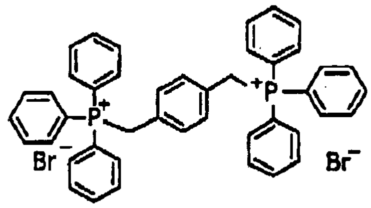

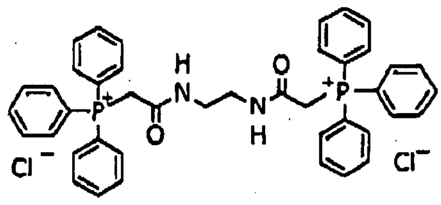

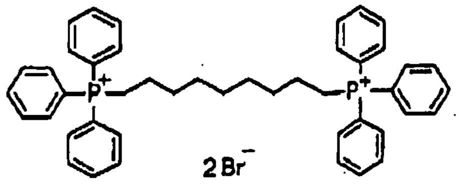

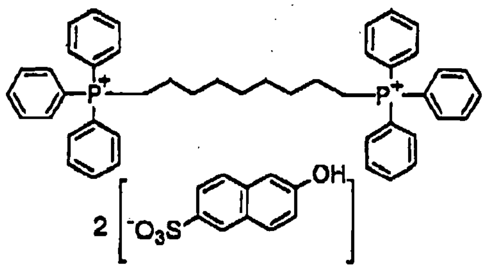

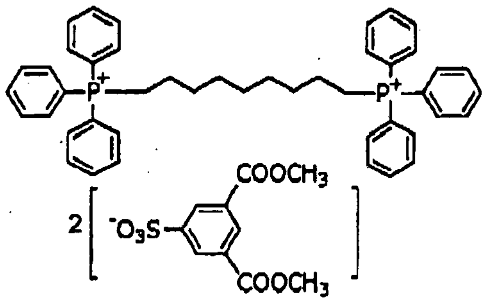

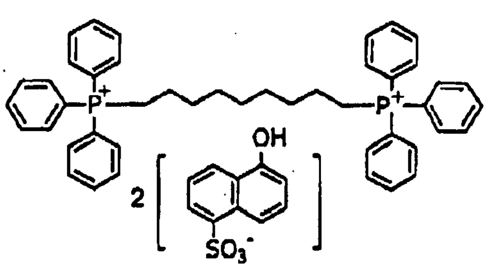

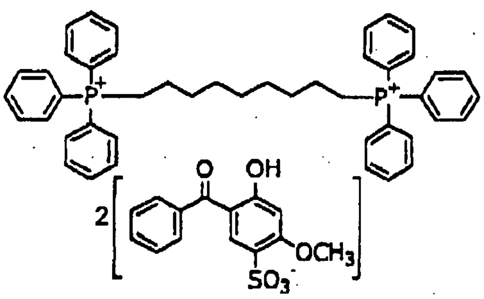

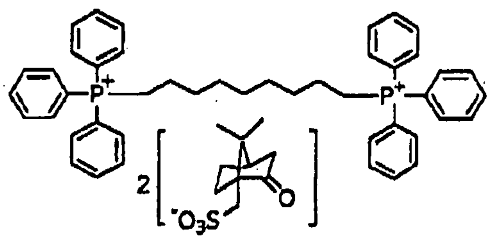

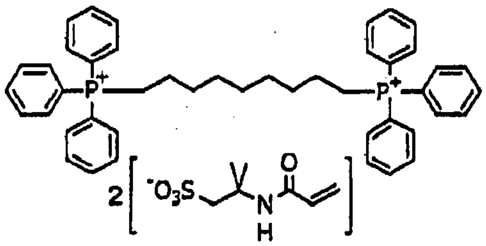

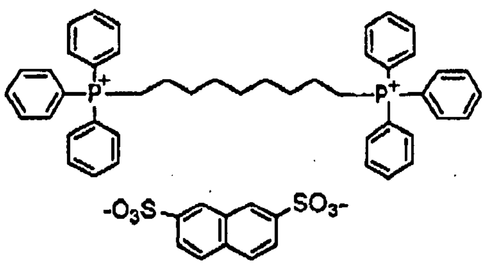

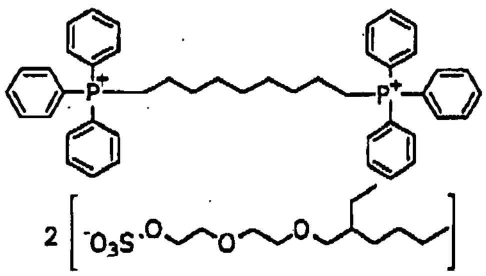

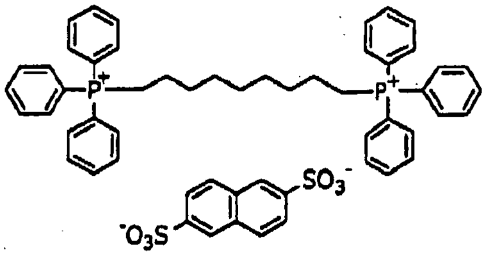

The amount of the phosphonium compound added to the image-recording layer and/or protective layer is preferably from 0.01 to 20% by weight, more preferably from 0.05 to 10% by weight, most preferably from 0.1 to 5% by weight, based on the solid content of each of the layer. In the above-described range, preferable ink receptivity is achieved.

<(F) Microcapsule or microbe>

In the invention, several embodiments can be employed in order to incorporate the above-described constituting components (A) to (D) of the image-recording layer and other constituting components described above into the image-recording layer. One embodiment is an image-recording layer of molecular dispersion type prepared by dissolving the constituting components in an appropriate solvent to coat as described, for example, in JP-A-2002-287334. Another embodiment is an image-recording layer of microcapsule type prepared by encapsulating all or part of the constituting components into microcapsule to incorporate into the image-recording layer as described, for example, in JP-A-2001-277740 and JP-A-2001-277742. In the image-recording layer of microcapsule type, the constituting components may be present outside the microcapsules. It is a more preferable embodiment of the Image-recording layer of microcapsule type that hydrophobic constituting components are encapsulated in microcapsules and hydrophilic components are present outside the microcapsules. A still another embodiment is an image-recording layer containing a crosslinked resin particle, that is, a microgel. The microgel can contain a part of the constituting components inside and/or on the surface thereof. Particularly, an embodiment of a reactive microgel containing the polymerizable compound on the surface thereof is preferable in view of the image-forming sensitivity and printing durability.
In order to achieve more preferable on-press development property, the image-recording layer is preferably the image-recording layer of microcapsule type or microgel type.
As a method of microencapsulation or microgelation of the constituting components of the image-recording layer, known methods can be used.
Methods of producing the microcapsule include, for example, a method of utilizing coacervation described in U.S. Patents 2,800,457 and 2,800,458, a method of using interfacial polymerization described in U.S. Patent 3,287,154, JP-B-38-19574 and JP-B-42-446, a method of using deposition of polymer described in U.S. Patents 3,418,250 and 3,660,304, a method of using an isocyanate polyol wall material described in U.S. Patent 3,796,669, a method of using an isocyanate wall material described in U.S. Patent 3,914,511, a method of using a urea-formaldehyde-type or urea-formaldehyde-resorcinol-type wall-forming material described in U.S. Patens 4,001,140, 4,087,376 and 4,089,802, a method of using a wall material, for example, a melamine-formaldehyde resin or hydroxycellulose described in U.S. Patent 4,025,445, an in-situ method by monomer polymerization described in JP-B-36-9163 and JP-B-51-9079, a spray drying method described in British Patent 930,422 and U.S. Patent 3,111,407, and an electrolytic dispersion cooling method described in British Patents 952,807 and 967,074, but the invention should not be construed as being limited thereto.
A preferable microcapsule wall used in the invention has three-dimensional crosslinking and has a solvent-swellable property. From this point of view, a preferable wall material of the microcapsule includes polyurea, polyurethane, polyester, polycarbonate, polyamide and a mixture thereof, and polyurea and polyurethane are particularly preferred. Further, a compound having a crosslinkable functional group, for example, an ethylenically unsaturated bond, capable of being introduced into the binder polymer described above may be introduced into the microcapsule wall.
On the other hand, methods of preparing the microgel include, for example, a method of utilizing granulation by interfacial polymerization described in JP-B-38-19574 and JP-B-42-446 and a method of utilizing granulation by dispersion polymerization in a non-aqueous system described in JP-A-5-61214, but the invention should not be construed as being limited thereto.
To the method utilizing interfacial polymerization, known production methods of microcapsule can be applied.
The microgel preferably used in the invention is granulated by interfacial polymerization and has three-dimensional crosslinking. From this point of view, a preferable material to be used includes polyurea, polyurethane, polyester, polycarbonate, polyamide and a mixture thereof, and polyurea and polyurethane are particularly preferred.
The average particle size of the microcapsule or microgel is preferably from 0.01 to 3.0μ m, more preferably from 0.05 to 2.0 μm, and particularly preferably from 0.10 to 1.0 μm. In the above-described range, favorable resolution and good preservation stability can be achieved.

[Formation of image-recording layer]

The image-recording layer according to the invention is formed by dissolving or dispersing each of the necessary constituting components described above in a solvent to prepare a coating solution for image-recording layer and coating the solution on an appropriate support. The solvent used include, for example, ethylene dichloride, cyclohexanone, methyl ethyl ketone, methanol, ethanol, propanol, ethylene glycol monomethyl ether, 1-methoxy-2-propanol, 2-methxyethyl acetate, 1-methoxy-2-propyl acetate, dimethoxyethane, methyl lactate, ethyl lactate, NN-dimethylacetoamide,

N,N-dimethylfornamide, tetramethylurea, N-methylpyrrolidone, dimethylsulfoxide, sulfolane, γ-butyrolactone, toluene and water, but the invention should not be construed as being limited thereto.

The solvents may be used individually or as a mixture. The solid content concentration of the coating solution is preferably from 1 to 50% by weight.

The image-recording layer according to the invention may also be formed by preparing plural coating solutions by dispersing or dissolving the same or different components described above into the same or different solvents and conducting repeatedly the coating and drying plural times.

The coating amount of the image-recording layer (solid content) formed on a support after drying may be varied according to the intended purpose but is preferably from 0.3 to 3.0 $g/m^2$. In the above-described range, preferable sensitivity and good film property of the image-recording layer can be achieved.

Various methods can be used for the coating. Examples of the coating method include bar coater coating, spin coating, spray coating, curtain coating, dip coating, air knife coating, blade coating and roll coating.

[Support]

A support for use in the lithographic printing plate precursor according to the invention is not particularly restricted as long as it is a dimensionally stable plate-like material. The support includes, for example, paper, paper laminated with plastic (for example, polyethylene, polypropylene or polystyrene), a metal plate (for example, aluminum, zinc or copper plate), a plastic film (for example, cellulose diacetate, cellulose triacetate, cellulose propionate, cellulose butyrate, cellulose acetate butyrate, cellulose nitrate, polyethylene terephthalate, polyethylene, polystyrene, polypropylene, polycarbonate or polyvinyl acetal film) and paper or a plastic film laminated or deposited with the metal described above. A preferred support includes a polyester film and an aluminum plate. Among them, the aluminum plate is preferred since it has good dimensional stability and is relatively inexpensive.

The aluminunn plate includes a pure aluminum plate, an alloy plate comprising aluminum as a main component and containing a trace amount of hetero elements and a thin film of aluminum or aluminum alloy laminated with plastic. The hetero element contained in the aluminum alloy includes, for example, silicon, iron, manganese, copper, magnesium, chromium, zinc, bismuth, nickel and titanium. The content of the hetero element in the aluminum alloy is preferably 10% by weight or less. Although a pure aluminum plate is preferred in the invention, since completely pure aluminum is difficult to be produced in view of the refining technique, the aluminum plate may slightly contain the hetero element. The composition is not specified for the aluminum plate and those materials conventionally known and used can be appropriately utilized.

The thickness of the support is preferably from 0.1 to 0.6 mm, more preferably from 0.15 to 0.4 mm, and still more preferably from 0.2 to 0.3 mm.

In advance of the use of aluminum plate, a surface treatment, for example, roughening treatment or anodizing treatment is preferably performed. The surface treatment facilitates improvement in the hydrophilic property and ensure for adhesion between the image-recording layer and the support. Prior to the roughening treatment of the aluminum plate, a degreasing treatment, for example, with a surfactant, an organic solvent or an aqueous alkaline solution is conducted for removing rolling oil on the surface thereof, if desired.

The roughening treatment of the surface of the aluminum plate is conducted by various methods and includes, for example, mechanical roughening treatment, electrochemical roughening treatment (roughening treatment of electrochemically dissolving the surface) and chemical roughening treatment (roughening treatment of chemically dissolving the surface selectively).

As the method of the mechanical roughening treatment, a known method, for example, ball graining, brush graining, blast graining or buff graining can be used.

The electrochemical roughening treatment method includes, for example, a method of conducting by passing alternating current or direct current in an electrolyte containing an acid, for example, hydrochloric acid or nitric acid. Also, a method of using a mixed acid described in JP-A-54-63902 can be exemplified.

The aluminum plate subjected to the roughening treatment is subjected, if desired, to an alkali etching treatment using an aqueous solution, for example, of potassium hydroxide or sodium hydroxide and further subjected to a neutralizing treatment, and then subjected to an anodizing treatment for improving the abrasion resistance, if desired.

As the electrolyte used for the anodizing treatment of the aluminum plate, various electrolytes capable of forming porous oxide film can be used. Ordinarily, sulfuric acid, hydrochloric acid, oxalic acid, chromic acid or a mixed acid thereof is used. The concentration of the electrolyte can be appropriately determined depending on the kind of the electrolyte.

Since the conditions for the anodizing treatment are varied depending on the electrolyte used; they cannot be defined commonly. However, it is ordinarily preferred that electrolyte concentration in the solution is from 1 to 80% by weight, liquid temperature is from 5 to 70°C, current density is from 5 to 60 $A/dm^2$, voltage is from 1 to 100 V, and electrolysis time is from 10 seconds to 5 minutes. The amount of the anodized film formed is preferably from 1.0 to 5.0

$g/m^2$ and more preferably from 1.5 to 4.0 $g/m^2$. In the above-described range, good printing durability and good scratch resistance in the non-image area of lithographic printing plate can be achieved.

The aluminum plate subjected to the anodizing treatment is then subjected to a hydrophilizing treatment on the surface thereof, if desired. The hydrophilizing treatment includes an alkali metal silicate method described in U.S. Patents 2,714,066, 3,181,461, 3,280,734 and 3,902,734. In the method, the support is subjected to an immersion treatment or an electrolytic treatment in an aqueous solution, for example, of sodium silicate. In addition, the hydrophiliing treatment includes, for example, a method of treating with potassium fluorozirconate described in JP-B-36-22063 and a method of treating with polyvinylphosphonic acid described in U.S. Patents 3,276,868, 4,153,461 and 4,689,272.

The support preferably has a center line average roughness of 0.10 to 1.2 $\mu$m. In the above-described range, good adhesion to the image-recording layer, good printing durability, and good resistance to stain can be achieved.

Further, color density of the support is preferably from 0.15 to 0.65 in terms of a reflection density value. In the above-described range, good image-forming property due to prevention of halation at the image exposure and good plate inspection property after development can be achieved.

[Backcoat layer]

After applying the surface treatment or forming an undercoat layer to the support, a backcoat layer can be provided on the back surface of the support, if desired.

The backcoat layer preferably used includes, for example, a coating layer comprising an organic polymer compound described in JP-A-5-45885 and a coating layer comprising a metal oxide obtained by hydrolysis and polycondensation of an organic metal compound or an inorganic metal compound described in JP-A-6-35174. Among them, use of an alkoxy compound of silicon, for example, $Si(OCH_3)_4$, $Si(OC_2H_5)_4$. $Si(OC_3H_7)4$, or $Si(OC_4H_9)_4$ is preferred since the starting material is inexpensive and easily available.

[Undercoat layer]

In the lithographic printing plate precursor according to the invention, an undercoat layer can be provided between the support and the image-recording layer, if desired. The undercoat layer makes removal of the image-recording layer from the support in the unexposed area easy so that the on-press development property can be improved. Further, it is advantageous that in the case of infrared laser exposure, since the undercoat layer acts as a heat insulating layer, heat generated upon the exposure does note diffuse into the support and is efficiently utilized so that increase in sensitivity can be achieved.

As a compound for the undercoat layer (undercoat compound), specifically, for example, a silane coupling agent having an addition-polymerizable ethylenic double bond reactive group described in JP-A-10-282679 and a phosphorus compound having an ethylenic double bond reactive group described in JP-A-2-304441 are preferably exemplified.

As a most preferable undercoat layer, an embodiment containing a polymer resin obtained by copolymerization of a monomer having an adsorbing group, a monomer having a hydrophilic group and a monomer having a crosslinkable group is exemplified.

The essential component in the polymer resin for use in the undercoat layer is an adsorbing group to the hydrophilic surface of the support. Whether the adsorptivity to the hydrophilic surface of the support is present or not can be judged, for example, by the following method.

Specifically, a test compound is dissolved in a solvent in which the test compound is easily soluble to prepare a coating solution, and the coating solution is coated and dried on a support so as to have the coating amount after drying of 30 $mg/m^2$. After thoroughly washing the support coated with the test compound using the solvent in which the test compound is easily soluble, the residual amount of the test compound that has not been removed by the washing is measured to calculate the adsorption amount to the support. For measuring the residual amount, the amount of the residual test compound may be directly determined, or it may be calculated from the amount of the test compound dissolved in the washing solution. The determination for the compound can be performed, for example, by fluorescent X-ray measurement, reflection spectral absorbance measurement or liquid chromatography measurement. The compound having the adsorptivity to support means a compound that remains by 1 $mg/m^2$ or more even after conducting the washing treatment described above.

The adsorbing group to the hydrophilic surface of the support is a functional group capable of forming a chemical bond (for example, an ionic bond, a hydrogen bond, a coordinate bond or a bond with intermolecular force) with a substance (for example, metal or metal oxide) or a functional group (for example, a hydroxy group) present on the surface of the support. The adsorbing group is preferably an acid group or a cationic group.

The acid group preferably has an acid dissociation constant (pKa) of 7 or less. Examples of the acid group include a phenolic hydroxy group, a carboxyl group, $-SO_3H$, $-OSO_3H$, $-PO_3H_2$, $-OPO_3H_2$, $-CONHSO_2-$, $-SO_2NHSO_2-$ and $-COCH_2COCH_3$. Among them, $-OPO_3H_2$ and $-PO_3H_2$ are particularly preferred. The acid group may be the form of

a metal salt

The cationic group is preferably an onium group. Examples of the onium group include an ammonium group, a phosphonium group, an arsonium group, a stibonium group, an oxonium group, a sulfonium group, a selenonium group, a stannonium group and iodonium group. Among them, the ammonium group, phosphonium group and sulfonium group are preferred, the ammonium group and phosphonium group are more preferred, and the ammonium group is most preferred.

Particularly preferable examples of the monomer having the adsorbing group include compounds represented by the following formula (vi) or (vii):

(vi)

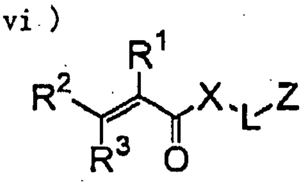

(vii)

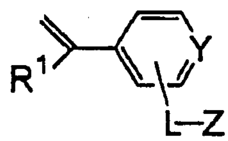

In formula (vi) or (vii), $R^1$, $R^2$ and $R^3$ each independently represents a hydrogen atom, halogen atom or an alkyl group having from 1 to 6 carbon atoms. $R^1$ and $R^2$ and $R^3$ each independently represents preferably a hydrogen atom or an alkyl group having from 1 to 6 carbon atoms, more preferably a hydrogen atom or an alkyl group having from 1 to 3 carbon atoms and, most preferably a hydrogen atom or a methyl group. It is particularly preferred that $R^2$ and $R^3$ each represents a hydrogen atom. Z represents a functional group adsorbing to the hydrophilic surface of the support.

In formula (vi), X represents an oxygen atom (-O-) or imino group (-NH-). Preferably, X represents an oxygen atom.

In formula (vi), L represents a divalent connecting group. It is preferred that L represents a divalent aliphatic group (for example, an alkylene group, a substituted alkylene group, an alkenylene group, a substituted alkenylene group, an alkinylene group or a substituted alkinylene group), a divalent aromatic group (for example, an arylene group or a substituted arylene group), a divalent heterocyclic group or a combination of each of the groups described above with an oxygen atom (-O-), a sulfur atom (-S-), an imino group (-NH-), a substituted imino group (-NR-, wherein R represents an aliphatic group, an aromatic group or a heterocyclic group) or a carbonyl group (-CO-).

The aliphatic group may form a cyclic structure or a branched structure. The number of carbon atoms of the aliphatic group is preferably from 1 to 20, more preferably from 1 to 15, and most preferably from 1 to 10. It is preferred that the aliphatic group is a saturated aliphatic group rather than an unsaturated aliphatic group. The aliphatic group may have a substituent. Examples of the substituent include a halogen atom, a hydroxy group, an aromatic group and a heterocyclic group.

The number of carbon atoms of the aromatic group is preferably from 6 to 20, more preferably from 6 to 15, and most preferably from 6 to 10. The aromatic group may have a substituent. Examples of the substituent include a halogen atom, a hydroxy group, an aliphatic group, an aromatic group and a heterocyclic group.

It is preferred that the heterocyclic group has a 5-membered or 6-membered ring as the heterocyclic ring. Other heterocyclic ring, an aliphatic ring or an aromatic ring may be condensed to the heterocyclic ring. The heterocyclic group may have a substituent. Example of the substituent include a halogen atom, a hydroxy group, an oxo group (=O), a thio group (-S), an imino group (=NH), a substituted imino group (=N-R, where R represents an aliphatic group, an aromatic group or a heterocyclic group), an aliphatic group, an aromatic group and a heterocyclic group.

It is preferred that L represents a divalent connecting group containing a plurality of polyoxyalkylene structures. It is more preferred that the polyoxyalkylene structure is a polyoxyethylene structure. Specifically, it is preferred that L contains $-(OCH_2CH_2)_n-$ (n is an integer of 2 or more).

In the formula (vii), Y represents a carbon atom or a nitrogen atom. In the case where Y is a nitrogen atom and L is connected to Y to form a quaternary pyridinium group, Z is not mandatory and may be a hydrogen atom, because the quaternary pyridinium group itself exhibits the adsorptivity. L represents a divalent connecting group same as that defined in formula (vi) or a single bond

The adsorptive functional group is the same as that described above.

Representative examples of the compound represented by formula (vi) or (vii) are set forth below.

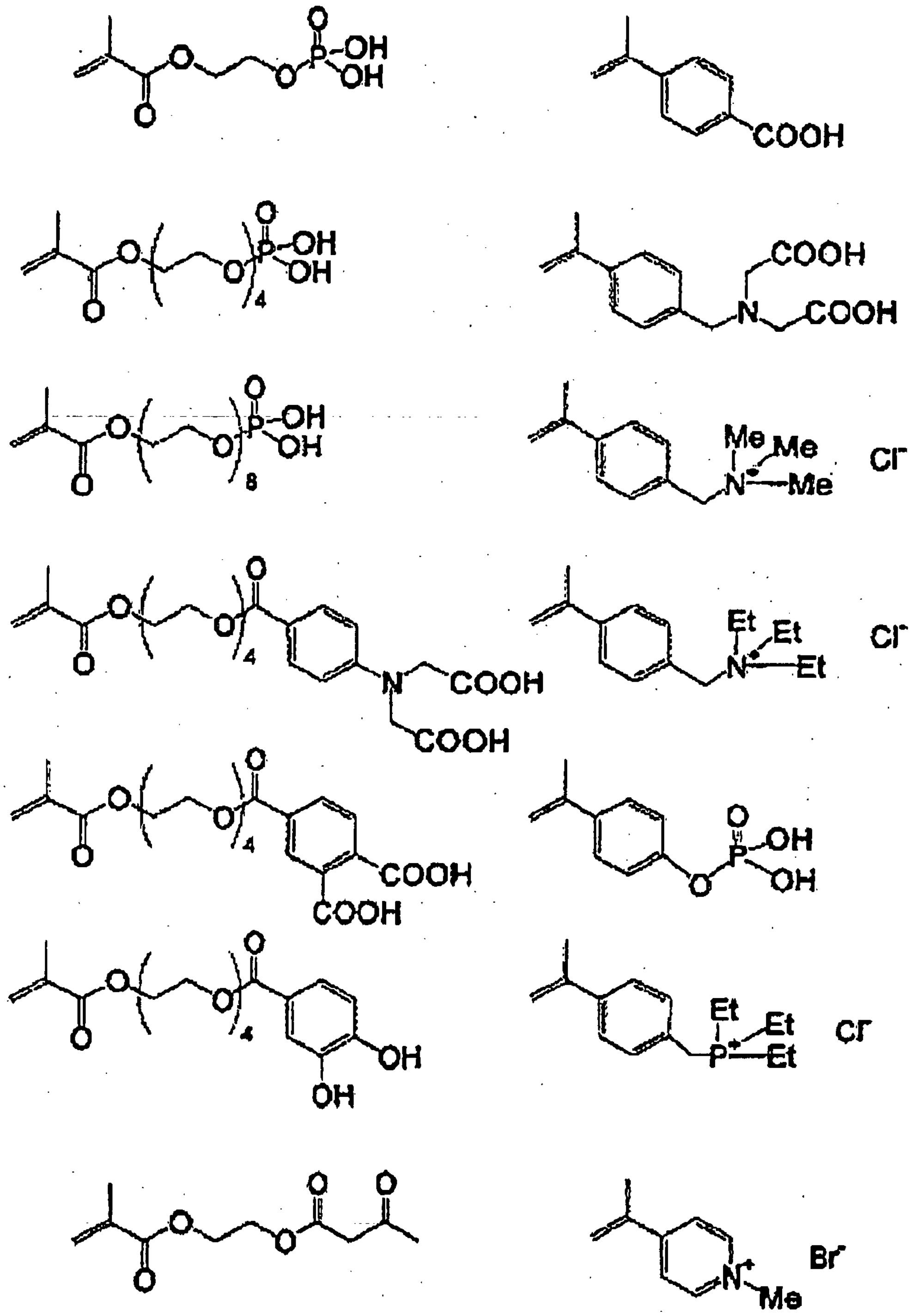

The hydrophilic group included in the polymer resin for the undercoat layer for use in the invention preferably includes, for example, a hydroxy group, a carboxyl group a carboxylate group, a hydroxyethyl group, a polyoxyethyl group, a hydroxypropyl group, a polyoxypropyl group, an amino group, an aminoethyl group, an aminopropyl group, an ammonium group, an amido group, a carboxymethyl group, a sulfo group or a phosphoric acid group. Among them, a monomer having a sulfo group exhibiting a highly hydrophilic property is preferable. Specific examples of the monomer having a sulfo group include sodium salt or an amine salt of methallyloxybenzenesulfonic acid, allyloxybenzenesulfonic acid, allylsulfonic acid, vinylsulfonic acid, p-styrenesulfonic acid, methallylsulfonic acid, acrylamide-tert-butylsulfonic acid, 2-acrylamido-2-methylpropanesulfonic acid and (3-acryloyloxypropyl)butylsulfonicacid. Among them, sodium salt of 2-acrylamido-2-methylpropanesulfonic acid is preferable in view of the hydrophilic property and handling property in the synthesis thereof

It is preferred that the polymer resin for the undercoat layer according to the invention has a crosslinking

property. A crosslinkable group acts to improve the adhesion to the image area. In order to impart the crosslinking property to the polymer resin for the undercoat layer, introduction of a crosslinkable functional group, for example, an ethylenically unsaturated bond into the side chain of the polymer or introduction by formation of a salt structure between a polar substituent of the polymer resin and a compound containing a substituent having a counter charge to the polar substituent of the polymer resin and an ethylenically unsaturated bond is used.

Examples of the polymer having an ethylenically unsaturated bond in the side chain thereof include a polymer of an ester or amide of acrylic acid or methacrylic acid, which is a polymer wherein the ester or amide residue (R in -COOR or -CONHR) has an ethylenically unsaturated bond.

Specific examples of the R, ester residue and amido residue are same as those of the R, ester residue and amido residue described for the binder polymer having a crosslinkable group of the image-recording layer above.

As a monomer having a crosslinkable group for the polymer resin for undercoat layer, an ester or amide of acrylic acid or methacrylic acid having the above-described crosslinkable group is preferred.

The content of the crosslinkable group in the polymer resin for undercoat layer (content of the radical polymerizable unsaturated double bond determined by iodine titration) is preferably from 0.1 to 10.0 mmol, more preferably from 1.0 to 7.0 mmol, and most preferably from 2.0 to 5.5 mmol, based on 1 g of the polymer resin. In the above-described range, preferable compatibility between the sensitivity and stain resistance and good preservation stability can be achieved.

The weight average molecular weight of the polymer resin for undercoat layer is preferably 5,000 or more, more preferably from 10,000 to 300,000. The number average molecular weight of the polymer resin is preferably 1,000 or more, more preferably from 2,000 to 250,000. The polydispersity (weight average molecular weight/number average molecular weight) thereof is preferably from 1.1 to 10.

The polymer resin for undercoat layer may be any of a random polymer, a block polymer, a graft polymer and the like, and is preferably a random polymer.

The polymer resins for undercoat layer may be used individually or in a mixture of two or more thereof Also, two or more of the compounds having a functional group adsorbing to the hydrophilic surface of the support may be used together. A coating solution for undercoat layer is obtained by dissolving the polymer resin for undercoat layer in an organic solvent (for example, methenol, ethanol, acetone or methyl ethyl ketone) and/or water. The coating solution for undercoat layer may contain an infrared absorbing agent

In order to coat the coating solution for undercoat layer on the support, various methods can be used. Examples of the method include bar coater coating, spin coating, spray coating, curtain coating, dip coating, air knife coating, blade coating and roll coating.

The coating amount (solid content) of the undercoat layer is preferably from 0.1 to 100 mg/m$^2$, and more preferably from 1 to 30 mg/m$^2$.

[Protective layer]

In the lithographic printing plate precursor according to the invention, a protective layer (overcoat layer) can be provided on the image-recording layer, if desired, for the purpose of imparting an oxygen blocking property, preventing occurrence of scratch or the like on the image-recording layer, preventing ablation caused by exposure with a high illuminance laser beam or the like.

Ordinarily, the exposure process of a lithographic printing plate precursor is performed in the air. The image-forming reaction occurred upon the exposure process in the image-recording layer may be inhibited by a low molecular compound, for example, oxygen or a basic substance present in the air. The protective layer prevents the low molecular compound, for example, oxygen or a basic substance from penetrating into the image-recording layer and as a result, the inhibition of image-forming reaction at the exposure process in the air can be avoided. Accordingly, the property required of the protective layer is to reduce permeability of the low molecular compound, for example, oxygen. Further, the protective layer preferably has good transparency to light used for the exposure, is excellent in adhesion to the image-recording layer, and can be easily removed during the on-press development processing step after the exposure. With respect to the protective layer having such properties, there are described, for example, in U.S. Patent 3,458,311 and JP-B-55-49729.

As a material for use in the protective layer, any water-soluble polymer and water-insoluble polymer can be appropriately selected to use. Specifically, a water-soluble polymer, for example, polyvinyl alcohol, modified polyvinyl alcohol, polyvinyl pyrrolidone, polyvinyl imidazole, polyacrylic acid, polyacrylamide, partially saponified product of polyvinyl acetate, ethylene-vinyl alcohol copolymer, water-soluble cellulose derivative, gelatin, starch derivative or gum arabic, and a polymer, for example, polyvinylidene chloride, poly(mth)acrylonitrile, polysulfone, polyvinyl chloride, polyethylene, polycarbonate, polystyrene, polyamide or cellophane are exemplified. The polymers may be used in combination of two or more thereof, if desired.

As a relatively useful material for use in the protective layer, a water-soluble polymer compound excellent in

crystallinity is exemplified. Specifically, polyvinyl alcohol, polyvinyl pyrrolidone, polyvinyl imidazole, a water-soluble acrylic resin, for example, polyacrylic acid, gelatin or gum arabic is preferably used. Above all, polyvinyl alcohol, polyvinyl pyrrolidone, and polyvinyl imidazole are more preferably used from the standpoint of capability of coating with water as a solvent and easiness of removal with dampening water at printing. Among them, polyvinyl alcohol (PVA) provides most preferable results on the fundamental properties, for example, oxygen blocking property or removability with development

The polyvinyl alcohol for use in the protective layer according to the invention may be partially substituted with ester, ether or acetal as long as it contains a substantial amount of unsubstituted vinyl alcohol units necessary for maintaining water solubility. Also, the polyvinyl alcohol may partially contain other copolymerization components. For instance, polyvinyl alcohols of various polymerization degrees having at random a various kind of hydrophilic modified cites, for example, an anion-modified cite modified with an anion, e.g., a carboxyl group or a sulfo group, a cation-modified cite modified with a cation, e.g., an amino group or an ammonium group, a silanol-modified cite or a thiol-modified cite, and polyvinyl alcohols of various polymerization degrees having at the terminal of the polymer having a various kind of modified cites, for example, the above-described anion-modified cite, cation modified cite, silanol-modified cite or thiol-modified cite, an alkoxy-modified cite, a sulfide-modified cite, an ester modified cite of vinyl alcohol with a various kind of organic acids, an ester modified cite of the above-described anion-modified cite with an alcohol or an epoxy-modified cite are also preferably used.

Preferable examples of the polyvinyl alcohol include those having a hydrolysis degree of 71 to 100% and a polymerization degree of 300 to 2,400. Specific examples of the polyvinyl alcohol include PVA-105, PVA-110, PVA-117, PVA-117K PVA-120, PVA-124, PVA-124H, PVA-CS, PVA-CST, PVA-HC, PVA-203, PVA-204, PVA-205, PVA-210, PVA-217, PVA-220, PVA-224, PVA-217EE, PVA-217E, PVA-220E, PVA-224E, PVA-405, PVA-420, PVA-613 and L-8 all produced by Kuraray Co., Ltd. Specific examples of the modified polyvinyl alcohol include that having an anion-modified cite, for example, KL-318, KL-118, KM-618, KM-118 or SK-5102, that having a cation-modified cite, for example, C-318, C-118 or CM-318, that having a terminal thiol-modified cite, for example, M-205 or M-115, that having a terminal sulfide-modified cite, for example, MP-103, MP-203, MP-102 or MP-202, that having an ester-modified cite with a higher fatty acid at the terminal, for example, HL-12E or HL-1203 and that having a reactive silane-modified cite, for example, R-1130, R-2105 or R-2130.

It is also preferable that the protective layer contains a stratiform compound. The stratiform compound is a particle having a thin tabular shape and includes, for instance, mica, for example, natural mica represented by the following formula: $A (B, C)_{2-5} D_4 O_{10} (OH, F, O)_2$, (wherein A represents any one of Li, K, Na, Ca, Mg and an organic cation, B and C each represents any one of Fe (II), Fe(III), Mn, Al, Mg and V, and D represents Si or Al) or synthetic mica; talc represented by the following formula: $3MgO{\cdot}4SiO{\cdot}H_2O$; teniolite; montmorillonite; saponite; hectolite; and zirconium phosphate.

Examples of the natural mica for use in the protective layer include muscovite, paragonite, phlogopite, biotite and lepidolite. Examples of the synthetic mica include non-swellable mica, for example, fluorphlogopite $KMg_3(AlSi_3O_{10})F_2$ or potassium tetrasilic mica $KMg_{2.5}(Si_4O_{10})F_2$, and swellable mica, for example, Na tetrasililic mica $NaMg_{2.5}(Si_0O_{10})F_2$, Na or Li teniolite $(Na, Li)Mg_2Li(Si_4O_{10})F_2$, or montmorillonite based Na or Li hectolite $(Na, Li)_{1/8}Mg2_{2/5}Li4_{1/8}(Si_4O_{10})F_2$. Synthetic smectite is also useful.

Of the stratiform compounds, fluorine-based swellable mica, which is a synthetic stratiform compound, is particularly useful in view of easy availability and uniformity. Specifically, the swellable synthetic mica and an swellable clay mineral, for example, montmorillonite, saponite, hectolite or bentonite have a stratiform structure comprising a unit crystal lattice layer having thickness of approximately 10 to 15 angstroms, and metallic atom substitution in the lattices thereof is remarkably large in comparison with other clay minerals. As a result, the lattice layer results in lack of positive charge and to compensate it, a cation, for example, $Li^+$, $Na^+$, $Ca^{2+}$, $Mg^{2+}$ or an organic cation, e.g., an amine salt, a quaternary ammonium salt, a phosphonium salt or a sulfonium salt is adsorbed between the lattice layers. The stratiform compound greatly swells upon contact with water. When share is applied under such condition, the stratiform crystal lattices are easily cleaved to form a stable sol in water. The bentnite and swellable synthetic mica have strongly such tendency.

With respect to the shape of the stratiform compound, the thinner the thickness or the larger the plain size as long as smoothness of coated surface and transmission of actinic radiation are not damaged, the better from the standpoint of control of diffusion. Therefore, an aspect ratio of the stratiform compound is ordinarily 20 or more, preferably 100 or more, and particularly preferably 200 or more. The aspect ratio is a ratio of thickness to major axis of particle and can be determined, for example, from a projection drawing of particle by a microphotography. The larger the aspect ratio, the greater the affect obtained.

As for the particle diameter of the stratiform compound, an average diameter is ordinarily from 1 to 20 $\mu m$, preferably from 1 to 10 $\mu m$, and particularly preferably from 2 to 5 $\mu m$. When the particle diameter is less than 1 $\mu m$, the inhibition of permeation of oxygen or moisture is insufficient and the effect of the stratiform compound can not be satisfactorily achieved. On the other hand, when it is larger than 20 $\mu m$, the dispersion stability of the particle in the coating solution is insufficient to cause a problem in that stable coating can not be performed. An average thickness of

the particle is ordinarily 0.1 μm or less, preferably 0.05 μm or less, and particularly preferably 0.01 μm or less. For example, with respect to the swellable synthetic mica that is the representative compound of the stratiform compounds, the thickness is approximately from 1 to 50 nm and the plain size is approximately from 1 to 20 μm.

When such a stratiform compound particle having a large aspect ratio is incorporated into the protective layer, strength of the coated layer increases and penetration of oxygen or moisture can be effectively inhibited so that the protective layer can be prevented from deterioration due to deformation, and even when the lithographic printing plate precursor is preserved for a long period of time under a high humidity condition, it is prevented from decrease in the image-forming property thereof due to the change of humidity and exhibits excellent preservation stability.

An amount of the stratiform compound contained in the protective layer is ordinarily from 5/1 to 1/100 in terms of a weight ratio of the stratiform compound to an amount of a binder used in the protective layer. When a plural kind of the stratiform compounds is used together, it is preferred that the total amount of the stratiform compounds is in the range of weight ratio described above.

As other composition for the protective layer, glycerol, dipropylene glycol or the like can be added in an amount corresponding to several % by weight of the binder to impart flexibility. Further, an anionic surfactant, for example, sodium alkyl sulfate or sodium alkyl sulfonate; an amphoteric surfactant, for example, alkylamino carboxylic acid salt or alkylamino dicarboxylic acid salt; or a non-ionic surfactant, for example, polyoxyethylene alkyl phenyl ether can be added. An amount of the surfactant added is from 0.1 to 100% by weight of the binder.

Further, for the purpose of improving the adhesion to the image-recording layer, for example, it is described in JP-A-49-70702 and BP-A-1303578 that sufficient adhesion can be obtained by mixing from 20 to 60% by weight of an acrylic emulsion, a water-insoluble vinyl pyrrolidone-vinyl acetate copolymer or the like in a hydrophilic polymer mainly comprising polyvinyl alcohol and coating the mixture on the image-recording layer. In the invention, any of such known techniques can be used.

Further, other functions can also be provided to be protective layer. For instance, by adding a coloring agent (for example, a water-soluble dye), which is excellent in permeability for infrared ray used for the exposure and capable of efficiently absorbing light at other wavelengths, a safe light adaptability can be improved without causing decrease in the sensitivity.

An example of common dispersing method for the stratiform compound used in the protective layer is described below. Specifically, from 5 to 10 parts by weight of a swellable stratiform compound that is exemplified as a preferable stratiform compound is added to 100 parts by weight of water to adapt the compound to water and to be swollen, followed by dispersing using a dispersing machine. The dispersing machine used include, for example, a variety of mills conducting dispersion by directly applying mechanical power, a high-speed agitation type dispersing machine providing a large shear force and a dispersion machine providing ultrasonic energy of high intensity. Specific examples thereof include a ball mill, a sand grinder mill, a visco mill, a colloid mill, a homogenizer, a dissolver, a polytron, a homomixer, a homoblender, a keddy mill, a jet agitor, a capillary type emulsifying device, a liquid siren, an electromagnetic strain type ultrasonic generator and an emulsifying device having Polman whistle. A dispersion containing from 5 to 10% by weight of the stratiform compound thus prepared is highly viscous or gelled and exhibits extremely good preservation stability. In the formation of a coating solution for protective layer using the dispersion, it is preferred that the dispersion is diluted with water, sufficiently stirred and then mixed with a binder solution.

To the coating solution for protective layer can, be added known additives, for example, an anionic surfactant, a nonionic surfactant, a cationic surfactant or a fluorine-based surfactant for improving coating property or a water-soluble plasticizer for improving physical property of the coated layer. Examples of the water-soluble plasticizer include propionamide, cyclohexanediol, glycerin or sorbitol. Also, a water-soluble (meth)acrylic polymer can be added. Further, to the coating solution may be added known additives for increasing adhesion to the image-recording layer or for improving time-lapse stability of the coating solution.

The coating solution for protective layer thus-prepared is coated on the image-recording layer provided on the support and then dried to form a protective layer. The coating solvent may be appropriately selected in view of the binder used, and when a water-soluble polymer is used, distilled water or purified water is preferably used as the solvent. A coating method of the protective layer is not particularly limited, and known methods, for example, methods described in U.S. Patent 3,458,311 and JP-B-55-49729 can be utilized. Specific examples of the coating method for the protective layer include a blade coating method, an air knife coating method, a gravure coating method, a roll coating method, a spray coating method, a dip coating method and a bar coating method.

A coating amount of the protective layer is preferably in a range from 0.01 to 10 $g/m^2$, more preferably in a range from 0.02 to 3 $g/m^2$, and most preferably in a range from 0.02 to 1 $g/m^2$ in terms of the coating amount after drying.

[Exposure]

As a light source for exposure of the lithographic printing plate precursor according to the invention, known light sources can be used without limitation. A preferred wavelength of the light source is from 300 to 1,200 nm. Specifically,

various kinds of lasers preferably used as the light source, and among them, a semiconductor laser emitting an infrared ray having a wavelength of 760 to 1,200 nm is preferably used.
The exposure mechanism used may be any of inner surface drum type, outer surface drum type and flat bed type can be used.
Further, other exposure light sources used for the lithographic printing plate precursor according to the invention include, for example, a super-high pressure, high pressure, medium pressure or low pressure mercury lamp, a chemical lamp, a carbon arc lamp, a xenon lamp, a metal halide lamp, a variety of visible or ultraviolet laser lamps, a fluorescent lamp, a tungsten lamp and sunlight.

[Printing method]

In the lithographic printing method according to the invention, the lithographic printing plate precursor of the invention is exposed imagewise by an infrared laser and then, without undergoing the development processing step, oily ink and an aqueous component are supplied to the lithographic printing plate precursor to perform printing.
More specifically, there are illustrated a method wherein the lithographic printing plate precursor is exposed by an infrared laser and without undergoing the development processing step, mounted on a printing machine to perform printing and a method wherein the lithographic printing plate precursor is mounted on a printing machine, exposed by an infrared laser on the printing machine to perform printing without undergoing the development processing step.
After the imagewise exposure of the lithographic printing plate precursor by an infrared laser, when an aqueous component and oily ink are supplied to perform printing without undergoing the development processing step, for example, a wet development processing step, in the exposed area of the image-recording layer, the image-recording layer cured by the exposure forms the oily ink receptive area having an oleophilic surface. On the other hand, in the unexposed area, the uncured image-recording layer is removed by dissolution or dispersion with the aqueous component and/or oily ink supplied to reveal a hydrophilic surface of support in the area.
As a result, the aqueous component is adhered on the revealed hydrophilic surface, the oily ink is adhered to the exposed area of the image-recording layer, and thus printing is initiated. While either the aqueous component or the oily ink may be supplied at first to the plate surface, it is preferred to supply the oily ink at first in view of preventing the aqueous component from contamination with the image-recording layer of the unexposed area. As the aqueous component and oily ink, dampening water and printing ink for conventional lithographic printing are used respectively.
Thus, the lithographic printing plate precursor is subjected to the on-press development on an offset printing machine and used as it is for printing a large number of sheets.

EXAMPLES

The present invention will be described in more detail with reference to the following examples, but the invention should not be construed as being limited thereto.

EXAMPLES 1 TO 11 AND COMPARATIVE EXAMPLE 1

[Preparation of lithographic printing plate precursor]

(1) Preparation of support

An aluminum plate (material: JIS 1050) having a thickness of 0.3 mm was subjected to a degreasing treatment at 50°C for 30 seconds using a 10% by weight aqueous sodium aluminate solution in order to remove rolling oil on the surface thereof and then grained the surface thereof using three nylon brushes embedded with bundles of nylon bristle having a diameter of 0.3 mm and an aqueous suspension (specific gravity: 1,1 g/cm$^3$) of pumice having a median size of 25 μm, followed by thorough washing with water. The plate was etched by immersing in a 25% by weight aqueous sodium hydroxide solution of 45°C for 9 seconds, washed with water, then immersed in a 20% by weight aqueous nitric acid solution at 60°C for 20 seconds, and washed with water. The etching amount of the grained surface was about 3 g/m$^2$.
Then, using an alternating current of 60 Hz, an electrochemical roughening treatment was continuously carried out on the plate. The electrolyte used was a 1% by weight aqueous nitric acid solution (containing 0.5% by weight of aluminum ion) and the electrolyte temperature was 50°C. The electrochemical roughening treatment was conducted using an alternating current source, which provides a rectangular alternating current having a trapezoidal waveform such that the time TP necessary for the current value to reach the peak from zero was 0.8 msec and the duty ratio was 1:1, and using a carbon electrode as a counter electrode. A ferrite was used as an auxiliary anode. The current density was 30 A/dm$^2$ in terms of the peak value of the electric current, and 5% of the electric current flowing from the electric source was divided to the auxiliary anode. The quantity of electricity in the nitric acid electrolysis was 175 C/dm$^2$ in terms

of the quantity of electricity when the aluminum plate functioned as an anode. The plate was then washed with water by spraying.

The plate was further subjected to an electrochemical roughening treatment in the same manner as in the nitric acid electrolysis above using as an electrolyte, a 0.5% by weight aqueous hydrochloric acid solution (containing 0.5% by weight of aluminum ion) having temperature of 50°C and under the condition that the quantity of electricity was 50 $C/dm^2$ in terms of the quantity of electricity when the aluminum plate functioned as an anode. The plate was then washed with water by spraying. The plate was subjected to an anodizing treatment using as an electrolyte, a 15% by weight aqueous sulfuric acid solution (containing 0.5% by weight of aluminum ion) at a current density of 15 $A/dm^2$ to form a direct current anodized film of 2.5 $g/m^2$, washed with water and dried. The center line average roughness (Ra) of the substrate was measured using a stylus having a diameter of 2 μm and it was found to be 0.51 μm. Moreover, Undercoat solution (1) shown below was coated on the substrate so as to have a dry coating amount of 6 $mg/m^2$ to prepare a support

Undercoat solution (1)

| | |
|---|---|
| Undercoat compound (1) shown below (weight average molecular weight: 60,000) | 0.017 g |
| Methanol | 9.00 g |
| Water | 1.00 g |

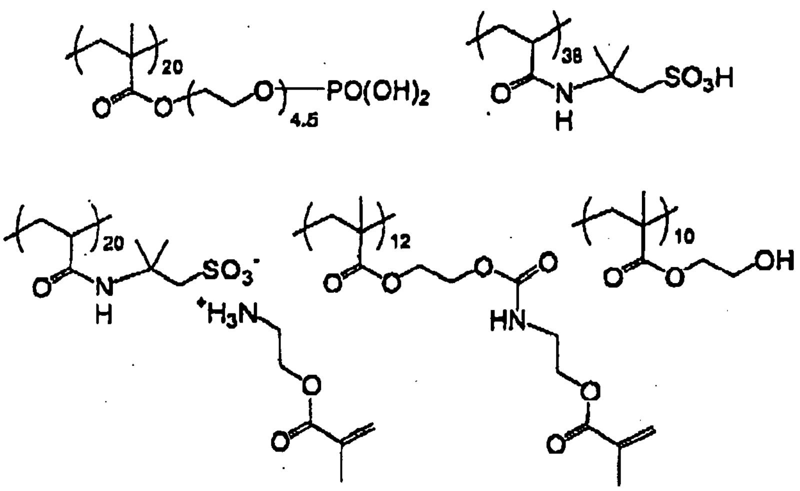

Undercoat compound (1)

(2) Formation of image-recording layer and protective layer

Coating solution for image-recording layer having the composition shown below was coated on the above-described support provided with the undercoat layer by a bar and dried in an oven at 100°C for 60 seconds to form an image-recording layer having a dry coating amount of 1.0 $g/m^2$. Subsequently, Coating solution for protective layer having the composition shown below was coated on the image-recording layer by a bar and dried in an oven at 120°C for 60 seconds to form a protective layer having a dry coating amount of 0.15 $g/m^2$, thereby preparing Lithographic printing plate precursors (1) to (11) and Lithographic printing plate precursor for comparison (R1), respectively.

The coating solution for image-recording layer was prepared by mixing each of Photosensitive solutions (1) to (11) and (R1) shown below with Microgel solution (1) shown below just before the coating, followed by stirring.

In Photosensitive solution (R1) used for Comparative Example 1, Comparative polymerization initiator (1) having a structure shown below was used in place of the specific compound according to the invention.

Photosensitive solutions (1) to (11) and (R1)

| | |
|---|---|
| Binder polymer (1) shown below (weight average molecular wight: 95,000) | 0.177 g |

(continued)

| | |
|---|---|
| Specific compound according to the invention or Comparative polymerization initiator (1) shown in Table 1 | X g |
| Sensitizing dye (Infrared absorbing agent shown in Table 1) | Y g |
| Polymerizable compound (Aronics M-215®, produced by Toagosei Co., Ltd.) | 0.319 g |
| Phosphonium compound (1) shown below | 0.035 g |
| Fluorine-based surfactant (1) shown below (weight average molecular weight: 35,000) | 0.004 g |
| Anionic surfactant (Pionine A-24-EA®, roduced by Takemoto Oil and Fat Co., Ltd., an aqueous 40% by weight solution) | 0.125 g |
| Methyl ethyl ketone | 2.554 g |
| 1-Methoxy-2-propanol | 7.023 g |
| Microgel solution (1) | 1.800 g |
| Water | 1.678 g |

Preparation of Microgel (1)

An oil phase component was prepared by dissolving 10.0 g of adduct of trimethylol propane and xylene diisocyanate (Takenaxe D-110N®, produced by Mitsui Takeda Chemical Co., Ltd., a 75% by weight ethyl acetate solution), 6.00 g of a polymerizable monomer (Aronics M-215®, produced by Toagosei Co., Ltd.) and 0.12 g of Pionine A-41C® (produced by Takemoto Oil and Fat Co., Ltd.) in 16.67 g of ethyl acetate. As an aqueous phase component, 37.5 g of a 4% by weight aqueous solution of PVA-205 was prepared. The oil phase component and the aqueous phase component were mixed and emulsified using a homogenizer at 12,000 rpm for 10 minutes. The resulting emulsion was added to 25 g of distilled water and stirred at room temperature for 30 minutes and then at 40°C for 2 hours. The thus-obtained liquid was diluted using distilled water so as to have the solid concentradon of 21% by weight to prepare Microgel (1). The average particle size of the particle in Microgel (1) was 0.23 μm.

Structures of the respective compounds used in the above-described photosensitive solutions are shown below.

Infrared absorbing agent (1):

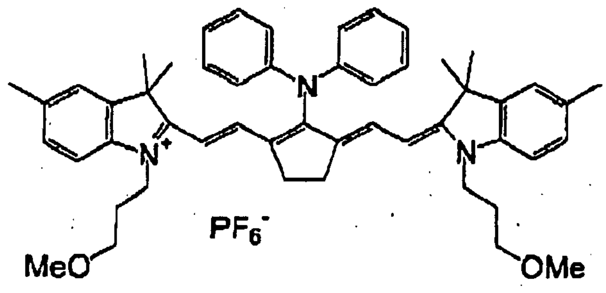

Infrared absorbing agent (2):

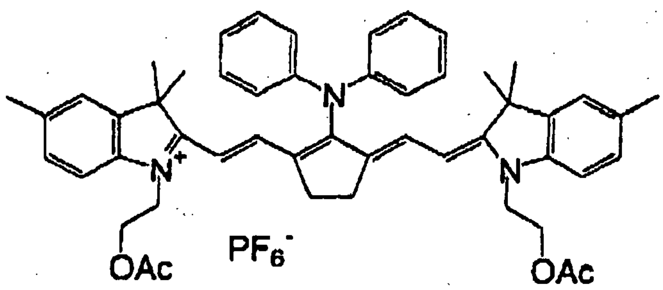

Infrared absorbing agent (3):

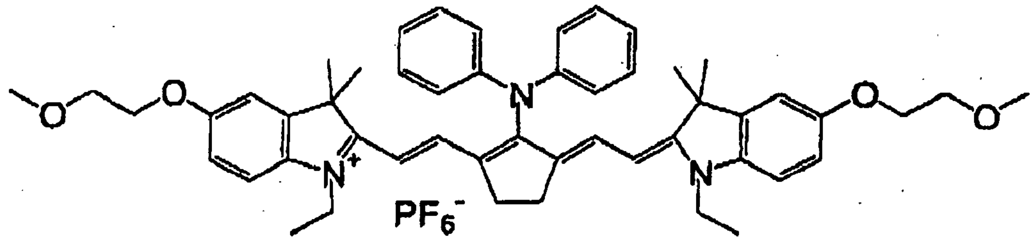

Infrared absorbing agent (4):

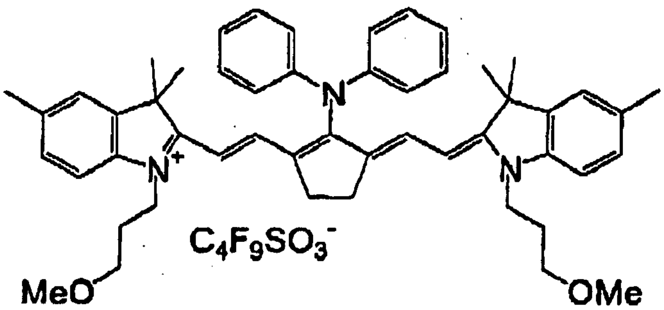

Binder polymer(1):

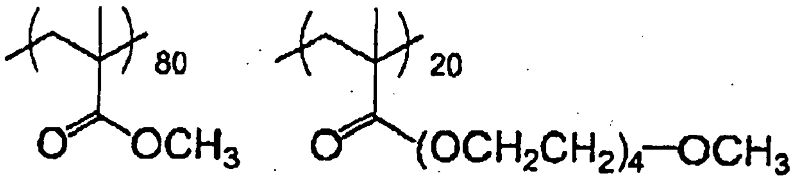

Fluorine-based surfactant (1);

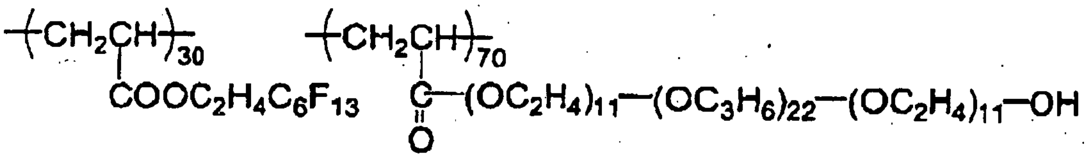

Phosphonium compound (I):

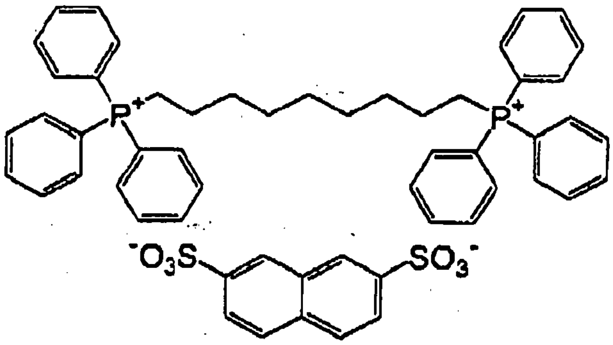

Comparative polymerization initiator (I):

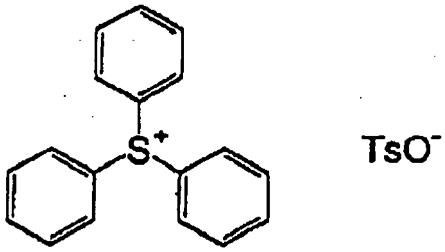

Coating solution for protective layer

| | |
|---|---|
| Dispersion of stratiform compound (1) shown below | 1.5 g |
| Polyvinyl alcohol (PVA-105, saponificafion degree: 98.5 % by mole, polymerization degree: 500, produced by Kuraray Co., Ltd.) | 0.06 g |
| Polyvinylpyrrolidone (K30, molecular weight Mw: 40,000, produced by Tokyo Chemical Industry Co., Ltd.) | 0.01 g |
| Copolymer of vinylpyrrolidone and vinyl acetate, (LUVITEC® VA64W, copolymerization ratio = 6/4, produced by ISP Co., Ltd.) | 0.01 g |
| Nonionic surfactant (EMALEX® 710, produced by Nihon-Emulsion Co., Ltd.) | 0.01 g |
| Ion-exchanged water | 6.0 g |

Preparation of Dispersion of stratiform compound (1)

To 193.6 g of ion-exchanged water was added 6.4 g of synthetic mica (Somasif® ME-100, produced by CO-OP Chemical Co., Ltd.) and the mixture was dispersed using a homogenizer until an average particle size (according to a laser scattering method) became 3 μm to prepare Dispersion of stratiform compound (1). The aspect ratio of the inorganic particle thus-dispersed was 100 or more.

[Evaluation of lithographic printing plate precursor]

Each of Lithographic printing plate precursors (1) to (11) and (R1) obtained was exposed by Trendsetter 3244VX (produced by Creo Co.) equipped with a water-cooled, 40 W infrared semiconductor laser under the conditions of output of 11.7W, a rotational number of an outer surface drum of 250 rpm and resolution of 2,400 dpi., and then the plate inspection property, on-press development property and printing durability were evaluated in the manner described below. The results obtained are shown in Table 1.

(Evaluation of plate inspection property)

The exposed lithographic printing plate precursor was allowed to stand for 30 minutes in a dark place having atmosphere of 25°C and 50% RH and then the plate inspection property was evaluated. Ease of plate inspection was

measured using an L value (luminance) of L*a*b* color system and difference (ΔL) between an L value of the exposed area and an L value of the unexposed area is determined. As the value of ΔL is large, the plate inspection property is more excellent. When the value of ΔL is 2.0 or more, it can be said that a plate inspection property with visual observation is excellent. The measurement was conducted according to SCE (specular competent exclude) system using a spectral colorimeter CM2600d and an operation soft CM-S100W produced by KONICA MINOLTA Inc. According to the SCE system, since the specular light is exclude and only the diffusion light is measured, evaluation of color close to evaluation with visual observation is conducted and the result well correlates with the practical human plate inspection.

(Evaluation of on-press development property)

The exposed lithographic printing plate precursor was mounted on a plate cylinder of a printing machine (SOR-M, produced by Heidelberg Co.) without conducting development processing. After supplying dampening water (EU-3 (etching solution, produced by Fuji Photo Film Co., Ltd.)/water/isopropyl alcohol = 1/89/10 (volume ratio)) and ink (TRANS-G (N) black ink, produced by Dainippon Ink and Chemicals, Inc.), printing was conducted at a printing speed of 6,000 sheets per hour.

A number of printing papers (on-press development property) required until reaching a state where the ink was not transferred in the unexposed area (non-image area) of the image-recording layer was determined. As the number of printing papers is small, it is evaluated that the on-press development property is more excellent.

(Evaluation of printing durability)

The printing was further continued and as increase in a number of printing papers, the image-recording layer was gradually abraded to cause decrease in the ink receptivity, resulting in decrease of ink density on printing paper. A number of printing papers obtained until the ink density (reflection density) decreased by 0.1 from that at the initiation of printing was determined to evaluate the printing durability. As the number of the printing papers is large, it is evaluated that the printing durability is more excellent

TABLE 1: Examples 1-11 and Comparative Example 1

| | Lithographic Printing Plate Precursor | Photosensitive Solution | Polymerization Initiator | | Infrared Absorbing Agent | | Result of Evaluation | | |
|---|---|---|---|---|---|---|---|---|---|
| | | | Structure | Amount added X(g) | Structure | Amount added Y(g) | Plate Inspection Property ΔL | On-press Development Property (index) | Printing Durability (index) |
| Example 1 | (15) | (15) | Aa-I | 0.1417 | (1) | 0.0308 | 4.0 | 50 | 214 |
| Example 2 | (16) | (16) | Aa-14 | 0.1371 | (1) | 0.0308 | 4.1 | 50 | 177 |
| Example 3 | (17) | (17) | Aa-I | 0.1504 | (1) | 0.0308 | 2.2 | 50 | 200 |
| Example 4 | (18) | (18) | Aa-6 | 0.1926 | (1) | 0.0308 | 3.0 | 50 | 214 |
| Example 5 | (19) | (19) | Aa-7 | 0.1225 | (1) | 0.0308 | 3.2 | 50 | 200 |
| Example 6 | (20) | (20) | Aa-19 | 0.1120 | (1) | 0.0308 | 4.0 | 50 | 171 |
| Example 7 | (21) | (21) | Aa-17 | 0.1293 | (1) | 0.0308 | 4.0 | 50 | 171 |
| Example 8 | (22) | (22) | Aa-20 | 0.1418 | (1) | 0.0308 | 4.0 | 50 | 200 |
| Example 9 | (32) | (32) | Aa-28 | 0.1357 | (1) | 0.0361 | 4.2 | 50 | 143 |
| Example 10 | (24) | (24) | Aa-2 | 0.1417 | (2) | 0.0319 | 4.0 | 60 | 214 |
| Example 11 | (25) | (25) | Aa-2 | 0.1411 | (3) | 0.0320 | 4.1 | 70 | 211 |
| Comparative Example 1 | (R1) | (R1) | (1) | 0.144 | (1) | 0.0308 | 0.2 | 100 | 100 |

(With respect to the on-press development property and printing durability, the number of sheets in Comparative Example 1 was used as the standard with an index of 100)

From the results shown in Table 2, it is confirmed that the lithographic printing plate precursors using the specific compound including a polymerizable group according to the invention exhibit good contrast between the exposed area and the unexposed area by the laser exposure to be provided with the excellent plate inspection property (visibility). It is also confirmed that each of the lithographic printing plate precursors is extremely excellent in the on-press development property and the printing durability.

[Synthesis example of the compound represented by formula (Ia)]

Synthesis Example 1: Synthesis of Compounds (Aa-1) and (Aa-2)

In 9.67 g of ethyl acetate was dissolve 5.0 g of 4-phenylpyridine and while keeping at 40°C, to the solution was added 7.4 g of sodium perborate tetrahydrate over a period of 30 minutes. The mixture was stirred at 40°C for 9 hours and allowed to cool to room temperature. To the mixture were added 60 ml of a 17% by weight aqueous sodium thiosulfate solution and a 17% by weight aqueous potassium carbonate solution in this order and the crystals thus-deposited were removed by filtration. To the filtrate was added a 20% by weight aqueous sodium chloride solution, followed by stirring at room temperature for 30 minutes and the crystals thus-deposited were collected by filtration and dried to obtain 3.5 g (yield: 63%) of 4-phenylpyridine-N-oxide. Separately, 13.0 g of 2-hydroxyethyl methacrylate and 20.0 g of p-toluenesulfonyl chloride were stirred in 11.8 g of pyridine for 3 hours while keeping the reaction temperature from 0 to 10°C and then the reaction mixture was extracted with ethyl acetate to obtain 26.0 g (yield: 92%) of 2-(toluene-4-sulfonyloxy)ethyl methacrylate. A mixture of 6.39 g of 2-(toluene-4-sulfonyloxy)ethyl methacrylate and 3.5 g of 4-phenylpyridine-N-oxide was stirred at 80°C for 6 hours and then allowed to cool to room temperature. To the mixture was added 25 ml of ethyl acetate, followed by stirring at room temperature for 30 minutes and the crystals thus-deposited were collected by filtration to obtain 7.2 g (yield: 77%) of Aazinium salt (Aa-1). In 20 ml of methanol was dissolved 7.2 g of Azinium salt (Aa-1) obtained and the resulting solution was dropwise added to an aqueous solution containing 15.0 of $KPF_6$ and 200 ml of water at room temperature, After the completion of the dropwise addition, the mixture was stirred for one hour. Then, the crystals thus-deposited were collected by nitration and dried to obtain 6.6 g (yield: 97%) of Azinium salt (Aa-2). The synthesis scheme is shown below. Azinium salt (Aa-1) and Azinium salt (Aa-2) were identified by $^1$H-NMR (solvent: DMSO).
Compound (Aa-1)
$^1$H-NMR (400 MHz, DMSO-d6): 8 9.53 (d, J=7.2 Hz, 2H), 8.61 (d, J=7.2 Hz. 2H). 8.08 (d, J=7.2 Hz, 2H), 7.67 (m, 3H), 7.47 (d, J=8.0 Hz, 2H), 7.10 (d, J=8.0 Hz, 2H), 5.96 (d, J=1,0 Hz, 1H). 5.69 (d, J=1.0 Hz, 1H), 5.00 (m, 2H), 4.58 (m, 2H), 2.28 (s. 3H), 1.83 (s, 3H).
Compound (Aa-2)
$^1$H-NMR (400 MHz, DMSO-d6): δ 9.53 (d, J=7.2 Hz, 2H), 8.61 (d, J=7.2 Hz, 2H), 8.08 (d, J=72 Hz, 2H), 7.67 (m, 3H), 5.96 (d, J=1.0 Hz, 1H, 5.69 (d, J=1.0 Hz, 1H), 5.00 (m, 2H), 4.58 (m, 2H), 1.83 (s, 3H),

[Reaction Formula]

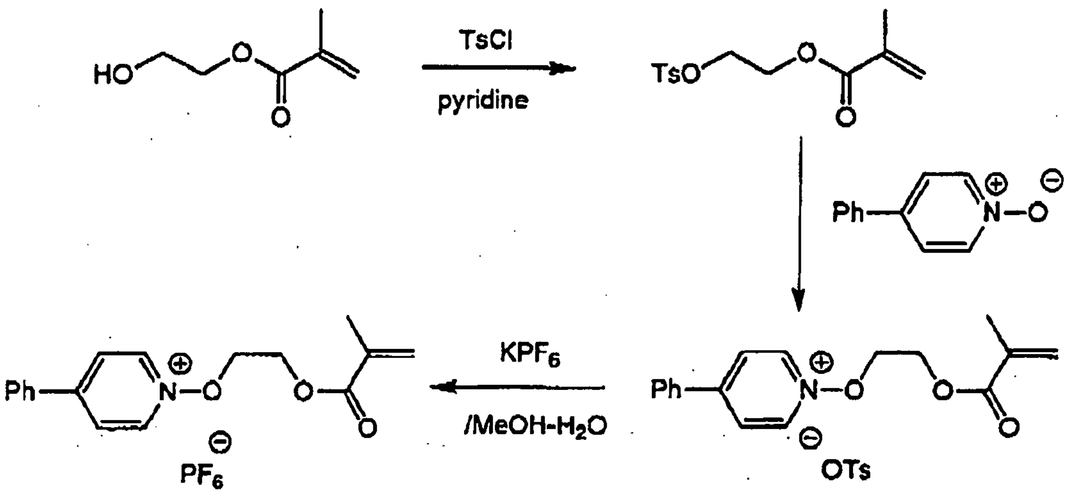


(Aa-2) (Aa-1)

Synthesis Example 2: Synthesis of Compound (Aa-14)

In 11.8 g of pyridine were stilled 11.6 g of 2-hydroxyethyl acrylate and 20.0 g of p-toluenesulfonyl chloride for 3 hours while keeping the reaction temperature from 0 to 10°C and then the reaction mixture was extracted with ethyl acetate to obtain 23.7 g (yield: 88%) of 2-(toluene-4-sulfonyloxy)ethyl acrylate. A mixture of 2.9 g of 2-(toluene-4-sulfonyloxy)ethyl acrylate and 1.5 g of 4-phenylpyridine-N-oxide was stirred at 60°C for 6 hours and then allowed to cool to room temperature. It was dissolved in 5 ml of acetone and the resulting solution was dropwise added to an aqueous solution containing 5.0 of $KPF_6$ and 100 ml of water at room temperature. After the completion of the dropwise addition, the mixture was stirred for 30 minutes. Then, the crystals thus-deposited were collected by filtration and dried to obtain 2.4 g (yield: 64%) of Azinium salt (Aa-14). Azinium salt (Aa-14) was identified by $^1$H-NMR (solvent: DMSO).
Compound (Aa-14)
$^1$H-NMR (400 MHz, DMSO-d6): $\delta$ 9.52 (d, J=7.2 Hz, 2H), 8.61 (d, J=7.2 Hz, 2H), 8.09 (d, J=72 Hz, 2H), 7.67 (m, 3H), 6.33 (dd, J=17.2 1.0 Hz, 1H), 6.16 (dd, J=17.2 10.4 Hz, 1H), 5.99 (dd. J=10.4, 1.0 Hz, 1H), 4.99 (m, 2H), 4.58 (m. 2H)..

Synthesis Example 3: Synthesis of Compound (Aa-20)

A mixture of 2.7 g of ethyl 2-(bromomethyl) acrylate and 2.0 g of 4-phenylpyridine-N-oxide was stirred at 60°C for 4 hours. After being allowed to cool to room temperature, it was dissolved in 15 ml of 2-propanol and the resulting solution was dropwise added with stirring to an aqueous solution containing 6.6 of potassium hexafluorophsphate and 100 ml of water at room temperature. The crystals thus-deposited were collected by filtration and dried to obtain 1.5 g (yield: 29%) of Azinium salt (Aa-20). Azinium salt (Aa-20) and was identified by $^1$H NMR (solvent: DMSO).
Compound (Aa-20)
$^1$H-NMR (400 MHz, DMSO-d6): $\delta$ 9.40 (d, J=7.2 Hz, 2H), 8.61 (d, J=7.2 Hz, 2H), 8.10 (d, J=7.2 Hz, 2H), 7.67 (m, 3H), 6.56 (s, 1H), 6.28 (s, 1H), 5.41 (s, 2H), 4.23 (q, J=7.2 Hz, 2H), 1.27 (t, J=7.2 Hz, 3H).

## Claims

1. A lithographic printing plate precursor comprising an image-recording layer which is capable of being removed with at least one of printing ink and dampening water and contains a compound of formula (Ia), a sensitizing dye and a compound having at least one addition-polymerizable ethylenically unsaturated bond:

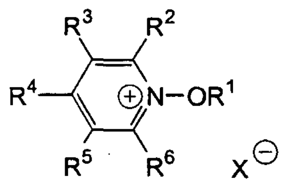

(Ia)

wherein

$R^1$ is a monovalent substituent,
$R^2$-$R^6$ each independently are H, halogen or a monovalent substituent, and
$X^{\theta}$ is an inorganic anion or a counter anion of a strong acid;

and at least one of $R^1$-$R^6$ is a polymerizable group selected from the formulae (2)-(4):,

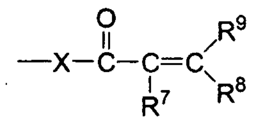

(2)

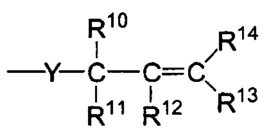

(3)

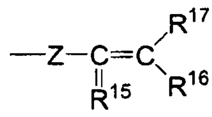

(4)

Wherein X, Y, and Z each are a single bond or an organic connecting group, and $R^7$-$R^{17}$ each independently are H or a monovalent substituent.

2. The lithographic printing plate precursor of claim 1, wherein the sensitizing dye is an IR-absorbing agent.

3. The lithographic printing plate precursor of claim 1 or 2, wherein in the compound of formula (Ia) at least one of combinations of $R^1$ and $R^2$, $R^2$ and $R^3$, $R^3$ and $R^4$, $R^4$ and $R^5$, $R^5$ and $R^6$, and $R^6$ and $R^1$ are combined with each other to form an aromatic ring or heterocyclic ring, so as to form a condensed ring as a whole.

4. The lithographic printing plate precursor of claim 1 or 2, wherein in the compound of formula (Ia) at least one of combinations of $R^2$ and $R^3$, $R^3$ and $R^4$, $R^2$ and $R^3$ and $R^4$ and $R^5$, and $R^2$ and $R^3$ and $R^5$ and $R^6$ are combined with each other to form an aromatic ring or heterocyclic ring including a substituent having 3-20 carbon atoms.

5. The lithographic printing plate precursor of claim 1 or 2, wherein in the compound of formula (Ia) $R^1$ is a straight chain, branched or cyclic $C_{1-20}$-alkyl group having as a substituent at least one of a benzene rink, a condensed ring formed from two or three benzene rings and a condensed ring formed from a benzene ring and a 5-membered unsaturated ring.

6. The lithographic printing plate precursor of claim 1 or 2, wherein the compound of formula (Ia) is a compound of formula (IIa), which contains in its molecule two or more skeletons of formula (Ia) connecting through $R^1$ in Formula (Ia):

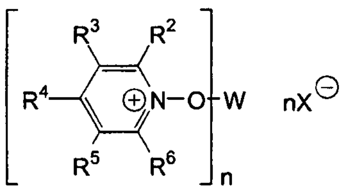

wherein

$R^2$-$R^6$ and $X^\theta$ are as defined in claim 1, and at least one of $R^2$-$R^6$ is a polymerizable group as defined in claim 1,
W is an n-valent organic connecting group, and
N is an integer of 2 or more.

7. The lithographic printing plate precursor of claim 6, wherein W in the formula (IIa) contains 1-5 oxygen atoms.

8. The lithographic printing plate precursor of claim 7, wherein W contains 1 oxygen atom.

9. The lithographic printing plate precursor of any of claims 2-8, wherein the IR-absorbing agent is a compound including a solvent-soluble group in its molecule.

10. The lithographic printing plate precursor of claim 9, wherein the solvent-soluble group is selected from alkyloxy, aryloxy, alkyloxycarbonyl and aryloxycarbonyl.

11. The lithographic printing plate precursor of any of claims 2-10, wherein the IR-absorbing agent has a salt structure comprising a cation and an anion, and at least one of the anion of the compound represented by formula (Ia) and the anion of the IR-absorbing agent is an inorganic anion.

12. The lithographic printing plate precursor of any of the preceding claims, wherein the image-recording layer further contains a binder polymer.

13. The lithographic printing plate precursor of any of the preceding claims, wherein the image-recording layer further contains a microcapsule or a microgel.

14. The lithographic printing plate precursor of any of the preceding claims, which comprises a support and an image-recording layer capable of being image-recorded with infrared laser exposure, and which is capable of performing printing by

- conducting image-recording on the precursor with an IR-laser and mounting the image-recorded precursor on

a printing machine without carrying out a development processing; or
- mounting the precursor on a printing machine and conducting image-recording on the mounted precursor with an IR-laser.

**15.** A lithographic printing method comprising the steps of:

- imagewise exposing the lithographic printing plate precursor of any of claims 2-13 with an IR-laser; and
- supplying oily ink and an aqueous component to the exposed precursor to perform printing without carrying out a development processing, wherein the area of the precursor unexposed to the IR-laser is removed during the printing step.

**Patentansprüche**

**1.** Lithografiedruckplattenvorläufer, umfassend eine Bildaufzeichnungsschicht, die mit zumindest einem von Drucktinte und Anfeuchtwasser entfernt werden kann und die eine Verbindung der Formel (Ia), einen Sensibilisierungsfarbstoff und eine Verbindung mit mindestens einer Additions-polymerisierbaren, ethylenisch ungesättigten Bindung enthält:

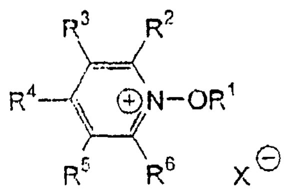

(Ia)

worin

$R^1$ ein monovalenter Subs'tituent ist,
$R^2$ bis $R^6$ jeweils unabhängig H, Halogen oder ein monovalenter Substituent sind, und
$X^{\theta}$ ein anorganisches Anion oder eine Gegenanion einer starken Säure ist;

und mindestens eins von $R^1$ bis $R^6$ eine polymerisierbare Gruppe ist, die ausgewählt ist aus den Formeln (2) bis (4) :

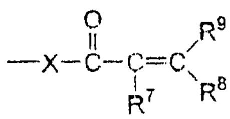

(2)

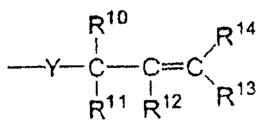

(3)

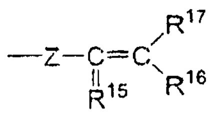

(4)

worin X, Y und Z jeweils eine Einfachbindung oder eine organische Verknüpfungsgruppe sind und $R^7$ bis $R^{17}$ jeweils unabhängig H oder en monovalenter Substituent sind.

**2.** Lithografiedruckplattenvorläufer gemäß Anspruch 1, worin der Sensibilisierungsfarbstoff ein IR-absorbierendes Mittel ist.

**3.** Lithografiedruckplattenvorläufer gemäß Anspruch 1 oder 2, worin in der Verbindung der Formel (Ia) mindestens eine der Kombinationen von $R^1$ und $R^2$, $R^2$ und $R^3$, $R^3$ und $R^4$, $R^4$ und $R^5$, $R^5$ und $R^6$ und $R^6$ und $R^1$ miteinander verbunden sind, um einen aromatischen Ring oder heterocyclischen Ring zu bilden, so dass insgesamt ein kondensierter Ring gebildet wird.

**4.** Lithografiedruckplattenvorläufer gemäß Anspruch 1 oder 2, worin in der Verbindung der Formel (Ia) mindestens eine der Kombinationen von $R^2$ und $R^3$, $R^3$ und $R^4$, $R^2$ und $R^3$ und $R^4$ und $R^5$, und $R^2$ und $R^3$ und $R^5$ und $R^6$ miteinander verbunden sind, um einen aromatischen Ring oder heterocyclischen Ring einschließlich eines Substituenten mit 3 bis 20 Kohlenstoffatomen zu bilden.

**5.** Lithografiedruckplattenvorläufer gemäß Anspruch 1 oder 2, worin in der Verbindung der Formel (Ia) $R^1$ eine gerad-

kettige, verzweigte oder cyclische $C_{1-20}$-Alkylgruppe ist, die als einen Substituenten mindestens eines von einem Benzolring, einem kondensierten Ring, der aus zwei oder drei Benzolringen gebildet ist, und einem kondensierten Ring, der aus einem Benzolring und einem 5-gliedrigen ungesättigten Ring gebildet ist, aufweist.

**6.** Lithografiedruckplattenvorläufer gemäß Anspruch 1 oder 2, worin die Verbindung der Formel (Ia) eine Verbindung der Formel (IIa) ist, die in ihrem Molekül zwei oder mehr Gerüste der Formel (Ia) aufweist, die durch $R^1$ in der Formel (Ia) verbunden sind:

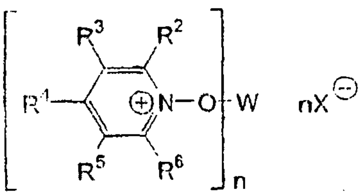

worin

$R^2$ und $R^6$ und $X^\theta$ wie in Anspruch 1 definiert sind und mindestens eines von $R^2$ bis $R^6$ eine polymerisierbare Gruppe, wie in Anspruch 1 definiert, ist,
W eine n-valente organische Verknüpfungsgruppe ist und
n eine ganze Zahl von 2 oder größer ist.

**7.** Lithografiedruckplattenvorläufer gemäß Anspruch 6, worin Wein der Formel (IIa) 1 bis 5 Sauerstoffatome enthält.

**8.** Lithografiedruckplattenvorläufer gemäß Anspruch 7, worin W ein Sauerstoffatom enthält.

**9.** Lithografiedruckplattenvorläufer gemäß irgendeinem der Ansprüche 2 bis 8, worin das IR-absorbierende Mittel eine Verbindung ist, die in ihrem Molekül eine Lösungsmittel-lösliche Gruppe enthält.

**10.** Lithografiedruckplattenvorläufer gemäß Anspruch 9, worin die Lösungsmittel-lösliche Gruppe ausgewählt ist aus Alkyloxy, Aryloxy, Alkyloxycarbonyl und Aryloxycarbonyl.

**11.** Lithografiedruckplattenvorläufer gemäß irgendeinem der Ansprüche 2 bis 10, worin das IR-absorbierende Mittel eine Salzstruktur aufweist, die ein Kation und ein Anion umfasst, und mindestens eines von dem Anion der durch die Formel (Ia) dargestellten Verbindung und dem Anion des IR-absorbierenden Mittels ein anorganisches Anion ist.

**12.** Lithografiedruckplattenvorläufer gemäß irgendeinem der vorhergehenden Ansprüche, worin die Bildaufzeichnungsschicht ferner ein Bindepolymer enthält.

**13.** Lithografiedruckplattenvorläufer gemäß irgendeinem der vorhergehenden Ansprüche, worin die Bildaufzeichnungsschicht ferner eine Mikrokapsel oder ein Mikrogel enthält.

**14.** Lithografiedruckplattenvorläufer gemäß irgendeinem der vorhergehenden Ansprüche, die einen Träger und eine Bildaufzeichnungsschicht umfasst, die in der Lage ist, durch Infrarotlaserbelichtung bildaufgezeichnet zu werden und welcher in der Lage ist, das Drucken durchzuführen durch das

- Durchführen einer Bildaufzeichnung auf dem Vorläufer mit einem IR-Laser und Montieren des bildaufgezeichneten Vorläufers auf einer Druckmaschine, ohne eine Entwicklungsverarbeitung durchzuführen; oder
- Montieren des Vorläufers auf einer Druckmaschine und Durchführen der Bildaufzeichnung auf dem montierten Vorläufer mit einem IR-Laser.

**15.** Lithografiedruckverfahren, umfassend die Schritte:

- bildweises Belichten des Lithografiedruckplattenvorläufers gemäß irgendeinem der Ansprüche 2 bis 13 mit einem IR-Laser; und
- Zuführen von öliger Tinte und einer wässrigen Komponente zu dem belichteten Vorläufer, um das Drucken durchzuführen, ohne eine Entwicklungsverarbeitung durchzuführen, worin die Fläche des mit dem IR-Laser

nicht belichteten Bereichs des Vorläufers während des Druckschritts entfernt wird.

## Revendications

1. Un précurseur de plaque d'impression lithographique comprenant une couche d'enregistrement d'image qui est apte à être enlevée avec au moins l'un d'une encre d'impression et d'une eau de mouillage et contient un composé de formule (Ia), un colorant sensibilisateur et un composé ayant au moins une liaison éthyléniquement insaturée polymérisable par addition:

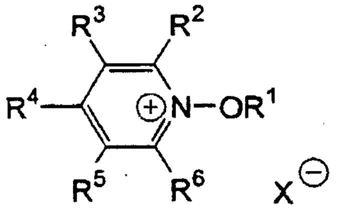

(Ia)

dans laquelle

$R^1$ est un substituant monovalent,
$R^2$-$R^6$ sont chacun indépendamment H, un halogène ou un substituant monovalent, et
$X^\theta$ est un anion inorganique ou un contre-anion d'un acide fort;

et au moins l'un de $R^1$-$R^6$ est un groupe polymérisable choisi parmi les formules (2)-(4):

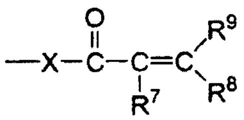

(2)

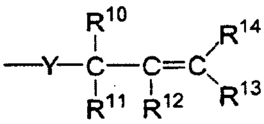

(3)

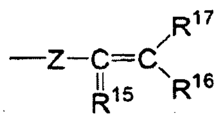

(4)

Dans lesquelles X, Y, et Z sont chacun une liaison simple ou un groupe de liaison organique, et $R^7$-$R^{17}$ sont chacun indépendamment H ou un substituant monovalent.

2. Le précurseur de plaque d'impression lithographique de la revendication 1, dans lequel le colorant sensibilisateur est un agent absorbant le rayonnement IR.

3. Le précurseur de plaque lithographique de la revendication 1 ou 2, dans lequel dans le composé de formule (Ia) au moins l'une des combinaisons de $R^1$ et $R^2$, $R^2$ et $R^3$, $R^3$ et $R^4$, $R^4$ et $R^5$, $R^5$ et $R^6$, et $R^6$ et $R^1$ sont combinées les unes avec les autres pour former un cycle aromatique ou un cycle hétérocyclique, de façon à former un cycle condensé dans son ensemble.

4. Le précurseur de plaque d'impression lithographique de la revendication 1 ou 2, dans lequel dans le composé de formule (Ia) au moins l'une des combinaisons de $R^2$ et $R^3$, $R^3$ et $R^4$, $R^2$ et $R^3$ et $R^4$ et $R^5$, et $R^2$ et $R^3$ et $R^5$ et $R^6$ sont combinées les unes avec les autres pour former un cycle aromatique ou un cycle hétérocyclique incluant un substituant ayant de 3 à 20 atomes de carbone.

5. Le précurseur de plaque d'impression lithographique de le revendication 1 ou 2, dans lequel dans le composé de formule (Ia) $R^1$ est un groupe alkyle en $C_1$-$C_{20}$ à chaîne droite, ramifiée ou cyclique ayant comme substituant au moins l'un d'un cycle benzénique, un cycle condensé formé de deux ou trois cycles benzéniques et un cycle condensé formé par un cycle benzénique et un cycle insaturé à 5 chaînons.

6. Le précurseur de plaque d'impression lithographique de la revendication 1 ou 2, dans lequel le composé de formule (Ia) est un composé de formule (IIa), qui contient dans sa molécule deux ou plus squelettes de formule (Ia) se

connectant par l'entremise de $R^1$ dans la formule (Ia):

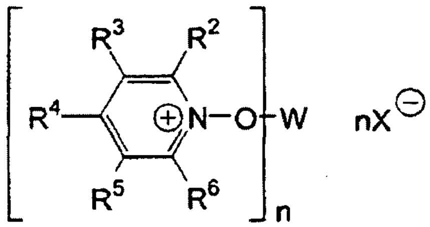

dans laquelle

$R^2$-$R^6$ et $X^\theta$ sont tels que définis dans la revendication 1, et au moins l'un de $R^2$-$R^6$ est un groupe polymérisable tel que défini dans la revendication 1,
W est un groupe organique de connexion n-valent, et
N est un entier de 2 ou plus.

**7.** Le précurseur de plaque d'impression lithographique de la revendication 6, dans lequel W dans la formule (IIa) contient de 1 à 5 atomes d'oxygène.

**8.** Le précurseur de plaque d'impression lithographique de la revendication 7, dans lequel W contient 1 atome d'oxygène.

**9.** Le précurseur de plaque d'impression lithographique de l'une quelconque des revendications 2-8, dans lequel l'agent absorbant le rayonnement IR est un composé incluant un groupe soluble dans un solvant dans sa molécule.

**10.** Le précurseur de plaque d'impression lithographique de la revendication 9, dans lequel le groupe soluble dans un solvant est choisi parmi alkyloxy, aryloxy, alkyloxycarbonyle et aryloxycarbonyle.

**11.** Le précurseur de plaque d'impression lithographique de l'une quelconque des revendications 2-10, dans lequel l'agent absorbant le rayonnement IR a une structure de sel comprenant un cation et un anion, et au moins l'un de l'anion du composé représenté par la formule (Ia) et de l'anion de l'agent absorbant le rayonnement IR est un anion inorganique.

**12.** Le précurseur de plaque d'impression lithographique de l'une quelconque des revendications précédentes, dans lequel la couche d'enregistrement d'image contient en outre un polymère liant.

**13.** Le précurseur de plaque d'impression lithographique de l'une quelconque des revendications précédentes, dans lequel la couche d'enregistrement d'image contient en outre une microcapsule ou un microgel.

**14.** Le précurseur de plaque d'impression lithographique de l'une quelconque des revendications précédentes, qui comprend un support et une couche d'enregistrement d'image apte à être enregistrée par exposition de laser infrarouge, et qui est apte à effectuer une impression par

- l'enregistrement sur le précurseur avec un laser IR et le montage du précurseur d'image enregistrée sur une machine d'impression sans réaliser un procédé de développement; ou
- le montage du précurseur sur une machine d'impression et l'enregistrement sur le précurseur monté avec un laser IR.

**15.** Un procédé d'impression lithographique comprenant les étapes de:

- exposition sous forme d'image du précurseur de plaque d'impression lithographique de l'une quelconque des revendications 2-13 avec un laser IR; et
- la fourniture d'encre huileuse et d'un composant aqueux au précurseur exposé pour accomplir l'impression sans réaliser un procédé de développement, dans laquelle la surface du précurseur non exposé au laser IR est enlevée pendant l'étape d'impression.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2938397 B
- JP 2001277740 A
- JP 2001277742 A
- JP 2002287334 A
- US 20030064318 A
- JP 11277927 A
- JP 2000335129 A
- JP 2003191657 A
- EP 1621340 A
- US 20030082475 A1
- US 20060057492 A1
- JP 2006241301 A
- US 6090865 A
- EP 0373862 A
- US 3905815 A
- JP 46004605 B
- JP 48035281 A
- JP 55032070 A
- JP 60239736 A
- JP 61169835 A
- JP 61169837 A
- JP 62058241 A
- JP 62212401 A
- JP 63070243 A
- JP 63298339 A
- JP 8108621 A
- US 3282693 A
- JP 59152396 A
- JP 61151197 A
- JP 63041483 A
- JP 63041484 A
- JP 2000249 A
- JP 2000291 A
- JP 2004705 A
- JP 3027393 A
- JP 3012403 A
- JP 5083588 A
- JP 6041170 A
- JP 1304453 A
- JP 1152109 A
- JP 6029285 B
- US 3479185 A
- US 4311783 A
- US 4622286 A
- JP 62143044 A
- JP 62150242 A
- JP 9188685 A
- JP 9188686 A
- JP 9188710 A
- JP 2000131837 A
- JP 2002107916 A
- JP 2764769 B
- JP 2002116539 A
- JP 6157623 A
- JP 6175564 A
- JP 6175561 A
- JP 6175554 A
- JP 6175553 A
- JP 6348011 A
- JP 712878S A
- JP 7140589 A
- JP 7306527 A
- JP 7292014 A
- JP 61166544 A
- JP 2002328465 A
- JP 2000066385 A
- JP 2000080068 A
- US 4069055 A
- JP 4365049 A
- US 4069056 A
- EP 104143 A
- US 339049 A
- US 410201 A
- JP 2150848 A
- JP 2296514 A
- EP 370693 A
- EP 390214 A
- EP 233567 A
- EP 297443 A
- EP 297442 A
- US 4933377 A
- US 161811 A
- US 4760013 A
- US 4734444 A
- US 2833827 A
- DE 2904626
- DE 3604580
- DE 3604581
- JP 2001343742 A
- JP 2002148790 A
- US 2850445 A
- JP 4420189 B
- JP 4537377 B
- JP 47002528 B
- JP 54155292 A
- JP 48084183 A
- JP 54151024 A
- JP 52112681 A

- JP 58015503 A
- JP 59140203 A
- JP 59001504 A
- JP 59189340 A
- JP 62174203 A
- JP 62001641 B
- US 4766055 A
- JP 63178105 A
- JP 63258903 A
- JP 3264771 A
- JP 6413140 A
- JP 6413141 A
- JP 6413142 A
- JP 6413143 A
- JP 6413144 A
- JP 6417048 A
- JP 1229003 A
- JP 1298348 A
- JP 1138204 A
- JP 2179643 A
- JP 2244050 A
- JP 63221110 A
- JP 4221958 A
- JP 4219756 A
- JP 6295061 A
- JP 8334897 A
- JP 58125246 A
- JP 59084356 A
- JP 60078787 A
- JP 58173696 A
- JP 58181690 A
- JP 58194595 A
- JP 58112793 A
- JP 58224793 A
- JP 59048187 A
- JP 59073996 A
- JP 60052940 A
- JP 60063744 A
- JP 58112792 A
- GB 434875 A
- US 5156938 A
- US 3881924 A
- JP 57142645 A
- US 4327169 A
- JP 58181051 A
- JP 58220143 A
- JP 59041363 A
- JP 59084248 A
- JP 59054249 A
- JP 59146063 A
- JP 59146061 A
- JP 59216146 A
- US 4283475 A
- JP 5013514 B
- JP 5019702 B
- US 4756993 A
- JP 2002278057 A
- JP 2001133969 A
- JP 3713034 B
- JP 59024147 B
- JP 6433104 A
- JP 6456767 A
- JP 2001714 A
- JP 2226148 A
- JP 2226149 A
- JP 4028499 B
- JP 46042363 B
- JP 2063053 A
- JP 2085858 A
- JP 2216154 A
- JP 57010605 A
- JP 2030321 B
- JP 1287105 A
- JP 62031844 A
- JP 62031848 A
- JP 62143043 A
- JP 59028325 B
- JP 61009621 B
- JP 59028326 B
- JP 59089803 A
- JP 8129257 A
- JP 51047334 B
- JP 57196231 A
- JP 59005240 A
- JP 59005241 A
- JP 1165613 A
- JP 54021726 B
- JP 48041708 B
- JP 51037193 A
- JP 2032293 B
- JP 2016765 B
- JP 58049860 B
- JP 56017654 B
- JP 62039417 B
- JP 62039418 B
- JP 63277653 A
- JP 63260909 A
- JP 1105238 A
- JP 48064183 A
- JP 49043191 B
- JP 52030490 B
- JP 46043946 B
- JP 140337 B
- JP 1040336 B
- JP 2025493 A
- JP 61022048 A
- JP 7021633 B
- JP 62170950 A
- JP 62226143 A
- JP 60168144 A
- JP 62293247 A
- JP 2006297907 A
- US 2800457 A
- US 2800458 A
- US 3287154 A
- JP 3819574 B

- JP 42446 B
- US 3418250 A
- US 3660304 A
- US 3796669 A
- US 3914511 A
- US 4001140 A
- US 4087376 A
- US 4089802 A
- US 4025445 A
- JP 369163 B
- JP 51009079 B
- GB 930422 A
- US 3111407 A
- GB 952807 A
- GB 967074 A
- JP 5061214 A
- JP 54063902 A
- US 2714066 A
- US 3181461 A
- US 3280734 A
- US 3902734 A
- JP 3622063 B
- US 3276868 A
- US 4153461 A
- US 4689272 A
- JP 5045885 A
- JP 6035174 A
- JP 10282679 A
- JP 2304441 A
- US 3458311 A
- JP 55049729 B
- JP 49070702 A


**Non-patent literature cited in the description**


- **I. REETZ et al.** *Polymer International,* 1997, vol. 43, 27-32
- **C. HANSCH ; A. LEO.** Substituent Constants for Correlation Analysis in Chemistry and Biology. John Wiley & Sons
- **A. J. LEO.** Calculating log Poct from structure. *Chem. Rev.,* 1993, vol. 93, 1281-1306
- **WAKABAYASH et al.** *Bull. Chem. Soc. Japan,* 1969, vol. 42, 2924
- **M. P. HUTT.** *Journal of Heterocyclic Chemistry,* 1970, vol. 1 (3
- *Nihon Shashin Gakkai 1968-nen Syunki Kenkyu Happyokai Koenyoshisyu,* 1968, 55
- **MARTIN KUNZ.** *Rad Tech '98, Proceeding,* 19 April 1998
- *Progress in Organic Coatings,* 1985, vol. 13, 123-150
- *J. C. S. Perkin II,* 1979, 1653-1660
- *Journal of Photopolymer Science and Technology,* 1995, 205-232
- *J. C. S Perkin II,* 1979, 156-162
- **S. L SCHLESINGER.** *Photogr. Sci. Eng.,* 1974, vol. 18, 387
- **T. S. BAL et al.** *Polymer,* 1980, vol. 21, 423
- **J.V. CRIVELLO et al.** *Macromolecules,* 1977, vol. 10 (6), 1307
- **J.V. CRIVELLO et al.** *J. Polymer Sci., Polymer Chem. Ed.,* 1979, vol. 17, 1047
- **C.S. WEN et al.** *Teh, Proc. Conf. Rad. Curing ASIA,* October 1988, 478
- Senryo Binran. 1970
- Colour Index. Saishin Ganryo Binran. 1977
- Saishin Ganryo Oyou Gijutsu. CMC Publishing Co., Ltd, 1986
- Insatsu Ink Gijutsu. CMC Publishing Co., Ltd, 1984
- Saiwai Shobo, Insatsu Ink Gijutsu. Kinzoku Sekken no Seishitsu to Oyo. CMC Publishing Co., Ltd, 1984
- Saishin Ganryo Oyo Gijutsu. CMC Publishing Co., Ltd, 1986
- *Nippon Secchaku Kyokaishi,* 1984, vol. 20 (7), 300-308